(19) Europäisches Patentamt
European Patent Office
Office européen des brevets

(11) EP 1 932 917 B1

(12) **EUROPEAN PATENT SPECIFICATION**

(45) Date of publication and mention of the grant of the patent:
**13.10.2010 Bulletin 2010/41**

(51) Int Cl.:
***C12N 15/82*** *(2006.01)*

(21) Application number: **07114182.4**

(22) Date of filing: **30.08.2000**

(54) **Plant sterol acyltransferases**

Pflanzen-Sterol-Acyltransferasen

Acyltransférases stérol de plante

(84) Designated Contracting States:
**AT BE CH CY DE DK ES FI FR GB GR IE IT LI LU MC NL PT SE**

(30) Priority: **30.08.1999 US 152493 P**

(43) Date of publication of application:
**18.06.2008 Bulletin 2008/25**

(62) Document number(s) of the earlier application(s) in accordance with Art. 76 EPC:
**00959644.6 / 1 210 417**

(73) Proprietor: **Monsanto Technology, LLC**
**St. Louis, MO 63167 (US)**

(72) Inventors:
- **Lassner, Michael**
  **Redwood City, CA 94063 (US)**
- **Van Eenennaam, Alison**
  **Davis, CA 95616 (US)**

(74) Representative: **von Kreisler Selting Werner**
**Deichmannhaus am Dom**
**Bahnhofsvorplatz 1**
**50667 Köln (DE)**

(56) References cited:
- **DATABASE UniProt [Online] 1 May 1999 (1999-05-01), "SubName: Full=F21M11.5 protein;" XP002494891 retrieved from EBI accession no. UNIPROT:Q9ZWC1 Database accession no. Q9ZWC1**
- **DATABASE Geneseq [Online] 18 February 1999 (1999-02-18), "Human C-raf target site nucleotide position 877." XP002497057 retrieved from EBI accession no. GSN:AAV91072 Database accession no. AAV91072**
- **TANIYAMA YOSHIO ET AL: "Cloning and expression of a novel lysophospholipase which structurally resembles lecithin cholesterol acyltransferase." BIOCHEMICAL AND BIOPHYSICAL RESEARCH COMMUNICATIONS, vol. 257, no. 1, 2 April 1999 (1999-04-02), pages 50-56, XP002161572 ISSN: 0006-291X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 932 917 B1

SEQUENCE LISTING

<110> Monsanto Technology LLC

<120> PLANT STEROL ACYLTRANSFERASES

<130> 071423ep

<150> EP 00959644.6
<151> 2000-08-30

<150> US 60/152,493
<151> 1999-08-30

<160> 80

<170> PatentIn Ver. 2.1

<210> 1
<211> 440
<212> PRT
<213> Homo sapiens

<400> 1

```
Met Gly Pro Pro Gly Ser Pro Trp Gln Trp Val Thr Leu Leu Leu Gly
 1               5                  10                  15

Leu Leu Leu Pro Pro Ala Ala Pro Phe Trp Leu Leu Asn Val Leu Phe
            20                  25                  30

Pro Pro His Thr Thr Pro Lys Ala Glu Leu Ser Asn His Thr Arg Pro
        35                  40                  45

Val Ile Leu Val Pro Gly Cys Leu Gly Asn Gln Leu Glu Ala Lys Leu
    50                  55                  60

Asp Lys Pro Asp Val Val Asn Trp Met Cys Tyr Arg Lys Thr Glu Asp
65                  70                  75                  80

Phe Phe Thr Ile Trp Leu Asp Leu Asn Met Phe Leu Pro Leu Gly Val
                85                  90                  95

Asp Cys Trp Ile Asp Asn Thr Arg Val Val Tyr Asn Arg Ser Ser Gly
            100                 105                 110

Leu Val Ser Asn Ala Pro Gly Val Gln Ile Arg Val Pro Gly Phe Gly
        115                 120                 125

Lys Thr Tyr Ser Val Glu Tyr Leu Asp Ser Ser Lys Leu Ala Gly Tyr
    130                 135                 140

Leu His Thr Leu Val Gln Asn Leu Val Asn Asn Gly Tyr Val Arg Asp
145                 150                 155                 160

Glu Thr Val Arg Ala Ala Pro Tyr Asp Trp Arg Leu Glu Pro Gly Gln
                165                 170                 175

Gln Glu Glu Tyr Tyr Arg Lys Leu Ala Gly Leu Val Glu Glu Met His
            180                 185                 190

Ala Ala Tyr Gly Lys Pro Val Phe Leu Ile Gly His Ser Leu Gly Cys
        195                 200                 205
```

```
Leu His Leu Leu Tyr Phe Leu Leu Arg Gln Pro Gln Ala Trp Lys Asp
    210                 215                     220

Arg Phe Ile Asp Gly Phe Ile Ser Leu Gly Ala Pro Trp Gly Gly Ser
225                 230                     235                 240

Ile Lys Pro Met Leu Val Leu Ala Ser Gly Asp Asn Gln Gly Ile Pro
                245                 250                     255

Ile Met Ser Ser Ile Lys Leu Lys Glu Glu Gln Arg Ile Thr Thr Thr
            260                 265                     270

Ser Pro Trp Met Phe Pro Ser Arg Met Ala Trp Pro Glu Asp His Val
        275                 280                     285

Phe Ile Ser Thr Pro Ser Phe Asn Tyr Thr Gly Arg Asp Phe Gln Arg
    290                 295                     300

Phe Phe Ala Asp Leu His Phe Glu Glu Gly Trp Tyr Met Trp Leu Gln
305                 310                     315                 320

Ser Arg Asp Leu Leu Ala Gly Leu Pro Ala Pro Gly Val Glu Val Tyr
                325                 330                     335

Cys Leu Tyr Gly Val Gly Leu Pro Thr Pro Arg Thr Tyr Ile Tyr Asp
            340                 345                     350

His Gly Phe Pro Tyr Thr Asp Pro Val Gly Val Leu Tyr Glu Asp Gly
        355                 360                     365

Asp Asp Thr Val Ala Thr Arg Ser Thr Glu Leu Cys Gly Leu Trp Gln
    370                 375                     380

Gly Arg Gln Pro Gln Pro Val His Leu Leu Pro Leu His Gly Ile Gln
385                 390                     395                 400

His Leu Asn Met Val Phe Ser Asn Leu Thr Leu Glu His Ile Asn Ala
                405                 410                     415

Ile Leu Leu Gly Ala Tyr Arg Gln Gly Pro Pro Ala Ser Pro Thr Ala
            420                 425                     430

Ser Pro Glu Pro Pro Pro Pro Glu
        435                 440
```

<210> 2
<211> 1299
<212> DNA
<213> Arabidopsis thaliana

<400> 2

atgaaaaaaa tatcttcaca ttattcggta gtcatagcga tactcgttgt ggtgacgatg 60
acctcgatgt gtcaagctgt gggtagcaac gtgtaccctt tgattctggt tccaggaaac 120
ggaggtaacc agctagaggt acggctggac agagaataca agccaagtag tgtctggtgt 180
agcagctggt tatatccgat tcataagaag agtggtggat ggtttaggct atggttcgat 240
gcagcagtgt tattgtctcc cttcaccagg tgcttcagcg atcgaatgat gttgtactat 300
gaccctgatt tggatgatta ccaaaatgct cctggtgtcc aaacccgggt tcctcatttc 360
ggttcgacca aatcacttct atacctcgac cctcgtctcc gagatgccac atcttacatg 420
gaacatttgg tgaaagctct agagaaaaaa tgcgggtatg ttaacgacca aaccatccta 480
ggagctccat atgatttcag gtacggcctg gctgcttcgg gccacccgtc ccgtgtagcc 540
tcacagttcc tacaagacct caaacaattg gtggaaaaaa ctagcagcga gaacgaagga 600
aagccagtga tactcctctc ccatagccta ggaggacttt tcgtcctcca tttcctcaac 660

cgtaccaccc cttcatggcg ccgcaagtac atcaaacact ttgttgcact cgctgcgcca 720
tggggtggga cgatctctca gatgaagaca tttgcttctg gcaacacact cggtgtccct 780
ttagttaacc ctttgctggt cagacggcat cagaggacct ccgagagtaa ccaatggcta 840
cttccatcta ccaaagtgtt tcacgacaga actaaaccgc ttgtcgtaac tccccaggtt 900
aactacacag cttacgagat ggatcggttt tttgcagaca ttggattctc acaaggagtt 960
gtgccttaca agacaagagt gttgccttta acagaggagc tgatgactcc gggagtgcca 1020
gtcacttgca tatatgggag aggagttgat acaccggagg ttttgatgta tggaaaagga 1080
ggattcgata agcaaccaga gattaagtat ggagatggag atgggacggt taatttggcg 1140
agcttagcag ctttgaaagt cgatagcttg aacaccgtag agattgatgg agtttcgcat 1200
acatctatac ttaaagacga gatcgcactt aaagagatta tgaagcagat ttcaattatt 1260
aattatgaat tagccaatgt taatgccgtc aatgaatga 1299

<210> 3
<211> 432
<212> PRT
<213> Arabidopsis thaliana

<400> 3

<400> 3

```
Met Lys Lys Ile Ser Ser His Tyr Ser Val Val Ile Ala Ile Leu Val
 1               5                  10                  15

Val Val Thr Met Thr Ser Met Cys Gln Ala Val Gly Ser Asn Val Tyr
            20                  25                  30

Pro Leu Ile Leu Val Pro Gly Asn Gly Gly Asn Gln Leu Glu Val Arg
        35                  40                  45

Leu Asp Arg Glu Tyr Lys Pro Ser Ser Val Trp Cys Ser Ser Trp Leu
    50                  55                  60

Tyr Pro Ile His Lys Lys Ser Gly Gly Trp Phe Arg Leu Trp Phe Asp
65                  70                  75                  80

Ala Ala Val Leu Leu Ser Pro Phe Thr Arg Cys Phe Ser Asp Arg Met
                85                  90                  95

Met Leu Tyr Tyr Asp Pro Asp Leu Asp Asp Tyr Gln Asn Ala Pro Gly
            100                 105                 110

Val Gln Thr Arg Val Pro His Phe Gly Ser Thr Lys Ser Leu Leu Tyr
        115                 120                 125

Leu Asp Pro Arg Leu Arg Asp Ala Thr Ser Tyr Met Glu His Leu Val
    130                 135                 140

Lys Ala Leu Glu Lys Lys Cys Gly Tyr Val Asn Asp Gln Thr Ile Leu
145                 150                 155                 160

Gly Ala Pro Tyr Asp Phe Arg Tyr Gly Leu Ala Ala Ser Gly His Pro
                165                 170                 175

Ser Arg Val Ala Ser Gln Phe Leu Gln Asp Leu Lys Gln Leu Val Glu
            180                 185                 190

Lys Thr Ser Ser Glu Asn Glu Gly Lys Pro Val Ile Leu Leu Ser His
        195                 200                 205

Ser Leu Gly Gly Leu Phe Val Leu His Phe Leu Asn Arg Thr Thr Pro
    210                 215                 220

Ser Trp Arg Arg Lys Tyr Ile Lys His Phe Val Ala Leu Ala Ala Pro
225                 230                 235                 240
```

```
Trp Gly Gly Thr Ile Ser Gln Met Lys Thr Phe Ala Ser Gly Asn Thr
                245                 250                 255

Leu Gly Val Pro Leu Val Asn Pro Leu Leu Val Arg Arg His Gln Arg
            260                 265                 270

Thr Ser Glu Ser Asn Gln Trp Leu Leu Pro Ser Thr Lys Val Phe His
        275                 280                 285

Asp Arg Thr Lys Pro Leu Val Val Thr Pro Gln Val Asn Tyr Thr Ala
    290                 295                 300

Tyr Glu Met Asp Arg Phe Phe Ala Asp Ile Gly Phe Ser Gln Gly Val
305                 310                 315                 320

Val Pro Tyr Lys Thr Arg Val Leu Pro Leu Thr Glu Glu Leu Met Thr
                325                 330                 335

Pro Gly Val Pro Val Thr Cys Ile Tyr Gly Arg Gly Val Asp Thr Pro
            340                 345                 350

Glu Val Leu Met Tyr Gly Lys Gly Gly Phe Asp Lys Gln Pro Glu Ile
        355                 360                 365

Lys Tyr Gly Asp Gly Asp Gly Thr Val Asn Leu Ala Ser Leu Ala Ala
    370                 375                 380

Leu Lys Val Asp Ser Leu Asn Thr Val Glu Ile Asp Gly Val Ser His
385                 390                 395                 400

Thr Ser Ile Leu Lys Asp Glu Ile Ala Leu Lys Glu Ile Met Lys Gln
                405                 410                 415

Ile Ser Ile Ile Asn Tyr Glu Leu Ala Asn Val Asn Ala Val Asn Glu
            420                 425                 430
```

<210> 4
<211> 1641
<212> DNA
<213> Arabidopsis thaliana

<400> 4

```
atgggagcga attcgaaatc agtaacggct tccttcaccg tcatcgccgt tttttttcttg 60
atttgcggtg gccgaactgc ggtggaggat gagaccgagt ttcacggcga ctactcgaag 120
ctatcgggta taatcattcc gggatttgcg tcgacgcagc tacgagcgtg gtcgatcctt 180
gactgtccat acactccgtt ggacttcaat ccgctcgacc tcgtatggct agacaccact 240
aagcttcttt ctgctgtcaa ctgctggttt aagtgtatgg tgctagatcc ttataatcaa 300
acagaccatc ccgagtgtaa gtcacggcct gacagtggtc tttcagccat cacagaattg 360
gatccaggtt acataacagg tcctctttct actgtctgga aagagtggct taagtggtgt 420
gttgagtttg gtatagaagc aaatgcaatt gtcgctgttc catacgattg gagattgtca 480
ccaaccaaat tggaagagcg tgacctttac tttcacaagc tcaagttgac ctttgaaact 540
gctttaaaac tccgtggcgg cccttctata gtatttgccc attcaatggg taataatgtc 600
ttcagatact ttctggaatg gctgaggcta gaaattgcac caaaacatta tttgaagtgg 660
cttgatcagc atatccatgc ttatttcgct gttggagctc ctcttcttgg ttctgttgag 720
gcaatcaaat ctactctctc tggtgtaacg tttggccttc ctgtttctga gggaactgct 780
cggttgttgt ccaattcttt tgcgtcgtca ttgtggctta tgccattttc aaagaattgc 840
aagggtgata acacatcctg gacgcatttt tctgggggtg ctgcaaagaa agataagcgc 900
gtataccact gtgatgaaga ggaatatcaa tcaaaatatt ctggctggcc gacaaatatt 960
attaacattg aaattccttc cactagcgtt acagaaacag ctctagtcaa catgaccagc 1020
atggaatgtg gccttcccac ccttttgtct ttcacagccc gtgaactagc agatgggact 1080
cttttcaaag caatagaaga ctatgaccca gatagcaaga ggatgttaca ccagttaaag 1140
```

```
aagttgtatc atgatgaccc tgtttttaat cctctgactc cttgggagag accacctata 1200
aaaaatgtat tttgcatata tggtgctcat ctaaagacag aggttggtta ttactttgcc 1260
ccaagtggca aaccttatcc tgataattgg atcatcacgg atatcattta cgaaactgaa 1320
ggttccctcg tgtcaaggtc tggaactgtg gttgatggga acgctggacc tataactggg 1380
gatgagacgg taccctatca ttcactctct tggtgcaaga attggctcgg acctaaagtt 1440
aacataacaa tggctcccca gccagaacac gatggaagcg acgtacatgt ggaactaaat 1500
gttgatcatg agcatgggtc agacatcata gctaacatga caaaagcacc aagggttaag 1560
tacataacct tttatgaaga ctctgagagc attccgggga agagaaccgc agtctgggag 1620
cttgataaaa gtgggtatta a                                             1641
```

<210> 5
<211> 546
<212> PRT
<213> Arabidopsis thaliana

<400> 5

```
Met Gly Ala Asn Ser Lys Ser Val Thr Ala Ser Phe Thr Val Ile Ala
1               5                   10                  15

Val Phe Phe Leu Ile Cys Gly Gly Arg Thr Ala Val Glu Asp Glu Thr
            20                  25                  30

Glu Phe His Gly Asp Tyr Ser Lys Leu Ser Gly Ile Ile Ile Pro Gly
        35                  40                  45

Phe Ala Ser Thr Gln Leu Arg Ala Trp Ser Ile Leu Asp Cys Pro Tyr
    50                  55                  60

Thr Pro Leu Asp Phe Asn Pro Leu Asp Leu Val Trp Leu Asp Thr Thr
65                  70                  75                  80

Lys Leu Leu Ser Ala Val Asn Cys Trp Phe Lys Cys Met Val Leu Asp
                85                  90                  95

Pro Tyr Asn Gln Thr Asp His Pro Glu Cys Lys Ser Arg Pro Asp Ser
            100                 105                 110

Gly Leu Ser Ala Ile Thr Glu Leu Asp Pro Gly Tyr Ile Thr Gly Pro
        115                 120                 125

Leu Ser Thr Val Trp Lys Glu Trp Leu Lys Trp Cys Val Glu Phe Gly
    130                 135                 140

Ile Glu Ala Asn Ala Ile Val Ala Val Pro Tyr Asp Trp Arg Leu Ser
145                 150                 155                 160

Pro Thr Lys Leu Glu Glu Arg Asp Leu Tyr Phe His Lys Leu Lys Leu
                165                 170                 175

Thr Phe Glu Thr Ala Leu Lys Leu Arg Gly Gly Pro Ser Ile Val Phe
            180                 185                 190

Ala His Ser Met Gly Asn Asn Val Phe Arg Tyr Phe Leu Glu Trp Leu
        195                 200                 205

Arg Leu Glu Ile Ala Pro Lys His Tyr Leu Lys Trp Leu Asp Gln His
    210                 215                 220

Ile His Ala Tyr Phe Ala Val Gly Ala Pro Leu Leu Gly Ser Val Glu
225                 230                 235                 240

Ala Ile Lys Ser Thr Leu Ser Gly Val Thr Phe Gly Leu Pro Val Ser
```

245 250 255

Glu Gly Thr Ala Arg Leu Leu Ser Asn Ser Phe Ala Ser Ser Leu Trp
260 265 270

Leu Met Pro Phe Ser Lys Asn Cys Lys Gly Asp Asn Thr Ser Trp Thr
275 280 285

His Phe Ser Gly Gly Ala Ala Lys Lys Asp Lys Arg Val Tyr His Cys
290 295 300

Asp Glu Glu Glu Tyr Gln Ser Lys Tyr Ser Gly Trp Pro Thr Asn Ile
305 310 315 320

Ile Asn Ile Glu Ile Pro Ser Thr Ser Val Thr Glu Thr Ala Leu Val
325 330 335

Asn Met Thr Ser Met Glu Cys Gly Leu Pro Thr Leu Leu Ser Phe Thr
340 345 350

Ala Arg Glu Leu Ala Asp Gly Thr Leu Phe Lys Ala Ile Glu Asp Tyr
355 360 365

Asp Pro Asp Ser Lys Arg Met Leu His Gln Leu Lys Lys Leu Tyr His
370 375 380

Asp Asp Pro Val Phe Asn Pro Leu Thr Pro Trp Glu Arg Pro Pro Ile
385 390 395 400

Lys Asn Val Phe Cys Ile Tyr Gly Ala His Leu Lys Thr Glu Val Gly
405 410 415

Tyr Tyr Phe Ala Pro Ser Gly Lys Pro Tyr Pro Asp Asn Trp Ile Ile
420 425 430

Thr Asp Ile Ile Tyr Glu Thr Glu Gly Ser Leu Val Ser Arg Ser Gly
435 440 445

Thr Val Val Asp Gly Asn Ala Gly Pro Ile Thr Gly Asp Glu Thr Val
450 455 460

Pro Tyr His Ser Leu Ser Trp Cys Lys Asn Trp Leu Gly Pro Lys Val
465 470 475 480

Asn Ile Thr Met Ala Pro Gln Pro Glu His Asp Gly Ser Asp Val His
485 490 495

Val Glu Leu Asn Val Asp His Glu His Gly Ser Asp Ile Ile Ala Asn
500 505 510

Met Thr Lys Ala Pro Arg Val Lys Tyr Ile Thr Phe Tyr Glu Asp Ser
515 520 525

Glu Ser Ile Pro Gly Lys Arg Thr Ala Val Trp Glu Leu Asp Lys Ser
530 535 540

Gly Tyr
545

<210> 6
<211> 1608
<212> DNA
<213> Arabidopsis thaliana

<400> 6

```
atgtctctat tactggaaga gatcattaga tcagtagagg ctttgctgaa gctcagaaat 60
cggaatcaag aaccctatgt tgacccgaat ctaaacccgg ttcttctagt tccaggaatc 120
gctggatcaa ttctaaacgc cgttgatcat gagaacggga acgaagaacg tgtttgggtt 180
aggatctttg gtgctgatca tgagtttcga acaaagatgt ggtctcgatt tgatccttca 240
actggtaaaa cgatatctct tgatccaaaa acgagtattg ttgttcctca agacagagct 300
gggctacatg caattgatgt cttagaccct gatatgattg ttggccgtga gtctgtgtac 360
tatttccatg agatgattgt tgagatgatc ggatggggat ttgaagaagg gaaaaccctt 420
tttggttttg gttatgattt ccgccaaagc aacagactgc aggaaacgtt ggaccagttt 480
gctaaaaagt tggaaactgt ttataaagcc tcaggagaga agaagattaa tgttattagt 540
cattctatgg gaggactatt ggtgaaatgt ttcatgggtc tgcatagtga tatattcgag 600
aagtatgtac agaattggat tgctattgct gctccatttc gaggtgctcc tggatatatc 660
acatcgactt tattgaatgg aatgtcgttt gtcaatggtt gggaacagaa ctttttcgtc 720
tctaagtgga gcatgcatca gctgcttatt gagtgtccat ccatatatga gctgatgtgt 780
tgtccgtatt ttaaatggga gctccctccc gtcttagagc tgtggagaga gaaagagagc 840
aatgatggag ttggaacctc tgatgttgtt cttgagtctt accgtagcct ggagagcctt 900
gaagttttta cgaaatctct ttcgaataat acagctgatt attgtggaga gtcgatcgat 960
cttcctttta actggaagat catggagtgg gctcacaaaa ccaagcaagt attagcctct 1020
gccaagctgc ctccgaaagt taaattctat aacatatatg ggaccaatct agaaacccct 1080
catagtgttt gctatgggaa tgagaagatg cccgttaaag atctaacgaa tctaagatac 1140
ttccagccga catatatatg cgtggatggt gatggcacag tcccgatgga atctgccatg 1200
gcggatgggc ttgaagcagt agcaagagtt ggagtccctg gtgagcaccg aggaatcctc 1260
aacgatcacc gtgtcttccg aatgctcaaa aaatggctaa atgtaggcga accagacccg 1320
ttctacaacc cagtaaacga ttatgtcatc cttcccacca catatgaatt tgagaaattc 1380
catgagaatg gactcgaggt tgcttccgtg aaagaatcgt gggacatcat atcagatgac 1440
aacaatatcg gcacaaccgg gtcaaccgtg aactccatat cagtctctca acctggagat 1500
gatcaaaacc ctcaagctga agctcgtgca accttaaccg tccaaccaca aagcgatggt 1560
agacaacatg tagagctcaa tgctgtaagt gtctctgttg atgcataa              1608
```

<210> 7
<211> 535
<212> PRT
<213> Arabidopsis thaliana

<400> 7

```
Met Ser Leu Leu Leu Glu Glu Ile Ile Arg Ser Val Glu Ala Leu Leu
 1               5                  10                  15

Lys Leu Arg Asn Arg Asn Gln Glu Pro Tyr Val Asp Pro Asn Leu Asn
            20                  25                  30

Pro Val Leu Leu Val Pro Gly Ile Ala Gly Ser Ile Leu Asn Ala Val
        35                  40                  45

Asp His Glu Asn Gly Asn Glu Glu Arg Val Trp Val Arg Ile Phe Gly
    50                  55                  60

Ala Asp His Glu Phe Arg Thr Lys Met Trp Ser Arg Phe Asp Pro Ser
65                  70                  75                  80

Thr Gly Lys Thr Ile Ser Leu Asp Pro Lys Thr Ser Ile Val Val Pro
                85                  90                  95

Gln Asp Arg Ala Gly Leu His Ala Ile Asp Val Leu Asp Pro Asp Met
            100                 105                 110

Ile Val Gly Arg Glu Ser Val Tyr Tyr Phe His Glu Met Ile Val Glu
        115                 120                 125

Met Ile Gly Trp Gly Phe Glu Glu Gly Lys Thr Leu Phe Gly Phe Gly
```

```
130                 135                 140

Tyr Asp Phe Arg Gln Ser Asn Arg Leu Gln Glu Thr Leu Asp Gln Phe
145                 150                 155                 160

Ala Lys Lys Leu Glu Thr Val Tyr Lys Ala Ser Gly Glu Lys Lys Ile
                165                 170                 175

Asn Val Ile Ser His Ser Met Gly Gly Leu Leu Val Lys Cys Phe Met
            180                 185                 190

Gly Leu His Ser Asp Ile Phe Glu Lys Tyr Val Gln Asn Trp Ile Ala
        195                 200                 205

Ile Ala Ala Pro Phe Arg Gly Ala Pro Gly Tyr Ile Thr Ser Thr Leu
    210                 215                 220

Leu Asn Gly Met Ser Phe Val Asn Gly Trp Glu Gln Asn Phe Phe Val
225                 230                 235                 240

Ser Lys Trp Ser Met His Gln Leu Leu Ile Glu Cys Pro Ser Ile Tyr
                245                 250                 255

Glu Leu Met Cys Cys Pro Tyr Phe Lys Trp Glu Leu Pro Pro Val Leu
            260                 265                 270

Glu Leu Trp Arg Glu Lys Glu Ser Asn Asp Gly Val Gly Thr Ser Asp
        275                 280                 285

Val Val Leu Glu Ser Tyr Arg Ser Leu Glu Ser Leu Glu Val Phe Thr
    290                 295                 300

Lys Ser Leu Ser Asn Asn Thr Ala Asp Tyr Cys Gly Glu Ser Ile Asp
305                 310                 315                 320

Leu Pro Phe Asn Trp Lys Ile Met Glu Trp Ala His Lys Thr Lys Gln
                325                 330                 335

Val Leu Ala Ser Ala Lys Leu Pro Pro Lys Val Lys Phe Tyr Asn Ile
            340                 345                 350

Tyr Gly Thr Asn Leu Glu Thr Pro His Ser Val Cys Tyr Gly Asn Glu
        355                 360                 365

Lys Met Pro Val Lys Asp Leu Thr Asn Leu Arg Tyr Phe Gln Pro Thr
    370                 375                 380

Tyr Ile Cys Val Asp Gly Asp Gly Thr Val Pro Met Glu Ser Ala Met
385                 390                 395                 400

Ala Asp Gly Leu Glu Ala Val Ala Arg Val Gly Val Pro Gly Glu His
                405                 410                 415

Arg Gly Ile Leu Asn Asp His Arg Val Phe Arg Met Leu Lys Lys Trp
            420                 425                 430

Leu Asn Val Gly Glu Pro Asp Pro Phe Tyr Asn Pro Val Asn Asp Tyr
        435                 440                 445

Val Ile Leu Pro Thr Thr Tyr Glu Phe Glu Lys Phe His Glu Asn Gly
    450                 455                 460

Leu Glu Val Ala Ser Val Lys Glu Ser Trp Asp Ile Ile Ser Asp Asp
```

```
465                 470                 475                 480

Asn Asn Ile Gly Thr Thr Gly Ser Thr Val Asn Ser Ile Ser Val Ser
                485                 490                 495

Gln Pro Gly Asp Asp Gln Asn Pro Gln Ala Glu Ala Arg Ala Thr Leu
            500                 505                 510

Thr Val Gln Pro Gln Ser Asp Gly Arg Gln His Val Glu Leu Asn Ala
        515                 520                 525

Val Ser Val Ser Val Asp Ala
    530                 535
```

<210> 8
<211> 1344
<212> DNA
<213> Arabidopsis thaliana

<400> 8

```
atgggctgga ttccgtgtcc gtgctgggga accaacgacg atgaaaacgc cggcgaggtg 60
gcggatcgtg atccggtgct tctagtatct ggaattggag gctctattct gcattctaag 120
aagaagaatt caaagtctga aattcgggtt tgggtccgaa tatttctagc taaccttgcc 180
tttaagcaga gcctctggtc tctctataat cccaaaactg gttatacaga gccgttggat 240
gataatattg aagtattggt ccctgatgat gaccatggac tctatgcaat tgacattcta 300
gatccctctt ggtttgtaaa gctttgtcac ttgacggagg tttatcactt tcacgatatg 360
atagaaatgc tggttggatg cggttataag aaggggacta cattattcgg ttatggttac 420
gatttccgtc aaagcaatag gatcgatcta cttatactag gtctgaagaa gaagctggaa 480
actgcatata aacgttcagg ggggagaaaa gtcactatca tctcccattc aatgggagga 540
cttatggttt catgtttcat gtatctccat ccggaggcat tttccaagta tgtaaataaa 600
tggattacaa ttgcaacacc tttccaagga gcaccagggt gcatcaatga ttcaatcttg 660
actggagtgc aatttgtgga agggttagaa agtttctttt ttgtgtcacg ttggacgatg 720
caccaactgt tggtcgaatg cccatctata tatgagatga tggcaaatcc agactttaag 780
tggaaaaagc aaccagagat tcgagtttgg cgtaagaaat ctgaaaacga cgttgatact 840
tctgtagaac tggaatcatt tggcttaatc gagagtattg atctattcaa cgatgcatta 900
aaaaataacg agctaagcta tggtgggaat aaaatagctt tgccctttaa ctttgctatc 960
ctcgactggg ctgctaagac aagagaaatt ctcaacaaag cgcaacttcc tgatggagtg 1020
tccttctata acatatatgg agtgtcactt aatacaccct ttgatgtttg ttatggcaca 1080
gagacttctc cgatagacga tttgtctgaa atatgtcaaa ctatgcctga gtatacatat 1140
gtagatggag atggaactgt ccctgctgaa tcagctgcag ctgctcagtt taaagcagtt 1200
gctagcgtag gagtttcggg tagccaccgc gggcttctcc gtgatgaaag agtgtttgag 1260
ctcattcaac aatggttagg agttgagccc aagaaggcta aacggaagca tttaaggact 1320
cacaaagtag ttgattctgg ttaa                                         1344
```

<210> 9
<211> 447
<212> PRT
<213> Arabidopsis thaliana

<400> 9

Met Gly Trp Ile Pro Cys Pro Cys Trp Gly Thr Asn Asp Asp Glu Asn
1 5 10 15

Ala Gly Glu Val Ala Asp Arg Asp Pro Val Leu Leu Val Ser Gly Ile
20 25 30

Gly Gly Ser Ile Leu His Ser Lys Lys Lys Asn Ser Lys Ser Glu Ile
35 40 45

```
Arg Val Trp Val Arg Ile Phe Leu Ala Asn Leu Ala Phe Lys Gln Ser
    50                  55                  60

Leu Trp Ser Leu Tyr Asn Pro Lys Thr Gly Tyr Thr Glu Pro Leu Asp
65                  70                  75                  80

Asp Asn Ile Glu Val Leu Val Pro Asp Asp Asp His Gly Leu Tyr Ala
                85                  90                  95

Ile Asp Ile Leu Asp Pro Ser Trp Phe Val Lys Leu Cys His Leu Thr
            100                 105                 110

Glu Val Tyr His Phe His Asp Met Ile Glu Met Leu Val Gly Cys Gly
        115                 120                 125

Tyr Lys Lys Gly Thr Thr Leu Phe Gly Tyr Gly Tyr Asp Phe Arg Gln
    130                 135                 140

Ser Asn Arg Ile Asp Leu Leu Ile Leu Gly Leu Lys Lys Lys Leu Glu
145                 150                 155                 160

Thr Ala Tyr Lys Arg Ser Gly Gly Arg Lys Val Thr Ile Ile Ser His
                165                 170                 175

Ser Met Gly Gly Leu Met Val Ser Cys Phe Met Tyr Leu His Pro Glu
            180                 185                 190

Ala Phe Ser Lys Tyr Val Asn Lys Trp Ile Thr Ile Ala Thr Pro Phe
        195                 200                 205

Gln Gly Ala Pro Gly Cys Ile Asn Asp Ser Ile Leu Thr Gly Val Gln
    210                 215                 220

Phe Val Glu Gly Leu Glu Ser Phe Phe Phe Val Ser Arg Trp Thr Met
225                 230                 235                 240

His Gln Leu Leu Val Glu Cys Pro Ser Ile Tyr Glu Met Met Ala Asn
                245                 250                 255

Pro Asp Phe Lys Trp Lys Lys Gln Pro Glu Ile Arg Val Trp Arg Lys
            260                 265                 270

Lys Ser Glu Asn Asp Val Asp Thr Ser Val Glu Leu Glu Ser Phe Gly
        275                 280                 285

Leu Ile Glu Ser Ile Asp Leu Phe Asn Asp Ala Leu Lys Asn Asn Glu
    290                 295                 300

Leu Ser Tyr Gly Gly Asn Lys Ile Ala Leu Pro Phe Asn Phe Ala Ile
305                 310                 315                 320

Leu Asp Trp Ala Ala Lys Thr Arg Glu Ile Leu Asn Lys Ala Gln Leu
                325                 330                 335

Pro Asp Gly Val Ser Phe Tyr Asn Ile Tyr Gly Val Ser Leu Asn Thr
            340                 345                 350

Pro Phe Asp Val Cys Tyr Gly Thr Glu Thr Ser Pro Ile Asp Asp Leu
        355                 360                 365

Ser Glu Ile Cys Gln Thr Met Pro Glu Tyr Thr Tyr Val Asp Gly Asp
    370                 375                 380
```

Gly Thr Val Pro Ala Glu Ser Ala Ala Ala Ala Gln Phe Lys Ala Val
385 390 395 400

Ala Ser Val Gly Val Ser Gly Ser His Arg Gly Leu Leu Arg Asp Glu
405 410 415

Arg Val Phe Glu Leu Ile Gln Gln Trp Leu Gly Val Glu Pro Lys Lys
420 425 430

Ala Lys Arg Lys His Leu Arg Thr His Lys Val Val Asp Ser Gly
435 440 445

<210> 10
<211> 3107
<212> DNA
<213> Arabidopsis thaliana

<400> 10

```
ccttttttgat ctttcagctc aatgagcttt tctcaatttt ttgggggaac tgaatatgtg 60
aatttcaaag tttccacatc gagtttattc acacgtcttg aatttcgtcc atcctcgttc 120
tgttatccag ctttgaactc ctcccgaccc tgctatggat atattaaaaa aaaagtgttt 180
tgtgggttgc atctttgtta cgatctgcat cttcttcttt cggctcagtg ttcatgtttt 240
tgctatggta gagatgggca atgttattgt tgatggtaac agtggtatag ttgatagtat 300
cttaactaat caattatctc tttgattcag gcctctatgt tgggtggaac acatgtcact 360
tgacaatgaa actgggttgg atccagctgg tattagagtt cgagctgtat caggactcgt 420
ggctgctgac tactttgctc ctggctactt tgtctgggca gtgctgattg ctaaccttgc 480
acatattgga tatgaagaga aaaatatgta catggctgca tatgactggc ggctttcgtt 540
tcagaacaca gaggttcttt tctcatcgtt ctttctatta ttctgttcca tgttacgttt 600
ctttcttcat tacttaaggc ttaaatatgt ttcatgttga attaataggt acgtgatcag 660
actcttagcc gtatgaaaag taatatagag ttgatggttt ctaccaatgg tggaaaaaaa 720
gcagttatag ttccgcattc catgggggtc ttgtattttc tacattttat gaagtgggtt 780
gaggcaccag ctcctctggg tggcgggggt gggccagatt ggtgtgcaaa gtatattaag 840
gcggtgatga acattggtgg accatttctt ggtgttccaa aagctgttgc agggcttttc 900
tctgctgaag caaaggatgt tgcagttgcc aggtattgaa tatctgctta tacttttgat 960
gatcagaacc ttggctctgg aactcaaagt tattctacta aatatcaatt ctaataacat 1020
tgctatatta tcgctgcaac tgacattggt tgattatttt gctgcttatg taactgaaac 1080
tctcttgaga ttagacaaat gatgaattga taattcttac gcattgctct gtgatgacca 1140
gtttcttagc ttcgacgata acatttgtca tactgtcttt tggagggcat tgaattttgc 1200
tatggaaagc gctggagctt ccatgcttgc attctttacc aattagcatt attctgcttc 1260
tttcaatttt cttgtatatg catctatggt cttttatttc ttcttaatta aagactcgtt 1320
ggagtagttg ctctattagt cgcttggttc cttaatatag aactttactt tcttcgaaaa 1380
ttgcagagcg attgccccag gattcttaga caccgatata tttagacttc agaccttgca 1440
gcatgtaatg agaatgacac gcacatggga ctcaacaatg tctatgttac cgaagggagg 1500
tgacacaata tggggcgggc ttgattggtc accggagaaa ggccacacct gttgtgggaa 1560
aaagcaaaag aacaacgaaa cttgtggtga agcaggtgaa aacggagttt ccaagaaaag 1620
tcctgttaac tatggaagga tgatatcttt tgggaaagaa gtagcagagg ctgcgccatc 1680
tgagattaat aatattgatt ttcgagtaag gacatataaa tcataataaa ccttgtacat 1740
tttgtgattg tatgatgaat atctgtacat tttatctggt gaagggtgct gtcaaaggtc 1800
agagtatccc aaatcacacc tgtcgtgacg tgtggacaga gtaccatgac atgggaattg 1860
ctgggatcaa agctatcgct gagtataagg tctacactgc tggtgaagct atagatctac 1920
tacattatgt tgctcctaag atgatggcgc gtggtgccgc tcatttctct tatgggattg 1980
ctgatgattt ggatgacacc aagtatcaag atcccaaata ctggtcaaat ccgttagaga 2040
caaagtaagt gatttcttga ttccaactgt atccttcgtc ctgatgcatt atcagtcttt 2100
ttgttttcgg tcttgttgga tatggttttc agctcaaagc ttacaaagct gtttctgagc 2160
ctttctcaaa aaggcttgct cagttatatt gaggtgctaa agttgataca tgtgactctt 2220
gcttataaat cctccgtttg gtttgttctg ctttttcaga ttaccgaatg ctcctgagat 2280
ggaaatctac tcattatacg gagtggggat accaacggaa cgagcatacg tatacaagct 2340
taaccagtct cccgacagtt gcatcccctt tcagatattc acttctgctc acgaggagga 2400
cgaagatagc tgtctgaaag caggagttta caatgtggat ggggatgaaa cagtacctgt 2460
cctaagtgcc gggtacatgt gtgcaaaagc gtggcgtggc aagacaagat tcaacccttc 2520
cggaatcaag acttacataa gagaatacaa tcactctccg ccggctaacc tgttggaagg 2580
gcgcgggacg cagagtggtg cccatgttga tatcatggga aactttgctt tgatcgaaga 2640

tatcatgagg gttgccgccg gaggtaacgg gtctgatata ggacatgacc aggtccactc 2700
tggcatattt gaatggtcgg agcgtattga cctgaagctg tgaatatcat gatctcttta 2760
agctgtcctg tcagcttatg tgaatccaat actttgaaag agagatcatc atcaattcat 2820
catcatcgtc atcatcatga tgctcaactc acaaagaagc ctgagaatga tactttggtg 2880
cgaaattctc aatacctctt taatattctt attgaatgta aattatacaa tcctatctaa 2940
tgtttgaacg ataacgcaaa acttgctgcg ccatgtttgt ttgtcttgtc aaaagcatca 3000
atttgtgggt tatacgtagt gtagaggatg attcaaattt gtgataaatt tggtaatcaa 3060
agttaattct gaaaatgcaa caccacatga actatgtcac taaggcc             3107
```

<210> 11
<211> 1680
<212> DNA

<213> Arabidopsis thaliana

<220>
<221> unsure
<222> (694)
<223> n=unkown

<400> 11

```
cgcataaggt gttcgagtgt ttgcagcttg agaagttccg agtccaagag acctggagcc 60
aaagatctga accataaaaa tgaccaatca aaatccatta agccaattca aatattcact 120
aaaaatgtta tagttctcat gaatactaac ataacaagtg aaagtaaatt taaaaatgtt 180
catggaccta acctggcgta acggtatgtc tttgccttca gcagaaagta aattactgac 240
ggctttaggg acacctaaaa aggcgggtcc aatgttgacg acggatttga tgtgtttggc 300
acaccaacct ggaccacccc caccgcctcc atcaggaaga ggtgtttcta cccatttaag 360
gaagtgaagg aaatagatag cccccattga atgcggaacc accacaactt tcttaaaccc 420
attggtggca tacattagct cgattttgct cttcagtcta cttaacgatt ggtcacgtac 480
ctgctcggtt tcaatccaaa aactatagat tagtccaaag ctctacaaca atatgtaatt 540
acatacacta aagtagctaa tcatggaggt cttatagtat atcattatca tcattctcta 600
gaccaccagt gttgtcaatg tgatcatata ggtattaata acgactaatc tgagcatacc 660
tcggtgttat ggaaagagag tctccaatca taangaggcc atgtgaaggt tcttgccttc 720
atatccaatt tttgccaaat tctctatgag aactgcccaa gcaaagtagc atggtgcgaa 780
atagtctgca gccactagtc ctgggactgc tcggacacgg attcccggtg gatcgagacc 840
ggtctcactg tctagagata agtgctccaa ccagcacaat ggcctataaa tcaaattaca 900
acaattaaac gaccaagtat acacttcaaa ctaattcaga attgagaaaa tcgaaatgct 960
aaccagaaaa tcatgtaaat caaaaaccgt aacaatcaat atatatatat atattttcca 1020
gaatccatgt taaaaccata accaaaaata tatgaaaatt tagaaatact aaaataatat 1080
gttaaaactg atattctaaa tttagtaagt tttaaaatgc aatgaaatcg tcattcatgt 1140
tttgaacata aatatatttt atagttttgt aggacgattt tctacttcct atatagaaat 1200
caaaacttac ggtttccatt tccaaattcg aatgacattt aaaaacatat cccaaaaatc 1260
acgattaatt attaatttcc taaaaccatc catcattact tagaaaataa tattttcata 1320
aactagttgc aaaacaataa caaaacccaa agaaccatct ccacccatta accaaaatga 1380
aaatccaaag accatccata acaacaacag tataacacta cgtaaagcca attcaagaag 1440
aaaccaagct taaccaatta tacatacccc taaccagacg aaccaaacca atatctgacc 1500
gggtctataa aaatatctcg aaccgaacat aacggtctaa tgtgttacct tctaagaatc 1560
tcggagaagc tagcacccca aagacgttta cgaaagagtc cttcagcgca aggccgacct 1620
tcccaaagct cgagcccgcc ggttacaatc cccggaacaa gaatcaccgg atgaaacgcc 1680
```

<210> 12
<211> 264
<212> DNA
<213> Glycine max

<220>
<221> unsure
<222> (39)
<223> n=unknown

<220>
<221> unsure
<222> (175)
<223> n=unknown

<220>
<221> unsure
<222> (241)
<223> n=unknown

<400> 12

```
ccaagaactc gatgattact tcaacactcc tggggttgng acccgggtcc ctcactttgg 60
ttccaccaac tctcttctct catctcaatc ctcgtctcaa gcatatcacc ggatacatgg 120
cacccctggt agattcatta caaaagcttg gctacgctga tggtgagact ctgtntggag 180
cccttatga ctttagatat ggtctagctg ctgaaggtca cccttcacaa gtgggttcca 240
ngttcctcaa agatctaaag aatt                                        264
```

<210> 13
<211> 273
<212> DNA
<213> Glycine max

<220>
<221> unsure
<222> (12)
<223> n=unknown

<220>
<221> unsure
<222> (33)
<223> n=unknown

<220>
<221> unsure
<222> (252)
<223> n=unknown
<220>
<221> unsure
<222> (265)..(266)
<223> n=unknown

<220>
<221> unsure
<222> (272)
<223> n=unknown

<400> 13

```
ccaacatctg anaggggtag agtaggtatt tcnatctatt atccatattg tgatgaagaa 60
ggaacaagaa gagggtctca agattgaggt tgctacactc acagttacag tagttgttgt 120
gatgctgtca ttgctatgca catgtggggc aagcaacctc gaccctttga ttctaatacc 180
aggtaacgga gggaaccaac tagaagcaag gttgaccaat cagtacaagc cctctacttt 240
catctgcgat cntggtaccc tctcannaag ana                              273
```

<210> 14
<211> 419
<212> DNA
<213> Glycine max

<220>
<221> unsure
<222> (99)
<223> n=unknown

<220>
<221> unsure
<222> (346)
<223> n=unknown .

<220>
<221> unsure
<222> (352)
<223> n=unknown

<220>
<221> unsure
<222> (392)
<223> n=unknown

<220>
<221> unsure
<222> (405)
<223> n=unknown

<220>
<221> unsure
<222> (418)
<223> n=unknown

<400> 14

```
gctgcatatg attggagaat agcatttcag aacactgagg tgagggatca aacactaagt 60
cggataaaaa gcaacataga acttatggtt gctactaang gtggaaataa ggcagttatt 120
attccacatt caatgggggt cttgtacttc ctacatttta tgaaatgggt tgaagcacca 180
gctccaatgg gtggtggggg aggaccagat tggtgctcca aatatataaa ggcagttgta 240
aacattggtg gaccattttt aggtgttccc aaggctatag cagggctatt ctcagctgag 300
gccaaggata ttgctgttgc caggacgata gctccaggat ttttanataa cnatctgttt 360
ccgcattcaa acccttgcaa catgtaatga anatgaaccc gttcnttggg actcaacna  419
```

<210> 15
<211> 272
<212> DNA
<213> Glycine max

<220>
<221> unsure
<222> (1)..(272)
<223> n=unknown

<400> 15

```
tganttgatc ntgngaagtn attctgtgta ttanttccat gacatgaccg ttnnagatnc 60
gtaagtgang ggtntgaaga gggaaagacg ctttttggtn ttngatatga ttttcgccaa 120
agcaacaggt tgcaggaaac aatggatcgg ttggctgcna agttagaatc aanttataat 180
gccgcaggnn ggaagacaat aaacattata nttcattcta tggcggtct tttccnngan 240
atgtttcntg tgcctgcaaa gcgatatttt ga                               272
```

<210> 16
<211> 237
<212> DNA

<213> Glycine max

<220>
<221> unsure
<222> (1)..(237)
<223> n=unknown

<400> 16

```
gattttcgcc aaagcaacag gttgcaggaa acaatggatc ggttggctgc aaagttagaa 60
tcaatntata atgcngcagg agggaagana ataaacatta taactcattc tatgggcggt 120
cttttggtga aatgnttcat gtgcctgcaa agcgatattt ttgagaaata tgttaagaat 180
tgggttgcaa tttgtgcgcc attccagggt gcaccaggaa ccatcaattc naccttt    237
```

<210> 17
<211> 244
<212> DNA
<213> Glycine max

<220>
<221> unsure
<222> (1)..(244)
<223> n=unknown

<400> 17

```
gattttcgcc aaagcaacag gttgcaggaa acaatggatc ggtnggctgc aaagttagaa 60
tgcaatttat aatgctgcag gagggaagaa aataaacatt ataactcatt ctatgggcgg 120
tcttttggtg aaatgtttca tgtgcctgca aagcgatatt tttgagaaat atgttaagaa 180
ttgggttgca atttgtgcgc cattccaggg tgcaccagga accatcaatt ctaccttttt 240
aaat                                                              244
```

<210> 18
<211> 263
<212> DNA
<213> Glycine max

<400> 18

```
gatgaaacta aaccgtgggc gactaagctt gtttactcgg ttgatttatg gcaagatcaa 60
gttcgttgct tcatagaaga ggtcattggt gaaccagtct atcttgtggg caactcacta 120
ggaggattgg ttgcattgta ttttgcggca aacaaccctc atttagtgaa aggtgtcgca 180
ttgcttaagc aacacctttt tggggggtttc tgccaaatcc cataaaaagt ccaagactag 240
cgaaaatatt tccatgggcc gga                                          263
```

<210> 19
<211> 311
<212> DNA
<213> Zea mays

<220>
<221> unsure
<222> (1)..(311)
<223> n=unknown

<400> 19

```
cggacgctgg ncatgttcgg agccccctac gacttccgct acgcgccgcc gtcccccggc 60
cagacgtccg aggtgtactc ccgctacttc aaggagctga tggagctggt cgaggccgcg 120
agcgagagga cccggaagaa ggccgtcatc ctcggccaca gcttcggcgg catggtcgcg 180
ctcgagttcg tccggaacac tccgccggcg tggcggcgcg agcacatcga gcgcctcgtc 240
ctggtcgcgc cgacgctccc cggcgggttc ctggagccgg tgcgcaactt cgcgtccggg 300
acggacatcc t                                                      311
```

<210> 20

<211> 1155
<212> DNA
<213> Zea mays

<400> 20

```
tcgacccacg cgtccggcca caagaaccct ctcaagtcag actggtgcct cggaaagctg 60
agagccgcac tggaagacat gggataccga gacggagaca ccatgttcgg agccccctac 120
gacttccgct acgcgccgcc gtcccccggc cagacgtccg aggtgtactc ccgctacttc 180
aaggagctga tggagctggt cgaggccgca agcgagagga cccggaagaa ggccgtcatc 240
ctcggccaca gcttcggcgg catggtcgcg ctcgagttcg tccggaacac tccgccggcg 300
tggcggcgcg agcacatcga gcgcctcgtc ctggtcgcgc cgacgctccc cggcgggttc 360
ctggagccgg tgcgcaactt cgcgtccggg acggacatcc tmtacgtgcc agcgacgacg 420
ccgctggcca cgcgagccat gtgragragc ttcgagagcg ccatcgtgaa cttcccgtcg 480
ccggccgtgt tcgggcgcct gcaggcgccg ctcgtggtca ccagggagcg gaactactcc 540
gcgtccgcgc acgacatgga gcgcttcctc gccgccgtcg gctccggcga ggccgcggag 600
cccttcagga gacgggccgt ccccaagatg ggcagcttcg cggcgccgat ggtgcccatg 660
acgtacatca gcggggtcgg caacaggacg ccgctgcggc tggtgttctg ggcgacgac 720
ttcgacgcgg ccccggaggt ggcggcgtac ggggacggag atggcaagat caatttgatc 780
agcgtcttgg cgtttgagaa ggagatgcgt cggcagccgg agcagaagaa gcagttcaaa 840
tccatcaaga tcgataaggc ccagcattct acgatcgtca cggatgattt tgccctgcac 900
agggtcattc aagaaattgt tgaggccaat aatcagaaga ttccatccta aattcttcat 960
gtcatgtatg cattaccgag ctgtgggggc caatagtggg ttgggagttg ggacatcggt 1020
tccgtgctta aaacggtcgt ggtgtggtct caattcaatc gattagttat ttgttaacgt 1080
caattgcttg cctcaaaaaa aaaaaaaaaa aaaaaaaaaa aaaaaaaaaa aaaaaaaaaa 1140
aaaaaaaaar gggcg                                                  1155
```

<210> 21
<211> 328
<212> DNA
<213> Zea mays

<400> 21

```
gttggaatgc tcttcaactt tcttacgctc atttacattg cttttctctg cggtaaattg 60
ctggcttaaa tgcatgctgc ttgaacccta taatcagata gaccatcccg aatgcaagtc 120
aaggcctgat agtggtcttc tgcaattaca gagctggacc ctggttatat aacaggtcct 180
ctctcttcag tatggaaaga atgggtcaaa tggtgtgtag agtttggcat tgaagctaat 240
gcaattatcg ctgttccgta tgattggaga ctgcccccat caatgcttga ggagagagat 300
ctgtactttc acaattaaac aggatcag                                    328
```

<210> 22
<211> 356
<212> DNA
<213> Zea mays

<400> 22

```
gtctttctgc aattacagag ctggaccctg gttatataac aggtttcagg tcctctctct 60
tcagtatgga aagaatgggt caaatggtgt gtagagtttg gcattgaagc taatgcaatt 120
atcgctgttc cgtatgattg gagactgccc ccatcaatgc ttgaggagag agatctgtac 180
tttcacaaat taaagtttgt aacacttgcc tcaacttgtt atgaagcaac caatgctata 240
catctgttag gatcagtaag agttaatggc ccatgacgga ttcaggttcc tgctcaccaa 300
cagatcccac aagcatacgg ttaccgccaa tgcctgcagt tggacagtac caaccc     356
```

<210> 23
<211> 1552
<212> DNA
<213> Zea mays

<400> 23

```
tcgacccacg cgtccgcaga catgatcatt ggtgatgaca ctgtgtacta ctatcatgac 60
atgatagtgg aaatgattaa atggggatat caagaaggaa aaactctctt tggatttggt 120
tatgatttcc gtcaaagcaa caggctctca gagacacttg acagattttc taaaaagctg 180
gagtcagtgt acacagcttc tggtggaaag aagatcaatc tcattactca ttcaatgggg 240
ggattacttg tgaaatgttt catctcactg cacagtgata tatttgaaaa atatgtcaar 300
agttggatcg caattgctgc accattccar ggtgcccctg ggtamataac taccaktytg 360
ctgaatggaa tgtcttttgt craaggatgg gaaycaagat tctttatttc caaawkgkgt 420
atgcascaat tgctacttga gtgcccatca atctatgagk tgctgscaam ccctaacttt 480
ccagtggaga gacatcccac tgctacagat ttggagagag aatttggata mcagtggcaa 540
gaaaagtgcc ctgttagagt cgtatgagcc tgaggaagca ataaagatga ttaaagaggc 600
tctttccagt aatgagatca ttgctgatgg catgcatatt ccggtgcccc ttaatttgga 660
tatattgaat tgggcaaaga aacttatgat cttttatgca gtacaaagct tccggaatca 720
gtgaaattct acaacattta tgggattgat tatgatactc cacatactgt ctgctatggc 780
agtgaacagc agccggtttc aagtcttagt agcctcttat atgctcaggg aaaatacgtc 840
tatgttgatg gcgacggatc tgttcccgca gaatcagcaa aggctgacgg atttaatgca 900
gtggcaaggg ttggggttgc tcctgaccac cggggaatcg tgtgcagtcg ccgcgcgttc 960
cggatcgtcc agcactggct gcacgccgga gaacctgacc cgttctacga cccgctgagc 1020
gactatgtca tactcccaac acgcttacga aatcgagaag catcgtgaga aacacgggga 1080
tgtcacgtca gtagcggagg actgggagat catctcccct aacgacggca agaccatrrg 1140
gccaggcgag cttcctccta tggtcagcac actgaccacg agccgggaag gcaaggaggg 1200
agcactggaa gaggcgcatg ccaccgtggt cgttcacccg gagaagaagg gacggcagca 1260
tgtgcaagtt agggctgtgg gtgtcagcca tggtggctaa agccgtagga gccacgttgg 1320
ttgtctactc tatctagcag tagcagctat acctctgtgc acgcactgta aaattggatg 1380
tacatatatg gctatgacct ctgtagggat ctggttttag aagtataaat gggcaccctg 1440
cctgcttgta aatgttcaga accgaaaaca caggccctgt tctttttttt cctttttaaa 1500
aaaaataaaa agatggtaaa ggattccatt aaaaaaaaaa aaaaaaaagg cg         1552
```

<210> 24
<211> 227
<212> DNA
<213> Zea mays

<220>
<221> unsure
<222> (1)..(227)
<223> n=unknown

<400> 4

<400> 24
ttggttatga tttccgtcaa agcaacaggc tctcagagac acttgacaga ttttctaaaa 60
agctggagtc agtgtacaca gcttctggtg gaaagaagat caatctcatt actcattcaa 120
tgggggatt acttgtgaaa tgtntcatct cactgcacag tgatatatnt gaaaaatatg 180
tcaagagttg gntcgcaatt gcngcaccat tccaaggtgc ccctggg 227

<210> 25
<211> 1587
<212> DNA
<213> Zea mays

<220>
<221> unsure
<222> (1)..(1587)
<223> n=unknown

<400> 25

ggagattgtc gtgccggagg acgaccacgg cctgtttgcc atcgacattc ttgatccttc 60
ctggtttgta gaactcgacc cacgcgtccg cccaccgtcc gggagattgt cgtgccggag 120
gacgaccacg gcctgtttgc catcgacatt cttgatcctt cctggtttgt agaacttctc 180
catctgtcta tggtgtatca cttccatgat atgattgata tgctcataaa ctgtggatat 240
gagaaaggga ccacactatt tggatatggt tatgattttc gccaaagcaa caggatagac 300
aaagcgatgg ctggtttgag agcaaaactt gagacagctc ataagacctc tggagggaaa 360
aaagttaatt taatctcaca ttctatgggt ggattgctag tacgctgctt catgtctatg 420

aatcatgatg tattcactaa gtatgtcaac aaatggattt gcattgcttg tccattccaa 480

ggtgcccccg gatgcatcaa tgattctcta cttactggat tgcaatttgt ttatggtttt 540
gagagcttct ttttcgtatc tagatgggca atgcaccaat tgcttgtcga atgcccatca 600
atctatgaaa tgttaccaaa tccagaattc aagtggaagg aaaaaccaat tattcaggtt 660
tggcgtaaga accctgaaaa ggatggaact gtggagcttg ttcaatatga agcaactgat 720
tgtgtgtcct tgttcgaaga agctttaagg aataatgagc tcacgtataa cggaaagaaa 780
gtagcactac cattcaatat gtcagtcttc aaatgggcca ccaagactcg ccaaatccta 840
gacaatgctg aattaccaga tactgtgagc ttttacaata tatacgggac atcttatgaa 900
actccatacg atgtatgcta tggctcagaa agctctccga ttggagattt gtcagaagtg 960
tgtcacacag tgccggcata cacttatgtg gatggagatt gcacggttcc catagaatcg 1020
gcacgggctg atgggttctc tgcgaaagaa agagttggcg tcaaggcgga ccaccgtggc 1080
ctgctgtccg atgagaacgt attcaagctt ctcaagaaat ggctcggtgt gagcgagaag 1140
aagtcagagt ggcgttgcgt gtctaaatcc tactccaaag tgacctaatt gggttgcctg 1200
tagttcttca ggaagactgt tattttggcc tttcctcctg aagagaagat gaaacaaaat 1260
tctggtgatt gtattgtatg tctgcacgat gtaaatctct gcaagctgca cggaacaagg 1320
gattagtgcc cttgtacgat gtatcattgg caggcatttn tttttgaacc tangggcata 1380
tttntttgnc cttccactct ggacntagta aagaatatnt gaatcgacct tanttnnaan 1440
nngtctgnnn nnnnnnnnnn nnnnnnnnnn nnnnnnnnnn nnnnnnnnnn nnnnnnnnnn 1500
nnnnnnnnnn nnaaaaaaaa awgkgaagcc gntnntnntt tnaaaagnnt tttnnnaaaa 1560
aaaaaaaaaa aaaaaaaaaa aaaaaaa 1587

<210> 26
<211> 300
<212> DNA
<213> Zea mays

<220>

<221> unsure
<222> (1)..(300)
<223> n=unknown

<400> 26

```
gacaaagcga tggctggttt gagagcaaaa cttgagacag ctcataagac ctctggaggg 60
aaaaaagtta atttaatctc acattctatg ggtggattgc tagtacgctg cttcatgtct 120
atgaatcatg atgtgagttt tcatgttttc tgtgtttttt ttgcttttgc ataaatatcc 180
atgtcaattt cccccatttt ctaggtattc actangtatg tcaacaaatg gatttgcatt 240
gcttgtccat tccaaggtaa cttatgggac atttcaattg tttattanat natggggncc 300
```

<210> 27
<211> 1240
<212> DNA
<213> Zea mays

<220>
<221> unsure
<222> (1)..(1240)
<223> n=unknown

<400> 27

```
tcgacccacg cgtccggttc ccagttccca ccgtgtagat ggttctggta taaaatgtat 60
tgccatattt gtaacacaga ttactatata caggttcgtg atcaaacttt gagcagaata 120
aagascaata ttgaactcat agwagsgaca aatggtggaa atagggtggt ggkmgatccc 180
acnactccat ggggtcnttn attttntgcn ttttacgnaa tggntcgaag ccctcctccg 240
tggggggcagt gggtccgaac tggntgtaga accatataaa gctgtaatga atattggagg 300
atctttctta ggagttccta aggctgttgc tgggcttttt ttcttctaag caaaagatgt 360
tgccggttgc taggtataag taatgattca tttatttaaa gcaaaaggga atagcaaaag 420
aatgaatatt attggatgct cgacaagctt gcggagcttt tgctcccaag ccatcttctg 480
gacctcacaa gtccagggag tgcctgcctc tgatcctcat catcaggaac aggctcaagt 540
atgcaccgac ggtaccgtga ggtcatttct atcctgatgc aacaccatgt acttgttgat 600
ggcaaggtca ggactgacaa gacctaccct gctgggttca tggatgtcat ttccatccct 660

aagacaaacg agaactacag gctgctttcg tcttcaccca atcagggatg aggatgccaa 720
gttcaagctc tacaaggtga ggtctgttca gtttggccag aaagacatcc cctatctgaa 780
cacctacgac gaccgcacca tccgctaccc cgacccgctc atcaaggcca acgacaccat 840
caagatcgat ctggagacca acaagatcat ggacttcatc atgtttgacg tcggcaacgt 900
ggtcatggtg atcggcagga ggaataccgg gcgtgtagga gtgatcaara taagggagaa 960
gcataagggc aacttcgaga ccatccacgt gctgcttgra gctttttgct atgtctagtt 1020
ttctcctatt tgttgtacag gaaaacatag aatgaaattc aaatttggtg gccacaaaag 1080
tgtggagact tgatttcata taaagttagg cttaacatta gtgcaaacag ttgtatttta 1140
gtttagattt agagtacact atgtatgcgt tgtttgacaa tgcttattta tgatatattg 1200
aatggtactt atttatatta attaattaaa aaaaaaaaaa                       1240
```

<210> 28
<211> 324
<212> DNA
<213> Zea mays

<400> 28

cgaatgctcc tgacatggaa atattttcca tgtacggagt aggcattcct actgaaaggg 60
catatgtcta taagttggcc ccacaggcag aatgttatat acctttccga attgacacct 120
cggctgaagg cggggaggaa aatagctgct tgaaaggggg tgtttactta gccgatggtg 180
atgaaactgt tccagttctt agtgcgggct acatgtgtgc aaaaggatgg cgtggcaaaa 240
ctcgtttcaa ccctgccggc agcaagactt acgtgagaga atacagccat tcaccaccct 300
ctactctcct ggaaggcagg ggca 324

<210> 29
<211> 254
<212> DNA
<213> Zea mays

<400> 29

gaataaagag caacattgaa ctcatggtag caacaaatgg tggaaatagg gtggtggtga 60
tcccacactc catgggggtc ctctattttt tgcattttat gaaatgggtc gaagcacctc 120
ctcccatggg gggtggcggt ggtccagact ggtgtgagaa gcatattaaa gctgtaatga 180
atattggagg acctttctta ggagttccta aggctgttgc tggccttttc tcatctgaag 240
ccaaagatgt tgcc 254

<210> 30
<211> 518
<212> DNA
<213> Mus musculus

<400> 30

tggaggacaa cgcggggtct gatacgactc actataggga atttggccct cgagcagtag 60
attcggcacg atgggcacga ggactccatc atgttcctca agctttattc ctaccgggat 120
gtcaacctgt ggtgccgcca gcgaagggtc aaggccaaag ctgtctctac agggaagaag 180
gtcagtgggg ctgctgcgag caagctgtga gctatccaga caacctgacc taccgagatc 240
tcgattactt catctttgct cctactttgt gttatgaact caactttcct cggtcccccc 300
gaatacgaga gcgctttctg ctacgacgag ttcttgagat gctctttttt acccagcttc 360
aagtggggct gatccaacag tggatggtcc ctactatcca gaactccatg gaagcccttt 420
caagagcttc tgcagttttg gagaccgcga gttctacaga gattggtgga atgctgagtc 480
tgtcaccgac ttttggcaga actggaatat ccccgtgg 518

<210> 31
<211> 299
<212> DNA
<213> Mus musculus

<400> 31

ccatgatggc tcaggtccca ctggcctgga ttgtgggccg attcttccaa gggaactatg 60

gcaatgcagc tgtgtgggtg acactcatca ttgggcaacc ggtggctgtc tcatgtatgt 120
ccacgactac tacgtgctca actacgatgc cccagtgggt catgagctac tgccaaaggc 180
agccctccct aacctggccc tggagttctg gaggggttcc tggctgcctg cacactcctc 240
ctagtctggg aggcctctct gcccctatgc gctactcctg ctcttgggga tggcatttg 299

<210> 32
<211> 1895
<212> DNA
<213> Artificial Sequence

<220>
<223> Description of Artificial Sequence: Inferred cDNA sequence

<220>
<221> unsure
<222> (1)..(1895)
<223> n=unknown

<400> 32

gtctggtgtg atggggacag ggagggactt ccccttaccc agcactggtg ttggctgagg 60
tgggtgctga gtctcagagc ttggcatgga gaccagacag ggctgggtct gcaagcctga 120
ggctgccgcc ctgagctcgg gctgggacgt gcccagaggt gttgggagga tctggggtga 180
gtaccctgtg gccaggacta aaggggctnc accctcctgt ccatccctcg cagatcttga 240
gcaatgcccg gttatttctg gagaacctca tcaagtatgg catcctggtg gaccccatcc 300
aggtggtttc tctgttcctg aaggatccct atagctggcc cgccccatgc ctggttattg 360
cggccaatgt ctttgctgtg gctgcattcc aggttgagaa gcgcctggcg gtgggtgccc 420
tgacggagca ggcgggactg ctgctgcacg tggccaacct ggccaccatt ctgtgtttcc 480
cagcggctgt ggtcttactg gttgagtcta tcactccagt gggctccctg ctggcgctga 540
tggcgcacac catcctcttc ctcaagctct tctcctaccg cgacgtcaac tcatggtgcc 600
gcagggccag ggccaaggct gcctctgcag ggaagaaggc cagcagtgct gctgccccgc 660
acaccgtgag ctacccggac aatctgacct accgcgatct ctactacttc ctcttcgccc 720
ccaccttgtg ctacgagctc aactttcccc gctctccccg catccggaag cgctttctgc 780
tgcgacggat ccttgagatg ctgttcttca cccagctcca ggtggggctg atccagcagt 840
ggatggtccc caccatccag aactccatga agcccttcaa ggacatggac tactcacgca 900
tcatcgagcg cctcctgaag ctggcggtcc ccaatcacct catctggctc atcttcttct 960
actggctctt ccactcctgc ctgaatgccg tggctgagct catgcagttt ggagaccggg 1020
agttctaccg ggactggtgg aactccgagt ctgtcaccta cttctggcag aactggaaca 1080
tccctgtgca caagtggtgc atcagacact tctacaagcc catgcttcga cggggcagca 1140
gcaagtggat ggccaggaca ggggtgttcc tggcctcggc cttcttccac gagtacctgg 1200
tgagcgtccc tctgcgaatg ttccgcctct gggcgttcac gggcatgatg gctcagatcc 1260
cactggcctg gttcgtgggc cgctttttcc agggcaacta tggcaacgca gctgtgtggc 1320
tgtcgctcat catcggacag ccaatagccg tcctcatgta cgtccacgac tactacgtgc 1380
tcaactatga ggccccagcg gcagaggcct gagctgcacc tgagggcctg gcttctcact 1440
gccacctcac acccgctgcc agagcccacc tctcctccta ggcctcgagt gctggggatg 1500
ggcctggctg cacagcatcc tcctctggtc ccagggaggc ctctctgccc ctatggggct 1560
ctgtcctgca cccctcaggg atggcgacag caggccagac acagtctgat gccagctggg 1620
agtcttgctg accctgcccc gggtccgagg gtgtcaataa agtgctgtcc agtgacctct 1680
tcagcctgcc aggggcctgg ggcctggtgg ggggtatggc cacacccaca agggcgagtg 1740
ccagagctgt gtggacagct gtcccaggac ctgccgggga gcagcagctc cactgcagca 1800
gggcgggcat ggccggtagg gggagtgcaa ggccaggcag acgcccccat tccccacact 1860
cccctaccta gaaaagctca gctcaggcgt cctct 1895

<210> 33
<211> 1766
<212> DNA
<213> Artificial Sequence

<220>
<223> Description of Artificial Sequence: Inferred cDNA sequence

<400> 33

```
cacgactggg ccgcgacgtg gtgcgggccg aagccatggg cgaccgcgga ggcgcgggaa 60
gctctcggcg tcggaggacc ggctcgcggg tttccatcca gggtggtagt gggcccatgg 120
tagacgaaga ggaggtgcga gacgccgctg tgggccccga cttgggcgcc gggggtgacg 180
ctccggctcc ggctccggtt ccggctccag cccacacccg ggacaaagac cggcagacca 240
gcgtgggcga cggccactgg gagctgaggt gccatcgtct gcaagactct ttgttcagct 300
cagacagcgg tttcagcaat taccgtggta tcctgaattg gtgcgtggtg atgctgatcc 360
tgagtaatgc aaggttattt ttagagaatc ttatcaagta tggcatcctg gtggatccca 420
tccaggtggt gtctctgttt ctgaaggacc cctacagctg gcctgcccca tgcttgatca 480
ttgcatccaa tatctttatt gtggctacat ttcagattga gaagcgcctg tcagtgggtg 540
ccctgacaga gcagatgggg ctgctgctac atgtggttaa cctggccaca attatctgct 600
tcccagcagc tgtggcctta ctggttgagt ctatcactcc agtgggttcc ctgtttgctc 660
tggcatcata ctccatcatc ttcctcaagc ttttctccta ccgggatgtc aatctgtggt 720
gccgccagcg aagggtcaag gccaaagctg tgtctgcagg gaagaaggtc agtggggctg 780
ctgcccagaa cactgtaagc tatccggaca acctgaccta ccgagatctc tattacttca 840
tctttgctcc tactttgtgt tatgaactca actttcctcg atccccccga atacgaaagc 900
gctttctgct acggcgggtt cttgagatgc tctttttcac ccagcttcaa gtggggctga 960
tccagcagtg gatggtccct actatccaga actccatgaa gcccttcaag gacatggact 1020
attcacgaat cattgagcgt ctcttaaagc tggcggtccc caaccatctg atatggctca 1080
tcttcttcta ttggcttttc cactcatgtc tcaatgctgt ggcagagctc ctgcagtttg 1140
gagaccgcga gttctacagg gactggtgga atgctgagtc tgtcacctac ttttggcaga 1200
actggaatat ccccgtgcac aagtggtgca tcagacactt ctacaagcct atgctcagac 1260
tgggcagcaa caaatggatg gccaggactg ggtctttttt ggcgtcagcc ttcttccatg 1320
agtacctagt gagcattccc ctgaggatgt tccgcctctg ggcattcaca gccatgatgg 1380
ctcaggtccc actggcctgg attgtgaacc gcttcttcca agggaactat ggcaatgcag 1440
ctgtgtgggt gacactcatc attgggcaac cggtggctgt gctcatgtat gtccacgact 1500
actacgtgct caactatgat gccccagtgg gggcctgagc tactgccaaa ggccagccct 1560
ccctaacctg ggcctggagt tctggagggc ttcctggctg cctgcacact cctcctagtc 1620
tgggaggcct ctctgcccct atggggccta ctcctgctct tggggatggc acctgagtcc 1680
agctggtatg agccagtgct gggagtctgt gctgaccagg ggctgaggat atcaataaag 1740
agctatctaa aaaaaaaaaa aaaaaa                                      1766
```

<210> 34
<211> 409
<212> PRT
<213> Homo sapiens

<400> 34

```
Arg Arg Ser Leu Leu Asp Glu Leu Leu Glu Val Asp His Ile Arg Thr
 1               5                  10                  15

Ile Tyr His Met Phe Ile Ala Leu Leu Ile Leu Phe Ile Leu Ser Thr
            20                  25                  30

Leu Val Val Asp Tyr Ile Asp Glu Gly Arg Leu Val Leu Glu Phe Ser
        35                  40                  45

Leu Leu Ser Tyr Ala Phe Gly Lys Phe Pro Thr Val Val Trp Thr Trp
    50                  55                  60

Trp Ile Met Phe Leu Ser Thr Phe Ser Val Pro Tyr Phe Leu Phe Gln
65                  70                  75                  80

His Trp Arg Thr Gly Tyr Ser Lys Ser Ser His Pro Leu Ile Arg Ser
                85                  90                  95

Leu Phe His Gly Phe Leu Phe Met Ile Phe Gln Ile Gly Val Leu Gly
            100                 105                 110

Phe Gly Pro Thr Tyr Val Val Leu Ala Tyr Thr Leu Pro Pro Ala Ser
        115                 120                 125
```

```
Arg Phe Ile Ile Ile Phe Glu Gln Ile Arg Phe Val Met Lys Ala His
    130                 135                 140

Ser Phe Val Arg Glu Asn Val Pro Arg Val Leu Asn Ser Ala Lys Glu
145                 150                 155                 160

Lys Ser Ser Thr Val Pro Ile Pro Thr Val Asn Gln Tyr Leu Tyr Phe
                165                 170                 175

Leu Phe Ala Pro Thr Leu Ile Tyr Arg Asp Ser Tyr Pro Arg Asn Pro
            180                 185                 190

Thr Val Arg Trp Gly Tyr Val Ala Met Lys Phe Ala Gln Val Phe Gly
        195                 200                 205

Cys Phe Phe Tyr Val Tyr Tyr Ile Phe Glu Arg Leu Cys Ala Pro Leu
    210                 215                 220

Phe Arg Asn Ile Lys Gln Glu Pro Phe Ser Ala Arg Val Leu Val Leu
225                 230                 235                 240

Cys Val Phe Asn Ser Ile Leu Pro Gly Val Leu Ile Leu Phe Leu Thr
                245                 250                 255

Phe Phe Ala Phe Leu His Cys Trp Leu Asn Ala Phe Ala Glu Met Leu
            260                 265                 270

Arg Phe Gly Asp Arg Met Phe Tyr Lys Asp Trp Trp Asn Ser Thr Ser
        275                 280                 285

Tyr Ser Asn Tyr Tyr Arg Thr Trp Asn Val Val Val His Asp Trp Leu
    290                 295                 300

Tyr Tyr Tyr Ala Tyr Lys Asp Phe Leu Trp Phe Phe Ser Lys Arg Phe
305                 310                 315                 320

Lys Ser Ala Ala Met Leu Ala Val Phe Ala Val Ser Ala Val Val His
                325                 330                 335

Glu Tyr Ala Leu Ala Val Cys Leu Ser Phe Phe Tyr Pro Val Leu Phe
            340                 345                 350

Val Leu Phe Met Phe Phe Gly Met Ala Phe Asn Phe Ile Val Asn Asp
        355                 360                 365

Ser Arg Lys Lys Pro Ile Trp Asn Val Leu Met Trp Thr Ser Leu Phe
    370                 375                 380

Leu Gly Asn Gly Val Leu Leu Cys Phe Tyr Ser Gln Glu Trp Tyr Ala
385                 390                 395                 400

Arg Arg His Cys Pro Leu Lys Asn Pro
                405
```

<210> 35
<211> 409
<212> PRT
<213> Mus musculus

<400> 35

Arg Gln Ser Leu Leu Asp Glu Leu Phe Glu Val Asp His Ile Arg Thr

```
1               5                   10                  15
Ile Tyr His Met Phe Ile Ala Leu Leu Ile Leu Phe Val Leu Ser Thr
            20                  25                  30
Ile Val Val Asp Tyr Ile Asp Glu Gly Arg Leu Val Leu Glu Phe Asn
        35                  40                  45
Leu Leu Ala Tyr Ala Phe Gly Lys Phe Pro Thr Val Ile Trp Thr Trp
    50                  55                  60
Trp Ala Met Phe Leu Ser Thr Leu Ser Ile Pro Tyr Phe Leu Phe Gln
65                  70                  75                  80
Pro Trp Ala His Gly Tyr Ser Lys Ser Ser His Pro Leu Ile Tyr Ser
                85                  90                  95
Leu Val His Gly Leu Leu Phe Leu Val Phe Gln Leu Gly Val Leu Gly
            100                 105                 110
Phe Val Pro Thr Tyr Val Val Leu Ala Tyr Thr Leu Pro Pro Ala Ser
        115                 120                 125
Arg Phe Ile Leu Ile Leu Glu Gln Ile Arg Leu Ile Met Lys Ala His
    130                 135                 140
Ser Phe Val Arg Glu Asn Ile Pro Arg Val Leu Asn Ala Ala Lys Glu
145                 150                 155                 160
Lys Ser Ser Lys Asp Pro Leu Pro Thr Val Asn Gln Tyr Leu Tyr Phe
                165                 170                 175
Leu Phe Ala Pro Thr Leu Ile Tyr Arg Asp Asn Tyr Pro Arg Thr Pro
            180                 185                 190
Thr Val Arg Trp Gly Tyr Val Ala Met Gln Phe Leu Gln Val Phe Gly
        195                 200                 205
Cys Leu Phe Tyr Val Tyr Tyr Ile Phe Glu Arg Leu Cys Ala Pro Leu
    210                 215                 220
Phe Arg Asn Ile Lys Gln Glu Pro Phe Ser Ala Arg Val Leu Val Leu
225                 230                 235                 240
Cys Val Phe Asn Ser Ile Leu Pro Gly Val Leu Ile Leu Phe Leu Ser
                245                 250                 255
Phe Phe Ala Phe Leu His Cys Trp Leu Asn Ala Phe Ala Glu Met Leu
            260                 265                 270
Arg Phe Gly Asp Arg Met Phe Tyr Lys Asp Trp Trp Asn Ser Thr Ser
        275                 280                 285
Tyr Ser Asn Tyr Tyr Arg Thr Trp Asn Val Val Val His Asp Trp Leu
    290                 295                 300
Tyr Tyr Tyr Val Tyr Lys Asp Leu Leu Trp Phe Phe Ser Lys Arg Phe
305                 310                 315                 320
Lys Ser Ala Ala Met Leu Ala Val Phe Ala Leu Ser Ala Val Val His
                325                 330                 335
Glu Tyr Ala Leu Ala Ile Cys Leu Ser Tyr Phe Tyr Pro Val Leu Phe
```

340 345 350

Val Leu Phe Met Phe Phe Gly Met Ala Phe Asn Phe Ile Val Asn Asp
355 360 365

Ser Arg Lys Arg Pro Ile Trp Asn Ile Met Val Trp Ala Ser Leu Phe
370 375 380

Leu Gly Tyr Gly Leu Ile Leu Cys Phe Tyr Ser Gln Glu Trp Tyr Ala
385 390 395 400

Arg Gln His Cys Pro Leu Lys Asn Pro
405

<210> 36
<211> 429
<212> PRT
<213> Saccharomyces cerevisiae

<400> 36

```
Asp Lys Ala Asp Ala Pro Pro Gly Glu Lys Leu Glu Ser Asn Phe Ser
 1               5                  10                  15

Gly Ile Tyr Val Phe Ala Trp Met Phe Leu Gly Trp Ile Ala Ile Arg
            20                  25                  30

Cys Cys Thr Asp Tyr Tyr Ala Ser Tyr Gly Ser Ala Trp Asn Lys Leu
        35                  40                  45

Glu Ile Val Gln Tyr Met Thr Thr Asp Leu Phe Thr Ile Ala Met Leu
    50                  55                  60

Asp Leu Ala Met Phe Leu Cys Thr Phe Phe Val Val Phe Val His Trp
65                  70                  75                  80

Leu Val Lys Lys Arg Ile Ile Asn Trp Lys Trp Thr Gly Phe Val Ala
                85                  90                  95

Val Ser Ile Phe Glu Leu Ala Phe Ile Pro Val Thr Phe Pro Ile Tyr
            100                 105                 110

Val Tyr Tyr Phe Asp Phe Asn Trp Val Thr Arg Ile Phe Leu Phe Leu
        115                 120                 125

His Ser Val Val Phe Val Met Lys Ser His Ser Phe Ala Phe Tyr Asn
    130                 135                 140

Gly Tyr Leu Trp Asp Ile Lys Gln Glu Leu Glu Tyr Ser Ser Lys Gln
145                 150                 155                 160

Leu Gln Lys Tyr Lys Glu Ser Leu Ser Pro Glu Thr Arg Glu Ile Leu
                165                 170                 175

Gln Lys Ser Cys Asp Phe Cys Leu Phe Glu Leu Asn Tyr Gln Thr Lys
            180                 185                 190

Asp Asn Asp Phe Pro Asn Asn Ile Ser Cys Ser Asn Phe Phe Met Phe
        195                 200                 205

Cys Leu Phe Pro Val Leu Val Tyr Gln Ile Asn Tyr Pro Arg Thr Ser
    210                 215                 220
```

```
Arg Ile Arg Trp Arg Tyr Val Leu Glu Lys Val Cys Ala Ile Ile Gly
225                 230                 235                 240

Thr Ile Phe Leu Met Met Val Thr Ala Gln Phe Phe Met His Pro Val
                245                 250                 255

Ala Met Arg Cys Ile Gln Phe His Asn Thr Pro Thr Phe Gly Gly Trp
            260                 265                 270

Ile Pro Ala Thr Gln Glu Trp Phe His Leu Leu Phe Asp Met Ile Pro
        275                 280                 285

Gly Phe Thr Val Leu Tyr Met Leu Thr Phe Tyr Met Ile Trp Asp Ala
    290                 295                 300

Leu Leu Asn Cys Val Ala Glu Leu Thr Arg Phe Ala Asp Arg Tyr Phe
305                 310                 315                 320

Tyr Gly Asp Trp Trp Asn Cys Val Ser Phe Glu Glu Phe Ser Arg Ile
                325                 330                 335

Trp Asn Val Pro Val His Lys Phe Leu Leu Arg His Val Tyr His Ser
            340                 345                 350

Ser Met Gly Ala Leu His Leu Ser Lys Ser Gln Ala Thr Leu Phe Thr
        355                 360                 365

Phe Phe Leu Ser Ala Val Phe His Glu Met Ala Met Phe Ala Ile Phe
    370                 375                 380

Arg Arg Val Arg Gly Tyr Leu Phe Met Phe Gln Leu Ser Gln Phe Val
385                 390                 395                 400

Trp Thr Ala Leu Ser Asn Thr Lys Phe Leu Arg Ala Arg Pro Gln Leu
                405                 410                 415

Ser Asn Val Val Phe Ser Phe Gly Val Cys Ser Gly Pro
            420                 425
```

<210> 37
<211> 432
<212> PRT
<213> Saccharomyces cerevisiae

<400> 37

Glu Thr Val Val Thr Val Glu Thr Thr Ile Ile Ser Ser Asn Phe Ser
1 5 10 15

Gly Leu Tyr Val Ala Phe Trp Met Ala Ile Ala Phe Gly Ala Val Lys
20 25 30

Ala Leu Ile Asp Tyr Tyr Tyr Gln His Asn Gly Ser Phe Lys Asp Ser
35 40 45

Glu Ile Leu Lys Phe Met Thr Thr Asn Leu Phe Thr Val Ala Ser Val
50 55 60

Asp Leu Leu Met Tyr Leu Ser Thr Tyr Phe Val Val Gly Ile Gln Tyr
65 70 75 80

Leu Cys Lys Trp Gly Val Leu Lys Trp Gly Thr Thr Gly Trp Ile Phe
85 90 95

```
Thr Ser Ile Tyr Glu Phe Leu Phe Val Ile Phe Tyr Met Tyr Leu Thr
            100                 105                 110

Glu Asn Ile Leu Lys Leu His Trp Leu Ser Lys Ile Phe Leu Phe Leu
        115                 120                 125

His Ser Leu Val Leu Leu Met Lys Met His Ser Phe Ala Phe Tyr Asn
    130                 135                 140

Gly Tyr Leu Trp Gly Ile Lys Glu Glu Leu Gln Phe Ser Lys Ser Ala
145                 150                 155                 160

Leu Ala Lys Tyr Lys Asp Ser Ile Asn Asp Pro Lys Val Ile Gly Ala
                165                 170                 175

Leu Glu Lys Ser Cys Glu Phe Cys Ser Phe Glu Leu Ser Ser Gln Ser
            180                 185                 190

Leu Ser Asp Gln Thr Gln Lys Phe Pro Asn Asn Ile Ser Ala Lys Ser
        195                 200                 205

Phe Phe Trp Phe Thr Met Phe Pro Thr Leu Ile Tyr Gln Ile Glu Tyr
    210                 215                 220

Pro Arg Thr Lys Glu Ile Arg Trp Ser Tyr Val Leu Glu Lys Ile Cys
225                 230                 235                 240

Ala Ile Phe Gly Thr Ile Phe Leu Met Met Ile Asp Ala Gln Ile Leu
                245                 250                 255

Met Tyr Pro Val Ala Met Arg Ala Leu Ala Val Arg Asn Ser Glu Trp
            260                 265                 270

Thr Gly Ile Leu Asp Arg Leu Leu Lys Trp Val Gly Leu Leu Val Asp
        275                 280                 285

Ile Val Pro Gly Phe Ile Val Met Tyr Ile Leu Asp Phe Tyr Leu Ile
    290                 295                 300

Trp Asp Ala Ile Leu Asn Cys Val Ala Glu Leu Thr Arg Phe Gly Asp
305                 310                 315                 320

Arg Tyr Phe Tyr Gly Asp Trp Trp Asn Cys Val Ser Trp Ala Asp Phe
                325                 330                 335

Ser Arg Ile Trp Asn Ile Pro Val His Lys Phe Leu Leu Arg His Val
            340                 345                 350

Tyr His Ser Ser Met Ser Ser Phe Lys Leu Asn Lys Ser Gln Ala Thr
        355                 360                 365

Leu Met Thr Phe Phe Leu Ser Ser Val Val His Glu Leu Ala Met Tyr
    370                 375                 380

Val Ile Phe Lys Lys Leu Arg Phe Tyr Leu Phe Phe Phe Gln Met Leu
385                 390                 395                 400

Gln Met Pro Leu Val Ala Leu Thr Asn Thr Lys Phe Met Arg Asn Arg
                405                 410                 415

Thr Ile Ile Gly Asn Val Ile Phe Trp Leu Gly Ile Cys Met Gly Pro
            420                 425                 430
```

<210> 38
<211> 1942
<212> DNA
<213> Arabidopsis thaliana

<400> 38

```
ctctcgtgaa tcctttttcc tttcttcttc ttcttctctt cagagaaaac tttgcttctc 60
tttctataag gaaccagaca cgaatcccat tcccaccgat ttcttagctt cttccttcaa 120
tccgctcttt ccctctccat tagattctgt ttcctctttc aatttcttct gcatgcttct 180
cgattctctc tgacgcctct tttctcccga cgctgtttcg tcaaacgctt ttcgaaatgg 240
cgattttgga ttctgctggc gttactacgg tgacggagaa cggtggcgga gagttcgtcg 300
atcttgatag gcttcgtcga cggaaatcga gatcggattc ttctaacgga cttcttctct 360
ctggttccga taataattct ccttcggatg atgttggagc tcccgccgac gttagggatc 420
ggattgattc cgttgttaac gatgacgctc agggaacagc caatttggcc ggagataata 480
acggtggtgg cgataataac ggtggtggaa gaggcggcgg agaaggaaga ggaaacgccg 540
atgctacgtt tacgtatcga ccgtcggttc cagctcatcg gagggcgaga gagagtccac 600
ttagctccga cgcaatcttc aaacagagcc atgccggatt attcaacctc tgtgtagtag 660
ttcttattgc tgtaaacagt agactcatca tcgaaaatct tatgaagtat ggttggttga 720
tcagaacgga tttctggttt agttcaagat cgctgcgaga ttggccgctt ttcatgtgtt 780
gtatatccct ttcgatcttt cctttggctg cctttacggt tgagaaattg gtacttcaga 840
aatacatatc agaacctgtt gtcatctttc ttcatattat tatcaccatg acagaggttt 900
tgtatccagt ttacgtcacc ctaaggtgtg attctgcttt tttatcaggt gtcactttga 960
tgctcctcac ttgcattgtg tggctaaagt tggtttctta tgctcatact agctatgaca 1020
taagatccct agccaatgca gctgataagg ccaatcctga agtctcctac tacgttagct 1080
tgaagagctt ggcatatttc atggtcgctc ccacattgtg ttatcagcca agttatccac 1140
gttctgcatg tatacggaag ggttgggtgg ctcgtcaatt tgcaaaactg gtcatattca 1200
ccggattcat gggatttata atagaacaat atataaatcc tattgtcagg aactcaaagc 1260
atcctttgaa aggcgatctt ctatatgcta ttgaaagagt gttgaagctt tcagttccaa 1320
atttatatgt gtggctctgc atgttctact gcttcttcca cctttggtta aacatattgg 1380
cagagcttct ctgcttcggg gatcgtgaat tctacaaaga ttggtggaat gcaaaaagtg 1440
tgggagatta ctggagaatg tggaatatgc ctgttcataa atggatggtt cgacatatat 1500
acttcccgtg cttgcgcagc aagataccaa agacactcgc cattatcatt gctttcctag 1560
tctctgcagt ctttcatgag ctatgcatcg cagttccttg tcgtctcttc aagctatggg 1620
cttttcttgg gattatgttt caggtgcctt tggtcttcat cacaaactat ctacaggaaa 1680
ggtttggctc aacggtgggg aacatgatct tctggttcat cttctgcatt ttcggacaac 1740
cgatgtgtgt gcttctttat taccacgacc tgatgaaccg aaaaggatcg atgtcatgaa 1800
acaactgttc aaaaaatgac tttcttcaaa catctatggc ctcgttggat ctccgttgat 1860
gttgtggtgg ttctgatgct aaaacgacaa atagtgttat aaccattgaa gaagaaaaga 1920
caattagagt tgttgtatcg ca                                         1942
```

<210> 39
<211> 520
<212> PRT
<213> Arabidopsis thaliana

<400> 39

```
Met Ala Ile Leu Asp Ser Ala Gly Val Thr Thr Val Thr Glu Asn Gly
 1               5                  10                  15

Gly Gly Glu Phe Val Asp Leu Asp Arg Leu Arg Arg Arg Lys Ser Arg
            20                  25                  30

Ser Asp Ser Ser Asn Gly Leu Leu Leu Ser Gly Ser Asp Asn Asn Ser
        35                  40                  45

Pro Ser Asp Asp Val Gly Ala Pro Ala Asp Val Arg Asp Arg Ile Asp
    50                  55                  60

Ser Val Val Asn Asp Asp Ala Gln Gly Thr Ala Asn Leu Ala Gly Asp
65                  70                  75                  80
```

```
Asn Asn Gly Gly Gly Asp Asn Asn Gly Gly Gly Arg Gly Gly Gly Glu
                85                  90                  95

Gly Arg Gly Asn Ala Asp Ala Thr Phe Thr Tyr Arg Pro Ser Val Pro
            100                 105                 110

Ala His Arg Arg Ala Arg Glu Ser Pro Leu Ser Ser Asp Ala Ile Phe
        115                 120                 125

Lys Gln Ser His Ala Gly Leu Phe Asn Leu Cys Val Val Val Leu Ile
    130                 135                 140

Ala Val Asn Ser Arg Leu Ile Ile Glu Asn Leu Met Lys Tyr Gly Trp
145                 150                 155                 160

Leu Ile Arg Thr Asp Phe Trp Phe Ser Ser Arg Ser Leu Arg Asp Trp
                165                 170                 175

Pro Leu Phe Met Cys Cys Ile Ser Leu Ser Ile Phe Pro Leu Ala Ala
            180                 185                 190

Phe Thr Val Glu Lys Leu Val Leu Gln Lys Tyr Ile Ser Glu Pro Val
        195                 200                 205

Val Ile Phe Leu His Ile Ile Ile Thr Met Thr Glu Val Leu Tyr Pro
    210                 215                 220

Val Tyr Val Thr Leu Arg Cys Asp Ser Ala Phe Leu Ser Gly Val Thr
225                 230                 235                 240

Leu Met Leu Leu Thr Cys Ile Val Trp Leu Lys Leu Val Ser Tyr Ala
                245                 250                 255

His Thr Ser Tyr Asp Ile Arg Ser Leu Ala Asn Ala Ala Asp Lys Ala
            260                 265                 270

Asn Pro Glu Val Ser Tyr Tyr Val Ser Leu Lys Ser Leu Ala Tyr Phe
        275                 280                 285

Met Val Ala Pro Thr Leu Cys Tyr Gln Pro Ser Tyr Pro Arg Ser Ala
    290                 295                 300

Cys Ile Arg Lys Gly Trp Val Ala Arg Gln Phe Ala Lys Leu Val Ile
305                 310                 315                 320

Phe Thr Gly Phe Met Gly Phe Ile Ile Glu Gln Tyr Ile Asn Pro Ile
                325                 330                 335

Val Arg Asn Ser Lys His Pro Leu Lys Gly Asp Leu Leu Tyr Ala Ile
            340                 345                 350

Glu Arg Val Leu Lys Leu Ser Val Pro Asn Leu Tyr Val Trp Leu Cys
        355                 360                 365

Met Phe Tyr Cys Phe Phe His Leu Trp Leu Asn Ile Leu Ala Glu Leu
    370                 375                 380

Leu Cys Phe Gly Asp Arg Glu Phe Tyr Lys Asp Trp Trp Asn Ala Lys
385                 390                 395                 400

Ser Val Gly Asp Tyr Trp Arg Met Trp Asn Met Pro Val His Lys Trp
                405                 410                 415
```

```
Met Val Arg His Ile Tyr Phe Pro Cys Leu Arg Ser Lys Ile Pro Lys
            420                 425                 430

Thr Leu Ala Ile Ile Ile Ala Phe Leu Val Ser Ala Val Phe His Glu
        435                 440                 445

Leu Cys Ile Ala Val Pro Cys Arg Leu Phe Lys Leu Trp Ala Phe Leu
    450                 455                 460

Gly Ile Met Phe Gln Val Pro Leu Val Phe Ile Thr Asn Tyr Leu Gln
465                 470                 475                 480

Glu Arg Phe Gly Ser Thr Val Gly Asn Met Ile Phe Trp Phe Ile Phe
                485                 490                 495

Cys Ile Phe Gly Gln Pro Met Cys Val Leu Leu Tyr Tyr His Asp Leu
            500                 505                 510

Met Asn Arg Lys Gly Ser Met Ser
        515                 520
```

<210> 40
<211> 29
<212> DNA
<213> Artificial Sequence

<220>
<223> Description of Artificial Sequence: Synthetic oligonucleotide primer

<400> 40
tgcaaattga cgagcacacc aaccccttc 29

<210> 41
<211> 28
<212> DNA
<213> Artificial Sequence

<220>
<223> Description of Artificial Sequence: Synthetic oligonuclotide primer

<400> 41
aaggatgctt tgagttcctg acaatagg 28

<210> 42
<211> 1942
<212> DNA
<213> Arabidopsis thaliana

<400> 42

ctctcgtgaa tcctttttcc tttcttcttc ttcttctctt cagagaaaac tttgcttctc 60
tttctataag gaaccagaca cgaatcccat tcccaccgat ttcttagctt cttccttcaa 120
tccgctcttt ccctctccat tagattctgt ttcctctttc aatttcttct gcatgcttct 180
cgattctctc tgacgcctct tttctcccga cgctgtttcg tcaaacgctt ttcgaaatgg 240
cgattttgga ttctgctggc gttactacgg tgacggagaa cggtggcgga gagttcgtcg 300
atcttgatag gcttcgtcga cggaaatcga gatcggattc ttctaacgga cttcttctct 360
ctggttccga taataattct ccttcggatg atgttggagc tcccgccgac gttagggatc 420
ggattgattc cgttgttaac gatgacgctc agggaacagc caatttggcc ggagataata 480

acggtggtgg cgataataac ggtggtggaa gaggcggcgg agaaggaaga ggaaacgccg 540
atgctacgtt tacgtatcga ccgtcggttc cagctcatcg gagggcgaga gagagtccac 600
ttagctccga cgcaatcttc aaacagagcc atgccggatt attcaacctc tgtgtagtag 660
ttcttattgc tgtaaacagt agactcatca tcgaaaatct tatgaagtat ggttggttga 720
tcagaacgga tttctggttt agttcaagat cgctgcgaga ttggccgctt ttcatgtgtt 780
gtatatccct ttcgatcttt cctttggctg cctttacggt tgagaaattg gtacttcaga 840
aatacatatc agaacctgtt gtcatctttc ttcatattat tatcaccatg acagaggttt 900
tgtatccagt ttacgtcacc ctaaggtgtg attctgcttt tttatcaggt gtcactttga 960
tgctcctcac ttgcattgtg tggctaaagt tggtttctta tgctcatact agctatgaca 1020
taagatccct agccaatgca gctgataagg ccaatcctga agtctcctac tacgttagct 1080
tgaagagctt ggcatatttc atggtcgctc ccacattgtg ttatcagcca agttatccac 1140
gttctgcatg tatacggaag ggttgggtgg ctcgtcaatt tgcaaaactg gtcatattca 1200
ccggattcat gggatttata atagaacaat atataaatcc tattgtcagg aactcaaagc 1260
atcctttgaa aggcgatctt ctatatgcta ttgaaagagt gttgaagctt tcagttccaa 1320
atttatatgt gtggctctgc atgttctact gcttcttcca cctttggtta aacatattgg 1380
cagagcttct ctgcttcggg gatcgtgaat tctacaaaga ttggtggaat gcaaaaagtg 1440
tgggagatta ctggagaatg tggaatatgc ctgttcataa atggatggtt cgacatatat 1500
acttcccgtg cttgcgcagc aagataccaa agacactcgc cattatcatt gctttcctag 1560
tctctgcagt ctttcatgag ctatgcatcg cagttccttg tcgtctcttc aagctatggg 1620
cttttcttgg gattatgttt caggtgcctt tggtcttcat cacaaactat ctacaggaaa 1680
ggtttggctc aacggtgggg aacatgatct tctggttcat cttctgcatt ttcggacaac 1740
cgatgtgtgt gcttctttat taccacgacc tgatgaaccg aaaaggatcg atgtcatgaa 1800
acaactgttc aaaaaatgac tttcttcaaa catctatggc ctcgttggat ctccgttgat 1860
gttgtggtgg ttctgatgct aaaacgacaa atagtgttat aaccattgaa gaagaaaaga 1920
caattagagt tgttgtatcg ca 1942

<210> 43
<211> 234
<212> DNA
<213> Glycine max

<220>
<221> unsure
<222> (1)..(234)
<223> n=unknown

<400> 43

gtaagcttca agagcttagc atanttcctg gttgccccta ncattatgtt accagccaan 60
ctatcctcgc acaccttata ttcgaaaggg ttggctgttt cgccaacttg tcaactgata 120
atatttacag gagttatggg atttataata gaacaataca ttaatcccat tgtacaaaat 180
tcacagcatc ctctcaaggg aaaccttctt tacgccatcg agagagttct gaag 234

<210> 44
<211> 267
<212> DNA
<213> Glycine max

<400> 44

```
ctgcttttgt atctggtgtc acgttgatgc tattaacttg cattgtgtgg ttaaaattgg 60
tgtcatatgc acatacaaac tatgatatga gagcacttac tgtttcgaat gaaaagggag 120
aaacattacc caatactttg atatggagta tccgtacact gtgaccttca ggagtttggc 180
atacttcatg gttgctccta cattatgcta tcagacaagc tatcctcgca caccttcagt 240
tcgaaagggt tgggtgtttc gtcaact                                    267
```

<210> 45
<211> 275
<212> DNA
<213> Glycine max

<220>
<221> unsure
<222> (1)..(275)
<223> n=unknown

<400> 45

```
gtggaatgcc aaaactgttg aagattattg gaggatgtgg aatatgcctg ttcacaaatg 60
gatgatccgc cacctatatt ttccatgttt aaggcacggt ataccaaagg ccgttgctct 120
tttaattgcc ttcctggttc tgctttattc catgagctgt gcatcgctgt tccttgccca 180
catattcaag tngtgggttt cngnggaatt nagtttcagg tnccttgggt ttcnaccnna 240
attnntnggc naaaaaattc cnngaacccc ggggg                           275
```

<210> 46
<211> 257
<212> DNA
<213> Glycine max

<400> 46

```
aacggaattg agactccaga gaatatgcca aaatgtatta ataattgtca caacttggaa 60
ggcttttgga aaaactggca tgcttccttc aacaagtggc ttgtgaggta tatatacatt 120
cctcttgggg gatctaagaa aaagctacta aatgtgtggg ttgttttcac atttgttgca 180
atctggcatg atttagagtg gaagcttctt tcatgggcat ggttgacgtg tttattcttc 240
atccctgagt tggtttt                                               257
```

<210> 47
<211> 253
<212> DNA
<213> Zea mays

<400> 47

```
agaaaatgga acatgcctgt gcataaatgg attgttcgtc atatatattt tccttgcatg 60
cgaaatggta tatcaaagga agttgctgtt tttatatcgt tcttgtttct gctgtacttc 120
atgagttatg tgttgctgtt ccctgccaca tactcaagtt ctgggctttt tttaggaatc 180
atgcttcaga ttcccctcat catattgaca tcatacctca aaaataaatt cagtgacaca 240
atggttggca ata                                                   253
```

<210> 48
<211> 254

<212> DHA
<213> Zea mays

<400> 48

```
tgaagtatgg cttattaata agatctggct tttggtttaa tgctacatca ttgcgagact 60
ggccactgct aatgtgttgc cttagtctac ccatatttcc ccttggtgca tttgcagtcg 120
aaaagttggc attcaacaat ctcattagtg atcctgctac tacctgtttt cacatccttt 180
ttacaacatt tgaaattgta tatccagtgc tcgtgattct taagtgtgat tctgcagttt 240
tatcaggctt tgtg                                                   254
```

<210> 49
<211> 262
<212> DNA
<213> Zea mays

<400> 49

```
gaagtatggc ttattaataa gatctggctt ttggtttaat gctacatcat tgcgagactg 60
gccactgcta atgtgttgcc ttagtctacc catatttccc cttggtgcat ttgcagtcga 120
aaagttggca ttcaacaatc tcattagtga tcctgctact acctgttttc acatcctttt 180
tacaacattt gaaattgtat atccagtgct cgtgattctt aagtgtgatt ctgcagtttt 240
acaggctttg tgttgatgtt ta                                         262
```

<210> 50
<211> 325
<212> DNA
<213> Zea mays

<220>
<221> unsure
<222> (1) .. (325)
<223> n=unknown

<400> 50

```
taatcnaacc tcgntncngg ttcagctgta tnccatgaga tatgtaatgc ggtgccgtgc 60
cacatantca natctnggca tnncngggat catngttcag ataccgntgg nattcttgac 120
aagatatctc catgctacgt tcaagcatgt aatggtgggc aacatgatan tttggntctn 180
cagtatagtc ggacagccga tgtnnnnnna tctatactac catgacgtca tgaacaggca 240
ggcccaggca agtagatagt ncggcagaga catgtacttc aacatcganc atcagnagca 300
nacngagcga gcggcangaa ncagc                                      325
```

<210> 51
<211> 519
<212> DNA
<213> Mortierrella alpina

<220>
<221> unsure
<222> (1)..(519)
<223> n=unknown

<400> 51

```
gagnnnngna acgtttagcc tnccgtagcc gccaaaatcc aagggncnac cnaccctncg 60
ttanactnaa ttngaaaatn cnnncccaac ttnaggnact tnnagncccc ccnacttgac 120
aacggagcac tatatttacc ccgtggtngt tcaacccagc catctcaccc ttgcgagcat 180
tggtgctgct cttgataccc ttcatgctta actatctcat gatcttttac atcattttcg 240
agtgcatctg caacgccttt gcggaactaa gttgctttgc ggatcgcaac ttttacgagg 300
attggtggaa ctgcgtcagc tttgatgagt gggcacgcaa atggaacaag cctgtgcaac 360
acttcttgct ccgccacgtg tacgactcga gcatccgagt ccttccactt gtccgaaatc 420
caatgccgcn aattgcaaac gttccttccc ggtcgtcaat gcgttcaacg aacctgggtg 480
aagaatgggt ggtgacaacg ttaaagtgcg cccggtatc                        519
```

<210> 52
<211> 45
<212> DNA
<213> Artificial Sequence

<220>
<223> Description of Artificial Sequence: Oligonucleotide primer

<400> 52
ggatccgcgg ccgcacaatg aaaaaaatat cttcacatta ttcgg 45

<210> 53
<211> 40
<212> DNA
<213> Artificial Sequence

<220>
<223> Description of Artificial Sequence: Oligonucleotide primer

<400> 53
ggatcccctg caggtcattc attgacggca ttaacattgg 40

<210> 54
<211> 44
<212> DNA
<213> Artificial Sequence

<220>
<223> Description of Artificial Sequence: Synthetic oligonucleotide primer

<400> 54
ggatccgcgg ccgcacaatg ggagcgaatt cgaaatcagt aacg 44

<210> 55
<211> 40
<212> DNA
<213> Artificial Sequence

<220>
<223> Description of Artificial Sequence: Synthetic oligonucleotide primer

<400> 55
ggatcccctg caggttaata cccactttta tcaagctccc 40

<210> 56
<211> 41
<212> DNA

<213> Artificial Sequence

<220>
<223> Description of Artificial Sequence: Synthetic oligonucleotide primer

<400> 56
ggatccgcgg ccgcacaatg tctctattac tggaagagat c 41

<210> 57
<211> 41
<212> DNA
<213> Artificial Sequence

<220>
<223> Description of Artificial Sequence: Synthetic oligonucleotide primer

<400> 57
ggatcccctg caggttatgc atcaacagag acacttacag c 41

<210> 58
<211> 41
<212> DNA
<213> Artificial Sequence

<220>
<223> Description of Artificial Sequence: Synthetic oligonucleotide primer

<400> 58
ggatccgcgg ccgcacaatg ggctggattc cgtgtccgtg c 41

<210> 59
<211> 38
<212> DNA
<213> Artificial Sequence

<220>
<223> Description of Artificial Sequence: Synthetic oligonucleotide primer

<400> 59
ggatcccctg caggttaacc agaatcaact actttgtg 38

<210> 60
<211> 39
<212> DNA
<213> Artificial Sequence

<220>
<223> Description of Artificial Sequence: Oligonucleotide primer

<400> 60
tcgacctgca ggaagcttag aaatggcgat tttggattc 39

<210> 61
<211> 36
<212> DNA
<213> Artificial Sequence

<220>

<223> Description of Artificial Sequence: Oligonucleotide primer

<400> 61
ggatccgcgg ccgctcatga catcgatcct tttcgg 36

<210> 62
<211> 56
<212> DNA
<213> Artificial Sequence

<220>
<223> Description of Artificial Sequence: Annealed oligonucleotide adapter

<400> 62
cgcgatttaa atggcgcgcc ctgcaggcgg ccgcctgcag ggcgcgccat ttaaat 56

<210> 63
<211> 32
<212> DNA
<213> Artificial Sequence

<220>
<223> Description of Artificial Sequence: Ligating oligonucleotide

<400> 63
tcgaggatcc gcggccgcaa gcttcctgca gg 32

<210> 64
<211> 32
<212> DNA
<213> Artificial Sequence

<220>
<223> Description of Artificial Sequence: Ligating oligonucleotide

<400> 64
tcgacctgca ggaagcttgc ggccgcggat cc 32

<210> 65
<211> 32
<212> DNA
<213> Artificial Sequence

<220>
<223> Description of Artificial Sequence: Ligating oligonucleotide

<400> 65
tcgacctgca ggaagcttgc ggccgcggat cc 32

<210> 66
<211> 32
<212> DNA
<213> Artificial Sequence

<220>
<223> Description of Artificial Sequence: Ligating oligonucleotide

<400> 66
tcgaggatcc gcggccgcaa gcttcctgca gg 32

<210> 67
<211> 36
<212> DNA
<213> Artificial Sequence

<220>
<223> Description of Artificial Sequence: Ligating oligonucleotide

<400> 67
tcgaggatcc gcggccgcaa gcttcctgca ggagct 36

<210> 68
<211> 28
<212> DNA
<213> Artificial Sequence

<220>
<223> Description of Artificial Sequence: Ligating oligonucleotide

<400> 68
cctgcaggaa gcttgcggcc gcggatcc 28

<210> 69
<211> 36
<212> DNA
<213> Artificial Sequence

<220>
<223> Description of Artificial Sequence: Ligating oligonucleotide

<400> 69
tcgacctgca ggaagcttgc ggccgcggat ccagct 36

<210> 70
<211> 28
<212> DNA
<213> Artificial Sequence

<220>
<223> Description of Artificial Sequence: Ligating oligonucleotide

<400> 70
ggatccgcgg ccgcaagctt cctgcagg 28

<210> 71
<211> 39
<212> DNA
<213> Artificial Sequence

<220>
<223> Description of Artificial Sequence: Ligating oligonucleotide

<400> 71
gatcacctgc aggaagcttg cggccgcgga tccaatgca 39

<210> 72
<211> 31
<212> DNA
<213> Artificial Sequence

<220>
<223> Description of Artificial Sequence: Ligating oligonucleotide

<400> 72
ttggatccgc ggccgcaagc ttcctgcagg t 31

<210> 73
<211> 2013
<212> DNA
<213> Arabidopsis thaliana

<400> 73

```
atgcccctta ttcatcggaa aaagccgacg gagaaaccat cgacgccgcc atctgaagag 60
gtggtgcacg atgaggattc gcaaaagaaa ccacacgaat cttccaaatc ccaccataag 120
aaatcgaacg gaggagggaa gtggtcgtgc atcgattctt gttgttggtt cattgggtgt 180
gtgtgtgtaa cctggtggtt tcttctcttc ctttacaacg caatgcctgc gagcttccct 240
cagtatgtaa cggagcgaat cacgggtcct ttgcctgacc cgcccggtgt taagctcaaa 300
aaagaaggtc ttaaggcgaa acatcctgtt gtcttcattc ctgggattgt caccggtggg 360
ctcgagcttt gggaaggcaa acaatgcgct gatggtttat ttagaaaacg tttgtggggt 420
ggaacttttg gtgaagtcta caaaaggcct ctatgttggg tggaacacat gtcacttgac 480
aatgaaactg ggttggatcc agctggtatt agagttcgag ctgtatcagg actcgtggct 540
gctgactact ttgctcctgg ctactttgtc tgggcagtgc tgattgctaa ccttgcacat 600
attggatatg aagagaaaaa tatgtacatg gctgcatatg actggcggct ttcgtttcag 660
aacacagagg tacgtgatca gactcttagc cgtatgaaaa gtaatataga gttgatggtt 720
tctaccaacg gtggaaaaaa agcagttata gttccgcatt ccatgggggt cttgtatttt 780
ctacatttta tgaagtgggt tgaggcacca gctcctctgg gtggcggggg tgggccagat 840
tggtgtgcaa agtatattaa ggcggtgatg aacattggtg gaccatttct tggtgttcca 900
aaagctgttg cagggctttt ctctgctgaa gcaaaggatg ttgcagttgc cagagcgatt 960
gccccaggat tcttagacac cgatatattt agacttcaga ccttgcagca tgtaatgaga 1020
atgacacgca catgggactc aacaatgtct atgttaccga agggaggtga cacgatatgg 1080
ggcgggcttg attggtcacc ggagaaaggc cacacctgtt gtgggaaaaa gcaaaagaac 1140
aacgaaactt gtggtgaagc aggtgaaaac ggagtttcca agaaaagtcc tgttaactat 1200
ggaaggatga tatcttttgg gaaagaagta gcagaggctg cgccatctga gattaataat 1260
attgatttc gaggtgctgt caaaggtcag agtatcccaa atcacacctg tcgtgacgtg 1320
tggacagagt accatgacat gggaattgct gggatcaaag ctatcgctga gtataaggtc 1380
tacactgctg gtgaagctat agatctacta cattatgttg ctcctaagat gatggcgcgt 1440
ggtgccgctc atttctctta tggaattgct gatgatttgg atgacaccaa gtatcaagat 1500
cccaaatact ggtcaaatcc gttagagaca aaattaccga atgctcctga gatggaaatc 1560
tactcattat acggagtggg gataccaacg gaacgagcat acgtatacaa gcttaaccag 1620
tctcccgaca gttgcatccc ctttcagata ttcacttctg ctcacgagga ggacgaagat 1680
agctgtctga aagcaggagt ttacaatgtg gatggggatg aaacagtacc cgtcctaagt 1740
gccgggtaca tgtgtgcaaa agcgtggcgt ggcaagacaa gattcaaccc ttccggaatc 1800
aagacttata taagagaata caatcactct ccgccggcta acctgttgga agggcgcggg 1860
acgcagagtg gtgcccatgt tgatatcatg ggaaactttg ctttgatcga agatatcatg 1920
agggttgccg ccggaggtaa cggtctgat ataggacatg accaggtcca ctctggcata 1980
tttgaatggt cggagcgtat tgacctgaag ctg                             2013
```

<210> 74
<211> 671
<212> PRT
<213> Arabidopsis thaliana

<400> 74

```
Met Pro Leu Ile His Arg Lys Lys Pro Thr Glu Lys Pro Ser Thr Pro
 1               5                  10                  15

Pro Ser Glu Glu Val Val His Asp Glu Asp Ser Gln Lys Lys Pro His
            20                  25                  30

Glu Ser Ser Lys Ser His His Lys Lys Ser Asn Gly Gly Gly Lys Trp
        35                  40                  45

Ser Cys Ile Asp Ser Cys Cys Trp Phe Ile Gly Cys Val Cys Val Thr
    50                  55                  60

Trp Trp Phe Leu Leu Phe Leu Tyr Asn Ala Met Pro Ala Ser Phe Pro
```

```
65                  70                  75                  80
Gln Tyr Val Thr Glu Arg Ile Thr Gly Pro Leu Pro Asp Pro Pro Gly
                85                  90                  95
Val Lys Leu Lys Lys Glu Gly Leu Lys Ala Lys His Pro Val Val Phe
            100                 105                 110
Ile Pro Gly Ile Val Thr Gly Gly Leu Glu Leu Trp Glu Gly Lys Gln
        115                 120                 125
Cys Ala Asp Gly Leu Phe Arg Lys Arg Leu Trp Gly Gly Thr Phe Gly
    130                 135                 140
Glu Val Tyr Lys Arg Pro Leu Cys Trp Val Glu His Met Ser Leu Asp
145                 150                 155                 160
Asn Glu Thr Gly Leu Asp Pro Ala Gly Ile Arg Val Arg Ala Val Ser
                165                 170                 175
Gly Leu Val Ala Ala Asp Tyr Phe Ala Pro Gly Tyr Phe Val Trp Ala
            180                 185                 190
Val Leu Ile Ala Asn Leu Ala His Ile Gly Tyr Glu Glu Lys Asn Met
        195                 200                 205
Tyr Met Ala Ala Tyr Asp Trp Arg Leu Ser Phe Gln Asn Thr Glu Val
    210                 215                 220
Arg Asp Gln Thr Leu Ser Arg Met Lys Ser Asn Ile Glu Leu Met Val
225                 230                 235                 240
Ser Thr Asn Gly Gly Lys Lys Ala Val Ile Val Pro His Ser Met Gly
                245                 250                 255
Val Leu Tyr Phe Leu His Phe Met Lys Trp Val Glu Ala Pro Ala Pro
            260                 265                 270
Leu Gly Gly Gly Gly Gly Pro Asp Trp Cys Ala Lys Tyr Ile Lys Ala
        275                 280                 285
Val Met Asn Ile Gly Gly Pro Phe Leu Gly Val Pro Lys Ala Val Ala
    290                 295                 300
Gly Leu Phe Ser Ala Glu Ala Lys Asp Val Ala Val Ala Arg Ala Ile
305                 310                 315                 320
Ala Pro Gly Phe Leu Asp Thr Asp Ile Phe Arg Leu Gln Thr Leu Gln
                325                 330                 335
His Val Met Arg Met Thr Arg Thr Trp Asp Ser Thr Met Ser Met Leu
            340                 345                 350
Pro Lys Gly Gly Asp Thr Ile Trp Gly Gly Leu Asp Trp Ser Pro Glu
        355                 360                 365
Lys Gly His Thr Cys Cys Gly Lys Lys Gln Lys Asn Asn Glu Thr Cys
    370                 375                 380
Gly Glu Ala Gly Glu Asn Gly Val Ser Lys Lys Ser Pro Val Asn Tyr
385                 390                 395                 400
Gly Arg Met Ile Ser Phe Gly Lys Glu Val Ala Glu Ala Ala Pro Ser
```

```
                405                     410                     415

Glu Ile Asn Asn Ile Asp Phe Arg Gly Ala Val Lys Gly Gln Ser Ile
            420                     425                     430

Pro Asn His Thr Cys Arg Asp Val Trp Thr Glu Tyr His Asp Met Gly
        435                     440                     445

Ile Ala Gly Ile Lys Ala Ile Ala Glu Tyr Lys Val Tyr Thr Ala Gly
    450                     455                     460

Glu Ala Ile Asp Leu Leu His Tyr Val Ala Pro Lys Met Met Ala Arg
465                     470                     475                     480

Gly Ala Ala His Phe Ser Tyr Gly Ile Ala Asp Asp Leu Asp Asp Thr
                485                     490                     495

Lys Tyr Gln Asp Pro Lys Tyr Trp Ser Asn Pro Leu Glu Thr Lys Leu
            500                     505                     510

Pro Asn Ala Pro Glu Met Glu Ile Tyr Ser Leu Tyr Gly Val Gly Ile
        515                     520                     525

Pro Thr Glu Arg Ala Tyr Val Tyr Lys Leu Asn Gln Ser Pro Asp Ser
    530                     535                     540

Cys Ile Pro Phe Gln Ile Phe Thr Ser Ala His Glu Glu Asp Glu Asp
545                     550                     555                     560

Ser Cys Leu Lys Ala Gly Val Tyr Asn Val Asp Gly Asp Glu Thr Val
                565                     570                     575

Pro Val Leu Ser Ala Gly Tyr Met Cys Ala Lys Ala Trp Arg Gly Lys
            580                     585                     590

Thr Arg Phe Asn Pro Ser Gly Ile Lys Thr Tyr Ile Arg Glu Tyr Asn
        595                     600                     605

His Ser Pro Pro Ala Asn Leu Leu Glu Gly Arg Gly Thr Gln Ser Gly
    610                     615                     620

Ala His Val Asp Ile Met Gly Asn Phe Ala Leu Ile Glu Asp Ile Met
625                     630                     635                     640

Arg Val Ala Ala Gly Gly Asn Gly Ser Asp Ile Gly His Asp Gln Val
                645                     650                     655

His Ser Gly Ile Phe Glu Trp Ser Glu Arg Ile Asp Leu Lys Leu
            660                     665                     670
```

<210> 75
<211> 1986
<212> DNA
<213> Saccharomyces cerevisiae

<400> 75

```
atgggcacac tgtttcgaag aaatgtccag aaccaaaaga gtgattctga tgaaaacaat 60
aaaggggggtt ctgttcataa caagcgagag agcagaaacc acattcatca tcaacaggga 120
ttaggccata agagaagaag gggtattagt ggcagtgcaa aaagaaatga gcgtggcaaa 180
gatttcgaca ggaaaagaga cgggaacggt agaaaacgtt ggagagattc cagaagactg 240
attttcattc ttggtgcatt cttaggtgta cttttgccgt ttagctttgg cgcttatcat 300
gttcataata gcgatagcga cttgtttgac aactttgtaa attttgattc acttaaagtg 360
```

```
tatttggatg attggaaaga tgttctccca caaggtataa gttcgtttat tgatgatatt 420
caggctggta actactccac atcttcttta gatgatctca gtgaaaattt tgccgttggt 480
aaacaactct tacgtgatta taatatcgag gccaaacatc ctgttgtaat ggttcctggt 540
gtcatttcta cgggaattga aagctgggga gttattggag acgatgagtg cgatagttct 600
gcgcattttc gtaaacggct gtggggaagt ttttacatgc tgagaacaat ggttatggat 660
aaagtttgtt ggttgaaaca tgtaatgtta gatcctgaaa caggtctgga cccaccgaac 720
tttacgctac gtgcagcaca gggcttcgaa tcaactgatt atttcatcgc agggtattgg 780
atttggaaca aagttttcca aaatctggga gtaattggct atgaacccaa taaaatgacg 840
agtgctgcgt atgattggag gcttgcatat ttagatctag aaagacgcga taggtacttt 900
acgaagctaa aggaacaaat cgaactgttt catcaattga gtggtgaaaa agtttgttta 960
attggacatt ctatgggttc tcagattatc ttttacttta tgaaatgggt cgaggctgaa 1020
ggccctcttt acggtaatgg tggtcgtggc tgggttaacg aacacataga ttcattcatt 1080
aatgcagcag ggacgcttct gggcgctcca aaggcagttc cagctctaat tagtggtgaa 1140
atgaaagata ccattcaatt aaatacgtta gccatgtatg gtttggaaaa gttcttctca 1200
agaattgaga gagtaaaaat gttacaaacg tggggtggta taccatcaat gctaccaaag 1260
ggagaagagg tcatttgggg ggatatgaag tcatcttcag aggatgcatt gaataacaac 1320
actgacacat acggcaattt cattcgattt gaaaggaata cgagcgatgc tttcaacaaa 1380
aatttgacaa tgaaagacgc cattaacatg acattatcga tatcacctga atggctccaa 1440
agaagagtac atgagcagta ctcgttcggc tattccaaga atgaagaaga gttaagaaaa 1500
aatgagctac accacaagca ctggtcgaat ccaatggaag taccacttcc agaagctccc 1560
cacatgaaaa tctattgtat atacggggtg aacaacccaa ctgaaagggc atatgtatat 1620
aaggaagagg atgactcctc tgctctgaat ttgaccatcg actacgaaag caagcaacct 1680
gtattcctca ccgagggggga cggaaccgtt ccgctcgtgg cgcattcaat gtgtcacaaa 1740
tgggcccagg gtgcttcacc gtacaaccct gccggaatta acgttactat tgtggaaatg 1800
aaacaccagc cagatcgatt tgatatacgt ggtggagcaa aaagcgccga acacgtagac 1860
atcctcggca gcgcggagtt gaacgattac atcttgaaaa ttgcaagcgg taatggcgat 1920
ctcgtcgagc cacgccaatt gtctaatttg agccagtggg tttctcagat gcccttccca 1980
atgtaa                                                            1986
```

<210> 76
<211> 661
<212> PRT
<213> Saccharomyces cerevisiae

<400> 76

```
Met Gly Thr Leu Phe Arg Arg Asn Val Gln Asn Gln Lys Ser Asp Ser
 1               5                  10                  15

Asp Glu Asn Asn Lys Gly Gly Ser Val His Asn Lys Arg Glu Ser Arg
            20                  25                  30

Asn His Ile His His Gln Gln Gly Leu Gly His Lys Arg Arg Arg Gly
        35                  40                  45

Ile Ser Gly Ser Ala Lys Arg Asn Glu Arg Gly Lys Asp Phe Asp Arg
    50                  55                  60

Lys Arg Asp Gly Asn Gly Arg Lys Arg Trp Arg Asp Ser Arg Arg Leu
65                  70                  75                  80

Ile Phe Ile Leu Gly Ala Phe Leu Gly Val Leu Leu Pro Phe Ser Phe
                85                  90                  95

Gly Ala Tyr His Val His Asn Ser Asp Ser Asp Leu Phe Asp Asn Phe
            100                 105                 110

Val Asn Phe Asp Ser Leu Lys Val Tyr Leu Asp Asp Trp Lys Asp Val
        115                 120                 125

Leu Pro Gln Gly Ile Ser Ser Phe Ile Asp Asp Ile Gln Ala Gly Asn

    130                 135                 140
```

Tyr Ser Thr Ser Ser Leu Asp Asp Leu Ser Glu Asn Phe Ala Val Gly
145 150 155 160

Lys Gln Leu Leu Arg Asp Tyr Asn Ile Glu Ala Lys His Pro Val Val
165 170 175

Met Val Pro Gly Val Ile Ser Thr Gly Ile Glu Ser Trp Gly Val Ile
180 185 190

Gly Asp Asp Glu Cys Asp Ser Ser Ala His Phe Arg Lys Arg Leu Trp
195 200 205

Gly Ser Phe Tyr Met Leu Arg Thr Met Val Met Asp Lys Val Cys Trp
210 215 220

Leu Lys His Val Met Leu Asp Pro Glu Thr Gly Leu Asp Pro Pro Asn
225 230 235 240

Phe Thr Leu Arg Ala Ala Gln Gly Phe Glu Ser Thr Asp Tyr Phe Ile
245 250 255

Ala Gly Tyr Trp Ile Trp Asn Lys Val Phe Gln Asn Leu Gly Val Ile
260 265 270

Gly Tyr Glu Pro Asn Lys Met Thr Ser Ala Ala Tyr Asp Trp Arg Leu
275 280 285

Ala Tyr Leu Asp Leu Glu Arg Arg Asp Arg Tyr Phe Thr Lys Leu Lys
290 295 300

Glu Gln Ile Glu Leu Phe His Gln Leu Ser Gly Glu Lys Val Cys Leu
305 310 315 320

Ile Gly His Ser Met Gly Ser Gln Ile Ile Phe Tyr Phe Met Lys Trp
325 330 335

Val Glu Ala Glu Gly Pro Leu Tyr Gly Asn Gly Gly Arg Gly Trp Val
340 345 350

Asn Glu His Ile Asp Ser Phe Ile Asn Ala Ala Gly Thr Leu Leu Gly
355 360 365

Ala Pro Lys Ala Val Pro Ala Leu Ile Ser Gly Glu Met Lys Asp Thr
370 375 380

Ile Gln Leu Asn Thr Leu Ala Met Tyr Gly Leu Glu Lys Phe Phe Ser
385 390 395 400

Arg Ile Glu Arg Val Lys Met Leu Gln Thr Trp Gly Gly Ile Pro Ser
405 410 415

Met Leu Pro Lys Gly Glu Glu Val Ile Trp Gly Asp Met Lys Ser Ser
420 425 430

Ser Glu Asp Ala Leu Asn Asn Asn Thr Asp Thr Tyr Gly Asn Phe Ile
435 440 445

Arg Phe Glu Arg Asn Thr Ser Asp Ala Phe Asn Lys Asn Leu Thr Met
450 455 460

Lys Asp Ala Ile Asn Met Thr Leu Ser Ile Ser Pro Glu Trp Leu Gln
465 470 475 480

```
Arg Arg Val His Glu Gln Tyr Ser Phe Gly Tyr Ser Lys Asn Glu Glu
                485                 490                 495

Glu Leu Arg Lys Asn Glu Leu His His Lys His Trp Ser Asn Pro Met
            500                 505                 510

Glu Val Pro Leu Pro Glu Ala Pro His Met Lys Ile Tyr Cys Ile Tyr
        515                 520                 525

Gly Val Asn Asn Pro Thr Glu Arg Ala Tyr Val Tyr Lys Glu Glu Asp
    530                 535                 540

Asp Ser Ser Ala Leu Asn Leu Thr Ile Asp Tyr Glu Ser Lys Gln Pro
545                 550                 555                 560

Val Phe Leu Thr Glu Gly Asp Gly Thr Val Pro Leu Val Ala His Ser
                565                 570                 575

Met Cys His Lys Trp Ala Gln Gly Ala Ser Pro Tyr Asn Pro Ala Gly
            580                 585                 590

Ile Asn Val Thr Ile Val Glu Met Lys His Gln Pro Asp Arg Phe Asp
        595                 600                 605

Ile Arg Gly Gly Ala Lys Ser Ala Glu His Val Asp Ile Leu Gly Ser
    610                 615                 620

Ala Glu Leu Asn Asp Tyr Ile Leu Lys Ile Ala Ser Gly Asn Gly Asp
625                 630                 635                 640

Leu Val Glu Pro Arg Gln Leu Ser Asn Leu Ser Gln Trp Val Ser Gln
                645                 650                 655

Met Pro Phe Pro Met
            660
```

<210> 77
<211> 35
<212> DNA
<213> Artificial Sequence

<220>
<223> Description of Artificial Sequence: Synthetic oligonucleotide primer

<400> 77
ggatccgcgg ccgcacaatg ccccttattc atcgg 35

<210> 78
<211> 35
<212> DNA
<213> Artificial Sequence

<220>
<223> Description of Artificial Sequence: Synthetic oligonucleotide primer

<400> 78
ggatcccctg caggtcacag cttcaggtca atacg 35

<210> 79
<211> 37
<212> DNA
<213> Artificial Sequence

<220>
<223> Description of Artificial Sequence: Synthetic oligonucleotide primer

<400> 79
ggatccgcgg ccgcacaatg ggcacactct ttcgaag 37

<210> 80
<211> 39
<212> DNA
<213> Artificial Sequence

<220>
<223> Description of Artificial Sequence: Synthetic oligonucleotide primer

<400> 80
ggatcccctg caggttacat tgggcacact gtttcgaag 39

## Description

## BACKGROUND

Technical Field

The present invention is directed to plant acyltransferase-like nucleic acid and amino acid sequences and constructs, and methods related to their use in altering sterol composition and/or content, and oil composition and/or content in host cells and plants.

Related Art

Through the development of plant genetic engineering techniques, it is now possible to produce transgenic varieties of plant species to provide plants which have novel and desirable characteristics. For example, it is now possible to genetically engineer plants for tolerance to environmental stresses, such as resistance to pathogens and tolerance to herbicides. It is also possible to improve the nutritional characteristics of the plant, for example to provide improved fatty acid, carotenoid, sterol and tocopherol compositions. However, the number of useful nucleotide sequences for the engineering of such characteristics is thus far limited.

There is a need for improved means to obtain or manipulate compositions of sterols from biosynthetic or natural plant sources. The ability to increase sterol production or alter the sterol compositions in plants may provide for novel sources of sterols for use in human and animal nutrition.

Sterol biosynthesis branches from the farnesyl diphosphate intermediate in the isoprenoid pathway. Sterol biosynthesis occurs via a mevalonate dependent pathway in mammals and higher plants (Goodwin,(1981) Biosynthesis of Isoprenoid Compounds, vol 1 (Porter, J.W. & Spurgeon, S.L., eds) pp.443-480, John Wiley and Sons, New York), while in green algae sterol biosynthesis is thought to occur via a mevalonate independent pathway (Schwender, et al. (1997) Physiology, Biochemistry, and Molecular Biology of Plant Lipids, (Williams, J.P., Khan, M.U., and Lem, N.W., eds) pp. 180-182, Kluwer Academic Publishers, Norwell, MA).

The solubility characteristics of sterol esters suggests that this is the storage form of sterols (Chang, et al., (1997) Annu. Rev. Biochem., 66:613-638). Sterol O-acyltransferase enzymes such as acyl CoA:cholesterol acyltransferase (ACAT) and lecithin:cholesterol acyltransferase (LCAT) catalyze the formation of cholesterol esters, and thus are key to controlling the intracellular cholesterol storage. In yeast, it has been reported that overexpression of *LRO1,* a homolog of human LCAT, and phospholipid:diacylglycerol acyltransferase increased lipid synthesis (Oelkers et al., (2000) J. Biol. Chem., 26:15609-15612; Dahlqvist et al., (2000) Proc. Natl. Acad. Sci. USA, 97:6487-6492).

The characterization of various acyltransferase proteins is useful for the further study of plant sterol synthesis systems and for the development of novel and/or alternative sterol sources. Studies of plant mechanisms may provide means to further enhance, control, modify, or otherwise alter the sterol composition of plant cells. Furthermore, such alterations in sterol content and/or composition may provide a means for obtaining tolerance to stress and insect damage. Of particular interest are the nucleic acid sequences of genes encoding proteins which may be useful for applications in genetic engineering.

## SUMMARY OF THE INVENTION

The present invention is directed to lecithin:cholesterol acyltransferase-like polypeptides (also referred to herein as LCAT) and acyl CoA:cholesterol acyltransferase-like polypeptides (also referred to herein as ACAT). In particular the invention is related to polynucleotides encoding such sterol:acyltransferases, polypeptides encoded by such polynucleotides, and the use of such polynucleotides to alter sterol composition and oil production. The polynucleotides of the present invention include those derived from plant sources.

One aspect of the invention, therefore, is an isolated nucleic acid sequence encoding a plant lecithin:cholesterol acyltransferase-like polypeptide that is capable of utilizing lecithin as an acyl donor for acylation of sterols or glycerides to form esters, wherein the coding sequence comprises a polynucleotide selected from:

a) an isolated polynucleotide encoding the polypeptide of SEQ ID NO: 5 or SEQ ID NO: 5;
b) an isolated polynucleotide sequence comprising SEQ ID NO: 4;
c) an isolated polynucleotide having at least 95% sequence identity with SEQ ID NO: 4;
d) an isolated polynucleotide complementary to a polynucleotide of (a), (b) or (c); and
e) an isolated polynucleotide that hybridizes under stringent conditions to SEQ ID NO: 4.

Also provided is a recombinant nucleic acid construct comprising a regulatory sequence operably linked to a polynucleotide encoding a lecithin:cholesterol acyltransferase-like polypeptide and/or an acyl CoA:cholesterol acyltransferase-like polypeptide as defined above. In one embodiment, the sterol acyl transferases are plant sterol acyl transferases. In another embodiment, the recombinant nucleic acid constructs further comprises a termination sequence. The regulatory sequence can be a constitutive promoter, an inducible promoter, a developmentally regulated promoter, a tissue specific promoter, an organelle specific promoter, a seed specific promoter or a combination of any of the foregoing. Also provided is a plant containing this recombinant nucleic acid construct and the seed and progeny from such a plant. This recombinant nucleic acid construct can also be introduced into a suitable host cell to provide yet another aspect of the invention. If the host cell is a plant host cell, the cell can be used to generate a plant to provide another aspect of the invention. Further aspects include seed and progeny from such a plant. Yet another aspect is a purified polypeptide comprising SEQ ID NO: 5.

One aspect provides a method for producing a lecithin:cholesterol acyltransferase-like polypeptide or an acyl CoA:cholesterol acyltransferase-like polypeptide comprising culturing a host cell containing any recombinant nucleic acid construct of the present invention under condition permitting expression of said lecithin:cholesterol acyltransferase-like polypeptide or acyl CoA:cholesterol acyltransferase-like polypeptide.

Another aspect provides a method for modifying the sterol content of a host cell, comprising transforming a host cell with a recombinant construct containing a regulatory sequence operably linked to a polynucleotide encoding a lecithin:cholesterol acyltransferase-like polypeptide and culturing said host cell under conditions wherein said host cell expresses a lecithin:cholesterol acyltransferase-like polypeptide such that said host cell has a modified sterol composition as compared to host cells without the recombinant construct.

An additional aspect is a method for modifying the sterol content of a host cell comprising transforming a host cell with a recombinant construct containing a regulatory sequence operably linked to a polynucleotide encoding an acyl CoA:cholesterol acyltransferase-like polypeptide and culturing said host cell under conditions wherein said host cell expresses an acyl CoA:cholesterol acyltransferase-like polypeptide such that said host cell has a modified sterol composition as compared to host cells without the recombinant construct.

A further aspect is a plant comprising a recombinant construct containing a regulatory sequence operably linked to a polynucleotide encoding a lecithin:cholesterol acyltransferase-like polypeptide wherein expression of said recombinant construct results in modified sterol composition of said plant as compared to the same plant without said recombinant construct. Another aspect provides a plant comprising a recombinant construct containing a regulatory sequence operably linked to a polynucleotide encoding an acyl CoA:cholesterol acyltransferase-like polypeptide wherein expression of said recombinant construct results in modified sterol composition of said plant as compared to the same plant without said recombinant construct.

In still another aspect is provided a method for producing an oil with a modified sterol composition comprising providing any of the plants or host cells of the present invention and extracting oil from the plant by any known method. Also provided is an oil produced by the preceding method.

Still another aspect provides a method for altering oil production by a host cell comprising, transforming a host cell with a recombinant construct containing a regulatory sequence operably linked to a polynucleotide encoding a lecithin:cholesterol acyltransferase-like polypeptide and culturing the host cell under conditions wherein the host cell expresses a lecithin:cholesterol acyltransferase-like polypeptide such that the host cell has an altered oil production as compared to host cells without the recombinant construct.

Another aspect provides a method for altering oil production by a host cell comprising, transforming a host cell with a recombinant construct containing a regulatory sequence operably linked to a polynucleotide encoding an acyl CoA:cholesterol acyltransferase-like polypeptide and culturing the host cell under conditions wherein the host cell expresses an acyl CoA:cholesterol acyltransferase-like polypeptide such that the host cell has an altered oil production as compared to host cells without the recombinant construct.

Also provided is a plant comprising a recombinant construct containing a regulatory sequence operably linked to a polynucleotide encoding a lecithin:cholesterol acyltransferase-like polypeptide wherein expression of said recombinant construct results in an altered production of oil by said plant as compared to the same plant without said recombinant construct.

In a further aspect is provided a plant comprising a recombinant construct containing a regulatory sequence operably linked to a polynucleotide encoding an acyl CoA:cholesterol acyltransferase-like polypeptide wherein expression of said recombinant construct results in an altered production of oil by said plant as compared to the same plant without said recombinant construct. Additional aspects will be apparent from the descriptions and examples that follow.

## BRIEF DESCRIPTION OF THE DRAWINGS

These and other features, aspects, and advantages of the present invention will become better understood with regard to the following description, appended claims and accompanying figures where:

Figure 1 shows an alignment of yeast, human and rat lecithin:cholesterol acyltransferase protein sequences with *Arabidopsis* LCAT1, LCAT2, LCAT3, and LCAT4 deduced amino acid sequences.

Figure 2 shows the results of NMR sterol ester analysis on T2 seed from plant expressing LCAT4 under the control of the napin promoter (pCGN9998).

Figure 3 shows the results of HPLC/MS sterol analysis on oil extracted from T2 seed from control lines (pCGN8640) and lines expressing LCAT3 (pCGN9968) under the control of the napin promoter.

Figure 4 shows the results of HPLC/MS sterol analysis on oil extracted from T2 seed from control lines (pCGN8640), and plant line expressing LCAT1 (pCGN9962), LCAT2 (pCGN9983), LCAT3 (pCGN9968), and LCAT4 (pCGN9998) under the control of the napin promoter. Additionally, data from 3 lines expressing LCAT4 under the control of the 35S promoter (pCGN9996) are shown.

Figure 5 shows the results of Nir analysis of the oil content of T2 seed from control lines (pCGN8640), and plant lines expressing LCAT1 (pCGN9962), LCAT2 (pCGN9983), and LCAT3 (pCGN9968) under the control of the napin promoter. Additionally, data from 16 lines expressing LCAT2 under the control of the 35S promoter (pCGN9981) are shown.

## DETAILED DESCRIPTION

The present invention relates to a lecithin:cholesterol acyltransferase, particularly the isolated nucleic acid sequences encoding lecithin:cholesterol-like polypeptides (LCAT2) from plant sources Lecithin:cholesterol acyltransferase-like as used herein includes any nucleic acid sequence encoding an amino acid sequence from a plant source, such as a protein, polypeptide or peptide, obtainable from a cell source, which demonstrates the ability to utilize lecithin (phosphatidyl choline) as an acyl donor for acylation of sterols or glycerides to form esters under enzyme reactive conditions along with such proteins polypeptides and peptides. Acyl CoA:cholesterol acyltransferase-like as used herein includes any nucleic acid sequence encoding an amino acid sequence from a plant source, such as a protein, polypeptide or peptide, obtainable from a cell source, which demonstrates the ability to utilize acyl CoA as an acyl donor for acylation of sterols or glycerides to form esters under enzyme reactive conditions along with such proteins polypeptides and peptides. By "enzyme reactive conditions" is meant that any necessary conditions are available in an environment (i.e., such factors as temperature, pH, lack of inhibiting substances) which will permit the enzyme to function.

The term "sterol" as applied to plants refers to any chiral tetracyclic isopentenoid which may be formed by cyclization of squalene oxide through the transition state possessing stereochemistry similar to the *trans-syn-trans-anti-trans-anti* configuration, for example, protosteroid cation, and which retains a polar group at C-3 (hydroxyl or keto), an *all-trans-anti* stereochemistry in the ring system, and a side-chain 20R-configuration (Parker, et al. (1992) In Nes, et al., Eds., Regulation of Isopentenoid Metabolism, ACS Symposium Series No. 497, p. 110; American Chemical Society, Washington, D.C.).

Sterols may or may not contain a C-5-C-6 double bond, as this is a feature introduced late in the biosynthetic pathway. Sterols contain a $C_8$-$C_{10}$ side chain at the C-17 position.

The term "phytosterol," which applies to sterols found uniquely in plants, refers to a sterol containing a C-5, and in some cases a C-22, double bond. Phytosterols are further characterized by alkylation of the C-17 side-chain with a methyl or ethyl substituent at the C-24 position. Major phytosterols include, but are not limited to, sitosterol, stigmasterol, campesterol, brassicasterol, etc. Cholesterol, which lacks a C-24 methyl or ethyl side-chain, is found in plants, but is not unique thereto, and is not a "phytosterol."

"Phytostanols" are saturated forms of phytosterols wherein the C-5 and, when present, C-22 double bond(s) is (are) reduced, and include, but are not limited to, sitostanol, campestanol, and 22-dihydrobrassicastanol.

"Sterol esters" are further characterized by the presence of a fatty acid or phenolic acid moiety rather than a hydroxyl group at the C-3 position.

The term "sterol" includes sterols, phytosterols, phytosterol esters, phytostanols, and phytostanol esters.

The term "sterol compounds" includes sterols, phyotsterols, phytosterol esters, phytostanols, and phytostanol esters.

The term "phytosterol compound" refers to at least one phytosterol, at least one phytosterol ester, or a mixture thereof.

The term "phytostanol compound" refers to at least one phytostanol, at least one phytostanol ester, or a mixture thereof.

The term "glyceride" refers to a fatty acid ester of glycerol and includes mono-, di-, and tri-acylglycerols.

As used herein, "recombinant construct" is defined either by its method of production or its structure. In reference to its method of production, e.g., a product made by a process, the process is use of recombinant nucleic acid techniques, e.g., involving human intervention in the nucleotide sequence, typically selection or production. Alternatively, in terms of structure, it can be a sequence comprising fusion of two or more nucleic acid sequences which are not naturally contiguous or operatively linked to each other

As used herein, "regulatory sequence" means a sequence of DNA concerned with controlling expression of a gene; e.g. promoters, operators and attenuators. A "heterologous regulatory sequence" is one which differs from the regulatory sequence naturally associated with a gene.

As used herein, "polynucleotide" and "oligonucleotide" are used interchangeably and mean a polymer of at least two nucleotides joined together by a phosphodiester bond and may consist of either ribonucleotides or deoxynucleotides.

As used herein, "sequence" means the linear order in which monomers appear in a polymer, for example, the order of amino acids in a polypeptide or the order of nucleotides in a polynucleotide.

As used herein, "polypeptide", "peptide", and "protein" are used interchangeably and mean a compound that consist of two or more amino acids that are linked by means of peptide bonds.

As used herein, the terms "complementary" or "complementarity" refer to the pairing of bases, purines and pyrimidines, that associate through hydrogen bonding in double stranded nucleic acids. For example, the following base pairs are complementary: guanine and cytosine; adenine and thymine; and adenine and uracil. The terms, as used herein, include complete and partial complementarity.

**Isolated proteins, Polypeptides and Polynucleotides**

A first aspect of the present invention relates to isolated LCAT2 polynucleotides. The polynucleotide sequences of the present invention include isolated polynucleotides that encode the polypeptides of the invention having a deduced amino acid sequence selected from the group of sequences set forth in the Sequence Listing and to other polynucleotide sequences closely related to such sequences and variants thereof.

The invention provides a polynucleotide sequence identical over its entire length to each coding sequence as set forth in the Sequence Listing. The invention also provides the coding sequence for the mature polypeptide or a fragment thereof, as well as the coding sequence for the mature polypeptide or a fragment thereof in a reading frame with other coding sequences, such as those encoding a leader or secretory sequence, a pre-, pro-, or prepro- protein sequence. The polynucleotide can also include non-coding sequences, including for example, but not limited to, non-coding 5' and 3' sequences, such as the transcribed, untranslated sequences, termination signals, ribosome binding sites, sequences that stabilize mRNA, introns, polyadenylation signals, and additional coding sequence that encodes additional amino acids. For example, a marker sequence can be included to facilitate the purification of the fused polypeptide. The polynucleotides of the present invention preferably has SEQ ID NO:4.

The invention also relates to variants of the polynucleotides described herein that encode for variants of the polypeptides of the invention. Variants that are fragments of the polynucleotides of the invention can be used to synthesize full-length polynucleotides of the invention. Preferred embodiments are polynucleotides encoding polypeptide variants wherein 5 to 10, 1 to 5, 1 to 3, 2, 1 or no amino acid residues of a polypeptide sequence of the invention are substituted, added or deleted, in any combination. Particularly preferred are substitutions, additions, and deletions that are silent such that they do not alter the properties or activities of the polynucleotide or polypeptide.

The polynucleotides of the invention are at least 95% identical over their entire length with SEQ ID NO:4. Those with at least 97% identity are preferred and those with at least 98% and 99% identity are particularly preferred, with those at least 99% being the most preferred.

Preferred embodiments are polynucleotides that encode polypeptides that retain substantially the same biological function or activity as determined by the methods described herein as the mature polypeptides encoded by the polynucleotides set forth in the Sequence Listing.

The invention further relates to polynucleotides that hybridize under stringent conditions to the above-described polynucleotides. An example of stringent hybridization conditions is overnight incubation at 42°C in a solution comprising 50% formamide, 5x SSC (150 mM NaCl, 15 mM trisodium citrate), 50 mM sodium phosphate (pH 7.6), 5x Denhardt's solution, 10% dextran sulfate, and 20 micrograms/milliliter denatured, sheared salmon sperm DNA, followed by washing the hybridization support in 0.1x SSC at approximately 65°C. Also included are polynucleotides that hybridize under a wash stringency of 0.1X SSC or 0.1X SSPE (at 50°C. Other hybridization and wash conditions are well known and are exemplified in Sambrook, et al., Molecular Cloning: A Laboratory Manual, Second Edition, Cold Spring Harbor, NY (1989), particularly Chapter 11.

The invention also provides a polynucleotide consisting essentially of a polynucleotide sequence obtainable by screening an appropriate library containing the complete gene for a polynucleotide sequence set for in the Sequence Listing under stringent hybridization conditions with a probe having the sequence of said polynucleotide sequence or a fragment thereof; and isolating said polynucleotide sequence. Methods for screening libraries are well known in the art and can be found for example in Sambrook, et al., Molecular Cloning: A Laboratory Manual, Second Edition, Cold Spring Harbor, NY (1989), particularly Chapter 8 and Ausubel et al., Short Protocols in Molecular Biology, 3rd ed, Wiley and Sons, 1995, chapter 6. Nucleic acid sequences useful for obtaining such a polynucleotide include, for example, probes and primers as described herein and in particular SEQ ID NO:4. This sequence is particularly useful in screening libraries obtained from *Arabidopsis,* soybean and corn for sequences encoding lecithin:cholesterol acyltransferase and lecithin: cholesterol acyltransferase-like polypeptides.

As discussed herein regarding polynucleotide assays of the invention, for example, polynucleotides of the invention can be used as a hybridization probe for RNA, cDNA, or genomic DNA to isolate full length cDNAs or genomic clones encoding a polypeptide and to isolate cDNA or genomic clones of other genes that have a high sequence similarity to a polynucleotide set forth SEQ ID NO:4. Such probes will generally comprise at least 15 bases. Preferably such probes will have at least 30 bases and can have at least 50 bases. Particularly preferred probes will have between 30 bases and 50 bases, inclusive.

The coding region of each gene that comprises or is comprised by a polynucleotide sequence set forth in the Sequence Listing may be isolated by screening using a DNA sequence provided in the Sequence Listing to synthesize an oligonucleotide probe. A labeled oligonucleotide having a sequence complementary to that of a gene of the invention is then used to screen a library of cDNA, genomic DNA or mRNA to identify members of the library which hybridize to the probe. For example, synthetic oligonucleotides are prepared which correspond to the LCAT2 EST sequences. The oligonucleotides are used as primers in polymerase chain reaction (PCR) techniques to obtain 5' and 3' terminal sequence of LCAT2 genes. Alternatively, where oligonucleotides of low degeneracy can be prepared from particular LCAT2 peptides, such probes may be used directly to screen gene libraries for LCAT2 gene sequences. In particular, screening of cDNA libraries in phage vectors is useful in such methods due to lower levels of background hybridization.

Typically, a LCAT2 sequence obtainable from the use of nucleic acid probes will show 60-70% sequence identity between the target LCAT2 sequence and the encoding sequence used as a probe. However, lengthy sequences with as little as 50-60% sequence identity may also be obtained. The nucleic acid probes may be a lengthy fragment of the nucleic acid sequence, or may also be a shorter, oligonucleotide probe. When longer nucleic acid fragments are employed as probes (greater than about 100 bp), one may screen at lower stringencies in order to obtain sequences from the target sample which have 20-50% deviation (i.e., 50-80% sequence homology) from the sequences used as probe. Oligonucleotide probes can be considerably shorter than the entire nucleic acid sequence encoding an LCAT2 enzyme, but should be at least about 10, preferably at least about 15, and more preferably at least about 20 nucleotides. A higher degree of sequence identity is desired when shorter regions are used as opposed to longer regions. It may thus be desirable to identify regions of highly conserved amino acid sequence to design oligonucleotide probes for detecting and recovering other related LCAT2 genes. Shorter probes are often particularly useful for polymerase chain reactions (PCR), especially when highly conserved sequences can be identified. (*See,* Gould, et al., PNAS USA (1989) 86:1934-1938.). Another aspect of the present invention relates to the LCAT2 polypeptide of SEQ ID NO:5 as set forth in the Sequence Listing.

The polypeptides of the present invention can be mature protein or can be part of a fusion protein.

The polynucleotides and polypeptides of the invention can be used, for example, in the transformation of host cells, such as plant cells, animal cells, yeast cells, bacteria, bacteriophage, and viruses, as further discussed herein.

The invention also provides polynucleotides that encode a polypeptide that is a mature protein plus additional amino or carboxyl-terminal amino acids, or amino acids within the mature polypeptide (for example, when the mature form of the protein has more than one polypeptide chain). Such sequences can, for example, play a role in the processing of a protein from a precursor to a mature form, allow protein transport, shorten or lengthen protein half-life, or facilitate manipulation of the protein in assays or production. It is contemplated that cellular enzymes can be used to remove any additional amino acids from the mature protein.

A precursor protein, having the mature form of the polypeptide fused to one or more prosequences may be an inactive form of the polypeptide. The inactive precursors generally are activated when the prosequences are removed. Some or all of the prosequences may be removed prior to activation. Such precursor protein are generally called proproteins.

## Preparation of Expression Constructs and Methods of Use

Of interest is the use of the nucleotide sequences in recombinant DNA constructs to direct the transcription or transcription and translation (expression) of the acyltransferase sequences of the present invention in a host cell. Of particular interest is the use of the polynucleotide sequences of the present invention in recombinant DNA constructs to direct the transcription or transcription and translation (expression) of the acyltransferase sequences of the present invention in a host plant cell.

The expression constructs generally comprise a regulatory sequence functional in a host cell operably linked to a nucleic acid sequence encoding a lecithin:cholesterol acyltransferase-like polypeptide of the present invention and a transcriptional termination region functional in a host plant cell. Of particular interest is the use of promoters (also referred to as transcriptional initiation regions) functional in plant host cells.

Those skilled in the art will recognize that there are a number of promoters which are functional in plant cells, and have been described in the literature including constitutive, inducible, tissue specific, organelle specific, developmentally regulated and environmentally regulated promoters. Chloroplast and plastid specific promoters, chloroplast or plastid functional promoters, and chloroplast or plastid operable promoters are also envisioned.

One set of promoters are constitutive promoters such as the CaMV35S or FMV35S promoters that yield high levels of expression in most plant organs. Enhanced or duplicated versions of the CaMV35S and FMV35S promoters are useful in the practice of this invention (Odell, et al. (1985) Nature 313:810-812; Rogers, U.S. Patent Number 5,378, 619). Other useful constitutive promoters include the mannopine synthase (*mas*) promoter, the nopaline synthase (*nos*) promoter, and the octopine synthase (*ocs*) promoter.

Useful inducible promoters include heat-shock promoters (Ou-Lee et al. (1986) Proc. Natl. Acad. Sci. USA 83: 6815; Ainley et al. (1990) Plant Mol. Biol. 14: 949), a nitrate-inducible promoter derived from the spinach nitrite reductase gene (Back et al. (1991) Plant Mol. Biol. 17: 9), hormone-inducible promoters (Yamaguchi-Shinozaki et al. (1990) Plant Mol. Biol. 15: 905; Kares et al. (1990) Plant Mol. Biol. 15: 905), and light-inducible promoters associated with the small subunit of RuBP carboxylase and LHCP gene families (Kuhlemeier et al. (1989) Plant Cell 1: 471; Feinbaum et al. (1991) Mol. Gen. Genet. 226: 449; Weisshaar et al. (1991) EMBO J. 10: 1777; Lam and Chua (1990) Science 248: 471; Castresana et al. (1988) EMBO J. 7: 1929; Schulze-Lefert et al. (1989) EMBO J. 8: 651).

In addition, it may also be preferred to bring about expression of the acyltransferase gene in specific tissues of the plant, such as leaf, stem, root, tuber, seed and fruit, and the promoter chosen should have the desired tissue and developmental specificity. Examples of useful tissue-specific, developmentally-regulated promoters include fruit-specific promoters such as the E4 promoter (Cordes et al. (1989) Plant Cell 1:1025), the E8 promoter (Deikman et al. (1988) EMBO J. 7: 3315), the kiwifruit actinidin promoter (Lin et al. (1993) PNAS 90: 5939), the 2A11 promoter (Houck et al., U.S. Patent 4,943,674), and the tomato pZ130 promoter (U.S. Patents 5,175, 095 and 5,530,185); the β-conglycinin 7S promoter (Doyle et al. (1986) J. Biol. Chem. 261: 9228; Slighton and Beachy (1987) Planta 172: 356), and seed-specific promoters (Knutzon et al. (1992) Proc. Natl. Acad Sci. USA 89: 2624; Bustos et al. (1991) EMBO J. 10: 1469; Lam and Chua (1991) J. Biol. Chem. 266: 17131; Stayton et al. (1991) Aust. J. Plant. Physiol. 18: 507). Fruit-specific gene regulation is discussed in U.S. Patent 5,753,475. Other useful seed-specific promoters include the napin, phaseolin, zein, soybean trypsin inhibitor, 7S, ADR12, ACP, stearoyl-ACP desaturase, oleosin, *Lasquerella* hydroxylase, and barley aldose reductase promoters (Bartels (1995) Plant J. 7: 809-822), the EA9 promoter (U.S. Patent 5,420,034), and the Bce4 promoter (U.S. Patent 5,530,194). Useful embryo-specific promoters include the corn globulin 1 and oleosin promoters. Useful endosperm-specific promoters include the rice glutelin-1 promoter, the promoters for the low-pI β amylase gene (Amy32b) (Rogers et al. (1984) J. Biol. Chem. 259: 12234), the high-pI β amylase gene (Amy 64) (Khurseed et al. (1988) J. Biol. Chem. 263: 18953), and the promoter for a barley thiol protease gene ("Aleurain") (Whittier et al. (1987) Nucleic Acids Res. 15: 2515).

Of particular interest is the expression of the nucleic acid sequences of the present invention from transcription initiation regions which are preferentially expressed in a plant seed tissue. Examples of such seed preferential transcription initiation sequences include those sequences derived from sequences encoding plant storage protein genes or from genes involved in fatty acid biosynthesis in oilseeds. Examples of such promoters include the 5' regulatory regions from such genes as napin (Kridl et al., Seed Sci. Res. 1:209:219 (1991)), phaseolin, zein, soybean trypsin inhibitor, ACP, stearoyl-ACP desaturase, soybean α' subunit of β-conglycinin (soy 7s, (Chen et al., Proc. Natl. Acad. Sci., 83:8560-8564 (1986))) and oleosin. Seed-specific gene regulation is discussed in EP 0 255 378 B1 and U.S. Patents 5,420,034 and 5,608,152. Promoter hybrids can also be constructed to enhance transcriptional activity (Hoffman, U.S. Patent No. 5,106,739), or to combine desired transcriptional activity and tissue specificity.

It may be advantageous to direct the localization of proteins conferring LCAT2 to a particular subcellular compartment, for example, to the mitochondrion, endoplasmic reticulum, vacuoles, chloroplast or other plastidic compartment. For example, where the genes of interest of the present invention will be targeted to plastids, such as chloroplasts, for expression, the constructs will also employ the use of sequences to direct the gene to the plastid. Such sequences are referred to herein as chloroplast transit peptides (CTP) or plastid transit peptides (PTP). In this manner, where the gene of interest is not directly inserted into the plastid, the expression construct will additionally contain a gene encoding a transit peptide to direct the gene of interest to the plastid. The chloroplast transit peptides may be derived from the gene of interest, or may be derived from a heterologous sequence having a CTP. Such transit peptides are known in the art. See, for example, Von Heijne et al. (1991) Plant Mol. Biol. Rep. 9:104-126; Clark et al. (1989) J. Biol. Chem. 264: 17544-17550; della-Cioppa et al. (1987) Plant Physiol. 84:965-968; Romer et al. (1993) Biochem. Biophys. Res Commun. 196:1414-1421; and, Shah et al. (1986) Science 233:478-481.

Depending upon the intended use, the constructs may contain the nucleic acid sequence which encodes the entire LCAT protein or a portion of the LCAT protein. For example, where LCAT2 sequences used in constructs are intended for use as probes, it may be advantageous to prepare constructs containing only a particular portion of a LCAT2 encoding sequence, for example a sequence which is discovered to encode a highly conserved region.

Regulatory transcript termination regions may be provided in plant expression constructs of this invention as well. Transcript termination regions may be provided by the DNA sequence encoding the diacylglycerol acyltransferase or a convenient transcription termination region derived from a different gene source, for example, the transcript termination region which is naturally associated with the transcript initiation region. The skilled artisan will recognize that any convenient transcript termination region which is capable of terminating transcription in a plant cell may be employed in the constructs of the present invention.

Alternatively, constructs may be prepared to direct the expression of the LCAT sequences directly from the host plant cell plastid. Such constructs and methods are known in the art and are generally described, for example, in Svab, et al. (1990) Proc. Natl. Acad. Sci. USA 87:8526-8530 and Svab and Maliga (1993) Proc. Natl. Acad. Sci. USA 90:913-917 and in U.S. Patent Number 5,693,507.

A plant cell, tissue, organ, or plant into which the recombinant DNA constructs containing the expression constructs have been introduced is considered transformed, transfected, or transgenic. A transgenic or transformed cell or plant also includes progeny of the cell or plant and progeny produced from a breeding program employing such a transgenic plant as a parent in a cross and exhibiting an altered phenotype resulting from the presence of a LCAT2 nucleic acid sequence. Plant expression or transcription constructs having a plant LCAT2 as the DNA sequence of interest for increased or decreased expression thereof may be employed with a wide variety of plant life, particularly, plant life involved in the production of vegetable oils for edible and industrial uses. Most especially preferred are temperate oilseed crops. Plants of interest include, but are not limited to, rapeseed (Canola and High Erucic Acid varieties), sunflower, safflower, cotton, soybean, peanut, coconut and oil palms, and corn. Depending on the method for introducing the recombinant constructs into the host cell, other DNA sequences may be required. Importantly, this invention is applicable to dicotyledyons and monocotyledons species alike and will be readily applicable to new and/or improved transformation and regulation techniques.

Of particular interest, is the use of plant LCAT2 constructs in plants to produce plants or plant parts, including, but not limited to leaves, stems, roots, reproductive, and seed, with a modified content of lipid and/or sterol esters and to alter the oil production by such plants.

Of particular interest in the present invention, is the use of acyl CoA:cholesterol acyltransferase-like polypeptide (ACAT) genes in conjunction with the LCAT2 sequences to increase the sterol content of seeds. Thus, overexpression of a nucleic acid sequence encoding an ACAT and LCAT2 in an oilseed crop may find use in the present invention to increase sterol levels in plant tissues and/or increase oil production.

It is contemplated that the gene sequences may be synthesized, either completely or in part, especially where it is desirable to provide plant-preferred sequences. Thus, all or a portion of the desired structural gene (that portion of the gene which encodes the LCAT or ACAT protein) may be synthesized using codons preferred by a selected host. Host-preferred codons may be determined, for example, from the codons used most frequently in the proteins expressed in a desired host species.

One skilled in the art will readily recognize that antibody preparations, nucleic acid probes (DNA and RNA) and the like may be prepared and used to screen and recover "homologous" or "related" sequences from a variety of plant sources. Homologous sequences are found when there is an identity of sequence, which may be determined upon comparison of sequence information, nucleic acid or amino acid, or through hybridization reactions between a known LCAT2 and a candidate source. Conservative changes, such as Glu/Asp, Val/Ile, Ser/Thr, Arg/Lys and Gln/Asn may also be considered in determining sequence homology. Amino acid sequences are considered homologous by as little as 25% sequence identity between the two complete mature proteins. (*See* generally, Doolittle, R.F., OF URFS and ORFS (University Science Books, CA, 1986.)

Thus, other LCAT2s may be obtained from the specific sequences provided herein. Furthermore, it will be apparent that one can obtain natural and synthetic sequences, including modified amino acid sequences and starting materials for synthetic-protein modeling from the exemplified LCAT2 sequences and from sequences which are obtained through the use of such exemplified sequences. Modified amino acid sequences include sequences which have been mutated, truncated or increased, whether such sequences were partially or wholly synthesized. Sequences which are actually purified from plant preparations or are identical or encode identical proteins thereto, regardless of the method used to obtain the protein or sequence, are equally considered naturally derived.

For immunological screening, antibodies to the protein can be prepared by injecting rabbits or mice with the purified protein or portion thereof, such methods of preparing antibodies being well known to those in the art. Either monoclonal or polyclonal antibodies can be produced, although typically polyclonal antibodies are more useful for gene isolation. Western analysis may be conducted to determine that a related protein is present in a crude extract of the desired plant species, as determined by cross-reaction with the antibodies to the encoded proteins. When cross-reactivity is observed, genes encoding the related proteins are isolated by screening expression libraries representing the desired plant species. Expression libraries can be constructed in a variety of commercially available vectors, including lambda gt11, as described in Sambrook, et al. (Molecular Cloning: A Laboratory Manual, Second Edition (1989) Cold Spring Harbor Laboratory, Cold Spring Harbor, New York).

To confirm the activity and specificity of the proteins encoded by the identified nucleic acid sequences as acyltransferase enzymes, *in vitro* assays are performed in insect cell cultures using baculovirus expression systems. Such baculovirus expression systems are known in the art and are described by Lee, et al. U.S. Patent Number 5,348,886.

In addition, other expression constructs may be prepared to assay for protein activity utilizing different expression systems. Such expression constructs are transformed into yeast or prokaryotic host and assayed for acyltransferase activity. Such expression systems are known in the art and are readily available through commercial sources.

The method of transformation in obtaining such transgenic plants is not critical to the instant invention, and various methods of plant transformation are currently available. Furthermore, as newer methods become available to transform crops, they may also be directly applied hereunder. For example, many plant species naturally susceptible to *Agrobacterium* infection may be successfully transformed via tripartite or binary vector methods of *Agrobacterium* mediated transformation. In many instances, it will be desirable to have the construct bordered on one or both sides by T-DNA, particularly having the left and right borders, more particularly the right border. This is particularly useful when the construct uses *A. tumefaciens* or *A. rhizogenes* as a mode for transformation, although the T-DNA borders may find use with other modes of transformation. In addition, techniques of microinjection, DNA particle bombardment, and electroporation have been developed which allow for the transformation of various monocot and dicot plant species.

Normally, included with the DNA construct will be a structural gene having the necessary regulatory regions for expression in a host and providing for selection of transformant cells. The gene may provide for resistance to a cytotoxic agent, e.g. antibiotic, heavy metal, toxin, etc., complementation providing prototrophy to an auxotrophic host, viral immunity or the like. Depending upon the number of different host species the expression construct or components thereof are introduced, one or more markers may be employed, where different conditions for selection are used for the different hosts.

Examples of suitable selection markers include genes that confer resistance to bleomycin, gentamycin, glyphosate, hygromycin, kanamycin, methotrexate, phleomycin, phosphinotricin, spectinomycin, streptomycin, sulfonamide and sulfonylureas. Maliga et al., Methods in Plant Molecular Biology, Cold Spring Harbor Laboratory Press, 1995, p. 39. Examples of markers include, but are not limited to, alkaline phosphatase (AP), myc, hemagglutinin (HA), β glucuronidase (GUS), luciferase, and green fluorescent protein (GFP).

Where *Agrobacterium* is used for plant cell transformation, a vector may be used which may be introduced into the *Agrobacterium* host for homologous recombination with T-DNA or the Ti- or Ri-plasmid present in the *Agrobacterium* host. The Ti- or Ri-plasmid containing the T-DNA for recombination may be armed (capable of causing gall formation) or disarmed (incapable of causing gall formation), the latter being permissible, so long as the *vir* genes are present in the transformed *Agrobacterium* host. The armed plasmid can give a mixture of normal plant cells and gall.

In some instances where *Agrobacterium* is used as the vehicle for transforming host plant cells, the expression or transcription construct bordered by the T-DNA border region(s) will be inserted into a broad host range vector capable of replication in *E. coli* and *Agrobacterium,* there being broad host range vectors described in the literature. Commonly used is pRK2 or derivatives thereof. See, for example, Ditta, et al., (Proc. Nat. Acad. Sci., U.S.A. (1980) 77:7347-7351) and EP-A-0 120 515. Alternatively, one may insert the sequences to be expressed in plant cells into a vector containing separate replication sequences, one of which stabilizes the vector in *E. coli,* and the other in *Agrobacterium.* See, for example, McBride and Summerfelt (Plant Mol. Biol. (1990) 14:269-276), wherein the pRiHRI (Jouanin, et al., Mol. Gen. Genet. (1985) 201:370-374) origin of replication is utilized and provides for added stability of the plant expression vectors in host *Agrobacterium* cells.

Included with the expression construct and the T-DNA can be one or more markers, which allow for selection of transformed Agrobacterium and transformed plant cells. A number of markers have been developed for use with plant cells, such as resistance to chloramphenicol, kanamycin, the aminoglycoside G418 and hygromycin. The particular marker employed is not essential to this invention, one or another marker being preferred depending on the particular host and the manner of construction.

For transformation of plant cells using *Agrobacterium,* explants may be combined and incubated with the transformed *Agrobacterium* for sufficient time for transformation, the bacteria killed, and the plant cells cultured in an appropriate selective medium. Once callus forms, shoot formation can be encouraged by employing the appropriate plant hormones in accordance with known methods and the shoots transferred to rooting medium for regeneration of plants. The plants may then be grown to seed and the seed used to establish repetitive generations and for isolation of vegetable oils.

Thus, another aspect of the present invention provides methods for modifying the sterol and/or stanol composition of a host cell. Of particular interest are methods for modifying the sterol and/or stanol composition of a host plant cell. In general the methods involve either increasing the levels of sterol ester compounds as a proportion of the total sterol compounds. The method generally comprises the use of expression constructs to direct the expression of the polynucleotides of the present invention in a host cell.

Also provided are methods for reducing the proportion of sterol ester compounds as a percentage of total sterol compounds in a host plant cell. The method generally comprises the use of expression constructs to direct the suppression of endogenous acyltransferase proteins in a host cell.

Of particular interest is the use of expression constructs to modify the levels of sterol compounds in a host plant cell. Most particular, the methods find use in modifying the levels of sterol compounds in seed oils obtained from plant seeds.

Also of interest is the use of expression constructs of the present invention to alter oil production in a host cell and in particular to increase oil production. Of particular interest is the use of expression constructs containing nucleic acid sequences encoding LCAT2 polypeptides to transform host plant cells and to use these host cells to regenerate whole plants having increase oil production as compared to the same plant not containing the expression construct.

The oils obtained from transgenic plants having modified sterol compound content find use in a wide variety of applications. Of particular interest is the use of the oils containing modified levels of sterol compounds in applications involved in improving human nutrition and cardiovascular health. For example, phytostanols are beneficial for lowering serum cholesterol (Ling, et al. (1995) Life Sciences 57:195-206).

Cholesterol-lowering compositions comprise the oils and sterol ester compound compositions obtained using the methods of the present invention. Such cholesterol lowering compositions include, but are not limited to foods, food products, processed foods, food ingredients, food additive compositions, or dietary/nutritional supplements that contain oils and/or fats. Nonlimiting examples include margarines; butters; shortenings; cooking oils; frying oils; dressings, such as salad dressings; spreads; mayonnaises; and vitamin/mineral supplements. Patent documents relating to such compositions include, U.S. Patents 4,588,717 and 5,244,887, WO 96/38047, WO 97/42830, WO 98/06405, and WO 98/06714. Additional examples include toppings; dairy products such as cheese and processed cheese; processed meat; pastas; sauces; cereals; desserts, including frozen and shelf-stable desserts; dips; chips; baked goods; pastries; cookies; snack bars; confections; chocolates; beverages; unextracted seed; and unextracted seed that has been ground, cracked, milled, rolled, extruded, pelleted, defatted, dehydrated, or otherwise processed, but which still contains the oils, disclosed herein.

The cholesterol-lowering compositions can also take the form of pharmaceutical compositions comprising a cholesterol-lowering effective amount of the oils or sterol compound compositions obtained using the methods of the present invention, along with a pharmaceutically acceptable carrier, excipient, or diluent. These pharmaceutical compositions can be in the form of a liquid or a solid. Liquids can be solutions or suspensions; solids can be in the form of a powder, a granule, a pill, a tablet, a gel, or an extrudate. U.S. Patent 5,270,041 relates to sterol-containing pharmaceutical compositions.

The invention now being generally described, it will be more readily understood by reference to the following examples.

## EXAMPLES

**Example 1:** RNA Isolations

Total RNA from the inflorescence and developing seeds of *Arabidopsis thaliana* was isolated for use in construction of complementary (cDNA) libraries. The procedure was an adaptation of the DNA isolation protocol of Webb and Knapp (D.M. Webb and S.J. Knapp, (1990) Plant Molec. Reporter, 8, 180-185). The following description assumes the use of 1g fresh weight of tissue. Frozen seed tissue was powdered by grinding under liquid nitrogen. The powder was added to 10 ml REC buffer (50 mM Tris-HCl, pH 9, 0.8M NaCl, 10 mM EDTA, 0.5% w/v CTAB (cetyltrimethylammonium bromide)) along with 0.2 g insoluble polyvinylpolypyrrolidone, and ground at room temperature. The homogenate was centrifuged for 5 minutes at 12,000 x g to pellet insoluble material. The resulting supernatant fraction was extracted with chloroform, and the top phase was recovered.

The RNA was then precipitated by addition of 1 volume RecP (50 mM Tris-HCL pH9, 10 mM EDTA and 0.5% (w/v) CTAB) and collected by brief centrifugation as before. The RNA pellet was redissolved in 0.4 ml of 1M NaCl. The RNA pellet was redissolved in water and extracted with phenol/chloroform. Sufficient 3M potassium acetate (pH 5) was added to make the mixture 0.3 M in acetate, followed by addition of two volumes of ethanol to precipitate the RNA. After washing with ethanol, this final RNA precipitate was dissolved in water and stored frozen. Alternatively, total RNA may be obtained using TRIzol reagent (BRL-Lifetechnologies, Gaithersburg, MD) following the manufacturer's protocol. The RNA precipitate was dissolved in water and stored frozen.

**Example 2:** Identification of LCAT Sequences

Searches were performed on a Silicon Graphics Unix computer using additional Bioaccellerator hardware and GenWeb software supplied by Compugen Ltd. This software and hardware enabled the use of the Smith-Waterman algorithm in searching DNA and protein databases using profiles as queries. The program used to query protein databases was profilesearch. This is a search where the query is not a single sequence but a profile based on a multiple alignment of amino acid or nucleic acid sequences. The profile was used to query a sequence data set, i.e., a sequence database. The profile contained all the pertinent information for scoring each position in a sequence, in effect replacing the "scoring matrix" used for the standard query searches. The program used to query nucleotide databases with a protein profile was tprofilesearch. Tprofilesearch searches nucleic acid databases using an amino acid profile query. As the search is running, sequences in the database are translated to amino acid sequences in six reading frames. The output file for tprofilesearch is identical to the output file for profilesearch except for an additional column that indicates the frame in which the best alignment occurred.

The Smith-Waterman algorithm, (Smith and Waterman (1981) J. Molec. Biol. 147:195-197), was used to search for similarities between one sequence from the query and a group of sequences contained in the database.

A protein sequence of Lecithin: cholesterol acyltransferase from human (McLean J, et al. (1986) Nucleic Acids Res. 14(23):9397-406 SEQ ID NO:1)) was used to search the NCBI non-redundant protein database using BLAST. Three sequences were identified from *Arabidopsis,* GenBank accessions AC004557 ( referred to herein as LCAT1, SEQ ID NO:2), AC003027 (referred to herein as LCAT2, SEQ ID NO:4), and AL024486 (referred to herein as LCAT3, SEQ ID NO:6). The deduced amino acid sequences are provided in SEQ ID NOs: 3, 5, and 7, respectively.

The profile generated from the queries using PSI-BLAST was excised from the hyper text markup language (html) file. The worldwide web (www)/html interface to psiblast at ncbi stores the current generated profile matrix in a hidden field in the html file that is returned after each iteration of psiblast. However, this matrix has been encoded into string62 (s62) format for ease of transport through html. String62 format is a simple conversion of the values of the matrix into html legal ascii characters.

The encoded matrix width (x axis) is 26 characters, and comprise the consensus characters, the probabilities of each amino acid in the order A,B,C,D,E,F,G,H,I,K,L,M,N, P,Q,R,S,T,V,W,X,Y,Z (where B represents D and N, and Z represents Q and E, and X represents any amino acid), gap creation value, and gap extension value.

The length (y axis) of the matrix corresponds to the length of the sequences identified by PSI-BLAST. The order of the amino acids corresponds to the conserved amino acid sequence of the sequences identified using PSI-BLAST, with the N-terminal end at the top of the matrix. The probabilities of other amino acids at that position are represented for each amino acid along the x axis, below the respective single letter amino acid abbreviation.

Thus, each row of the profile consists of the highest scoring (consensus) amino acid, followed by the scores for each possible amino acid at that position in sequence matrix, the score for opening a gap that that position, and the score for continuing a gap at that position.

The string62 file is converted back into a profile for use in subsequent searches. The gap open field is set to 11 and the gap extension field is set to 1 along the x axis. The gap creation and gap extension values are known, based on the settings given to the PSI-BLAST algorithm. The matrix is exported to the standard GCG profile form. This format can be read by GenWeb.

The algorithm used to convert the string62 formatted file to the matrix is outlined in Table 1.

**Table 1**

| | |
|---|---|
| 1. | if encoded character z then the value is blast score min |
| 2. | if encoded character Z then the value is blast score max |
| 3. | else if the encoded character is uppercase then its value is (64-(ascii # of char)) |
| 4. | else if the encoded character is a digit the value is ((ascii # of char)-48) |
| 5. | else if the encoded character is not uppercase then the value is ((ascii # of char) - 87) |
| 6. | ALL B positions are set to min of D and N amino acids at that row in sequence matrix |
| 7. | ALL Z positions are set to min of Q amd E amino acids at that row in sequence matrix |
| 8. | ALL X positions are set to min of all amino acids at that row in sequence matrix |
| 9. | kBLAST_SCORE_MAX=999; |
| 10. | kBLAST_SCORE_MIN=-999; |
| 11. | all gap opens are set to 11 |
| 12. | all gap lens are set to 1 |

The protein sequences of LCAT1, LCAT2, and LCAT3 as well as the PSI-BLAST profile were used to search public and proprietary databases for additional LCAT sequences. Two EST sequences were identified which appear to be identical to LCAT1 and LCAT3, respectively. One additional *Arabidopsis* sequence was identified from the proprietary databases, LCAT4 (SEQ ID NO:8). The deduced protein sequence of LCAT4 is provided in SEQ ID NO:9. Two additional genomic sequences were identified using the PSI-BLAST profile from libraries of *Arabidopsis* ecotypes Columbia and Landsberg, LCAT7 (SEQ ID NO: 10) and LCAT8 (SEQ ID NO:11). The LCAT7 sequence was present in both the Columbia and Landsberg genomic libraries, while the LCAT8 sequence was only present in the Columbia library.

An open reading frame was predicted from the genomic sequence of LCAT7 in the Arabidopsis public database and this sequence was called MSH12 (referred to herein as LCAT5, SEQ ID NO: 73). The deduced protein sequence of LCAT5 is provided in SEQ ID NO: 74.

The PSI-BLAST profile and the LCAT sequences were used to query the public yeast database and proprietary libraries containing corn and soy EST sequences. The yeast genome contains only one gene, *LRO1* (LCAT Related Open reading frame, YNR008W, Figure 1) with distinct similarity to the human LCAT. The DNA sequence of *LRO1* is provided in SEQ ID NO: 75 and the protein sequence is provided in SEQ ID NO: 76. Seven EST sequences were identified from soybean libraries as being LCAT sequences. Two sequences from soy (SEQ ID NOs: 12 and 13) are most closely related to the *Arabidopsis* LCAT1 sequence, a single sequence was identified as being most closely related to LCAT2 (SEQ ID NO:14), three were closely related to LCAT3 (SEQ ID NOs: 15-17), and an additional single sequence was identified (SEQ ID NO:18). A total of 11 corn EST sequences were identified as being related to the *Arabidopsis* LCAT sequences. Two corn EST sequences (SEQ ID NOs: 19 and 20) were most closely related to LCAT1, two sequences were identified as closely related to LCAT2 (SEQ ID NOs: 21 and 22), four corn EST sequences were identified as closely related to LCAT3 (SEQ ID NOs: 23-26), and an additional three corn EST sequences were also identified (SEQ ID NOs: 27-29).

**Example 3 (Reference Example):** Identification of ACAT Sequences

Since plant ACATs are unknown in the art, searches were performed to identify known and related ACAT sequences from mammalian sources from public databases. These sequences were then used to search public and proprietary EST databases to identify plant ACAT-like sequences. A public database containing mouse Expressed Sequence Tag (EST) sequences (dBEST) was searched for ACAT-like sequences. The search identified two sequences (SEQ ID 30 and 31) which were related (approximately 20% identical), but divergent, to known ACAT sequences. In order to identify ACAT-like sequences from other organisms, the two mouse ACAT sequences were used to search public and proprietary databases containing EST sequences from human and rat tissues. Results of the search identified several sequences from the human database and from the rat database which were closely related to the mouse sequences. The human and rat ACAT-like EST sequences were assembled, using the GCG assembly program, to construct a complete inferred cDNA sequence by identifying overlapping sequences (SEQ ID NOs: 32 and 33, respectively).

The protein sequence of the human ACAT-like sequence was aligned with known ACAT sequences from human (Chang, et al. (1993) J. Biol. Chem. 268:20747-20755, SEQ ID NO:34), mouse (Uelmen, et al. (1995) J. Biol. Chem. 270:26192-26201 SEQ ID NO:35) and yeast (Yu, et al. (1996) J. Biol. Chem. 271:24157-24163, SEQ ID NO:36 and Yang, et al. (1996) Science 272:1353-1356, SEQ ID NO:37) using MacVector (Oxford Molecular, Inc.). Results of the alignment demonstrated that the sequence was related to the known sequences, however the related sequence was only about 25% similar to the known sequences.

The protein sequence of the human sterol O-acyltransferase (ACAT, Acyl CoA:Cholesterol acyltransferase, Accession number A48026) related sequence was used to search protein and nucleic acid Genbank databases. A single plant homologue was identified in the public *Arabidopsis* EST database (Accession A042298, SEQ ID NO:38). The protein sequence (SEQ ID NO:39)was translated from the EST sequence, and was found to contain a peptide sequence conserved in both mammalian and yeast ACATs (Chang et al., (1997) Ann. Rev. Biochem., 66:613-638).

To obtain the entire coding region corresponding to the *Arabidopsis* ACAT-like EST, synthetic oligo-nucleotide primers were designed to amplify the 5' and 3' ends of partial cDNA clones containing ACAT-like sequences. Primers were designed according to the *Arabidopsis* ACAT-like EST sequence and were used in Rapid Amplification of cDNA Ends (RACE) reactions (Frohman et al. (1988) Proc. Natl. Acad. Sci. USA 85:8998-9002).

Primers were designed (5'-TGCAAATTGACGAGCACACCAACCCCTTC-3' (SEQ ID NO:40) and 5'-AAGGATGCTTTGAGTTCCTGACAATAGG-3' (SEQ ID NO:41)) to amplify the 5' end from the Arabidopsis ACAT EST sequence. Amplification of flanking sequences from cDNA clones were performed using the Marathon cDNA Amplification kit (Clontech, CA). The sequence derived from the 5'-RACE amplification was used to search proprietary *Arabidopsis* EST libraries. A single EST accession, LIB25-088-C7 (SEQ ID NO:42), was identified which contained a sequence identical to the 5'-RACE sequence. Furthermore, LIB25-088-C7 was found to contain the complete putative coding sequence for the *Arabidopsis* ACAT-like product.

The nucleic acid as well as the putative translation product sequences of A042298 were used to search public and proprietary databases. Four EST sequences were identified in both soybean (SEQ ID NOs:43-46) and maize (SEQ ID NOs:47-50) proprietary databases, and a single ACAT-like sequence was identified from *Mortierrella alpina* EST sequences (SEQ ID NO:51). Sequence alignments between ACAT sequences from several different sources were compared to identify the similarity between the sequences. Nucleotide sequences from known human and mouse ACATs, as well as nucleotide sequences from known yeast ACATs were compared to the ACAT-like EST sequences from human and *Arabidopsis.*

Analysis of the sequence alignments revealed several classes of ACATs based on sequence similarity. The known human and mouse ACATs, being 88% similar in the nucleotide sequence, formed one class of ACATs. Another class of ACATs included the yeast ACATs which are less than 20% similar to the known human and mouse class ACATs.

The final class of ACATs included the Arabidopsis and human sequences disclosed in the present invention. This class is approximately 22% similar to the known human and mouse ACAT class and approximately 23% similar to the yeast class of ACATs. Thus, the ACAT sequences disclosed in the present invention represent a novel class of ACAT enzymes. Partial mouse sequences of this class are also provided.

**Example 4:** Expression Construct Preparation

Constructs were prepared to direct expression of the LCAT1, LCAT2, LCAT3, LCAT4, LCAT5 and the yeast LRO1 sequences in plants and cultured insect cells. The entire coding region of each LCAT was amplified from the appropriate EST clone or an Arabidopsis genomic cDNA library using the following oligonucleotide primers in a polymerase chain reactions (PCR). The LCAT1 coding sequence was amplified from the EST clone Lib25-082-Q1-E1-G4 using the primers 5'-GGATCCGCGGCCGCACAATGAAAAAAATATCTTCACATTATTCGG-3' (SEQ ID NO:52) and 5'-GGATCCCCTGCAGGTCATTCATTGACGGCATTAACATTGG-3' (SEQ ID NO:53). The LCAT2 coding sequence was amplified from an Arabidopsis genomic cDNA library using the synthetic oligo nucleotide primers
5'-GGATCCGCGGCCGCACAATGGGAGCGAATTCGAAATCAGTAACG-3' (SEQ ID NO:54) and 5'-GGATCCCCTGCAGGTTAATACCCACTTTTATCAAGCTCCC-3' (SEQ ID NO:55). The LCAT3 coding sequence was amplified from the EST clone LIB22-004-Q1-E1-B4 using the synthetic oligo nucleotide primers
5'-GGATCCGCGGCCGCACAATGTCTCTATTACTGGAA GAGATC-3' (SEQ ID NO:56) and 5'-GGATCCCCTGCAGGTTATGCATC AACAGAGACACTTACAGC-3' (SEQ ID NO:57). The LCAT4 coding sequence was amplified from the EST clone LIB23-007-Q1-E1-B5 using the synthetic oligo nucleotide primers
5'-GGATCCGCGGCCGCACAATGGGCTGGATTCCGTGTCCGTGC-3' (SEQ ID NO:58) and 5'-GGATCCCCTGCAGGTTAACCAGAATCAACTACTTTGTG-3' (SEQ ID NO:59).

The LCAT5 coding sequence was amplified from LIB23-053-Q1-E1-E3 using the synthetic oligo nucleotide primers
5'-GGATCCGCGGCCGCACAATGCCCCTTATTCATCGG-3' (SEQ ID NO:77) and 5'-GGATCCCCTGCAGGTCACAGCTTCAGGTCAATACG-3' (SEQ ID NO:78).

The yeast LRO1 coding sequence was amplified from genomic yeast DNA using the synthetic oligo nucleotide primers 5'GGATCCGCGGCCGCACAATGGGCACACTGTTTCGAAG3' (SEQ ID NO:79)
and 5'GGATCCCCTGCAGGTTACATTGGGAAGGGCATCTGAG3' (SEQ ID NO:80). The entire coding region of the *Arabidopsis* ACAT sequence (SEQ ID NO: 42) was amplified from the EST clone LIB25-088-C7 using oligonucleotide primers
5'-TCGACCTGCAGGAAGCTTAGAAATGGCGATTTTGGATTC-3' (SEQ ID NO: 60) and
5'-GGATCCGCGGCCGCTCATGACATCGATCCTTTTCGG-3' (SEQ ID NO: 61) in a polymerase chain reaction (PCR).

Each resulting PCR product was subcloned into pCR2.1Topo (Invitrogen) and labeled pCGN9964 (LCAT1), pCGN9985 (LCAT2), pCGN9965 (LCAT3), pCGN9995 (LCAT4), pCGN10964 (LCAT5), pCGN10963 (*LRO1*), and pCGN8626 (ACAT). Double stranded DNA sequence was obtained to verify that no errors were introduced by the PCR amplification.

4A. Baculovirus Expression Constructs

Constructs are prepared to direct the expression of the *Arabidopsis* LCAT and yeast LCAT sequences in cultured insect cells. The entire coding region of the LCAT proteins was removed from the respective constructs by digestion with *Not*I and *Sse*8387I, followed by gel electrophoresis and gel purification. The fragments containing the LCAT coding sequences were cloned into *Not*I and *Pst*I digested baculovirus expression vector pFastBac1 (Gibco-BRL, Gaithersburg, MD). The resulting baculovirus expression constructs were referred to as pCGN9992 (LCAT1), pCGN9993 (LCAT2), pCGN9994 (LCAT3), pCGN10900 (LCAT4), pCGN10967 (LCAT5), and pCGN10962 (*LRO1*).

4B. Plant Expression Construct Preparation

A plasmid containing the napin cassette derived from pCGN3223 (described in U.S. Patent No. 5,639,790, the entirety of which is incorporated herein by reference) was modified to make it more useful for cloning large DNA fragments containing multiple restriction sites, and to allow the cloning of multiple napin fusion genes into plant binary transformation vectors. An adapter comprised of the self annealed oligonucleotide of sequence 5'-CGCGATTTAAATGGCGCGCCCTGCAGGCGGCCGCCTGCAGGGCGCGCCATTTAAAT-3' (SEQ ID NO:62) was ligated into the cloning vector pBC SK+ (Stratagene) after digestion with the restriction endonuclease BssHII to construct vector pCGN7765. Plamids pCGN3223 and pCGN7765 were digested with NotI and ligated together. The resultant vector, pCGN7770, contained the pCGN7765 backbone with the napin seed specific expression cassette from pCGN3223.

The cloning cassette, pCGN7787, contained essentially the same regulatory elements as pCGN7770, with the exception of the napin regulatory regions of pCGN7770 have been replaced with the double CAMV 35S promoter and the tml polyadenylation and transcriptional termination region.

A binary vector for plant transformation, pCGN5139, was constructed from pCGN1558 (McBride and Summerfelt, (1990) Plant Molecular Biology, 14:269-276). In pCGN5139, the polylinker of pCGN1558 was replaced as a HindIII/Asp718 fragment with a polylinker containing unique restriction endonuclease sites, AscI, PacI, XbaI, SwaI, BamHI, and NotI. The Asp718 and HindIII restriction endonuclease sites are retained in pCGN5139.

A series of turbo binary vectors was constructed to allow for the rapid cloning of DNA sequences into binary vectors containing transcriptional initiation regions (promoters) and transcriptional termination regions.

The plasmid pCGN8618 was constructed by ligating oligonucleotides 5'-TCGAGGATCCGCGGCCGCAAGCTTCCTGCAGG-3' (SEQ ID NO:63) and 5'-TCGACCTGCAGGAAGCTTGCGGCCGCGGATCC-3' (SEQ ID NO:64) into SalI/XhoI-digested pCGN7770. A fragment containing the napin promoter, polylinker and napin 3' region was excised from pCGN8618 by digestion with Asp718I; the fragment was blunt-ended by filling in the 5' overhangs with Klenow fragment then ligated into pCGN5139 that had been digested with Asp718I and HindIII and blunt-ended by filling in the 5' overhangs with Klenow fragment. A plasmid containing the insert oriented so that the napin promoter was closest to the blunted Asp718I site of pCGN5139 and the napin 3' was closest to the blunted HindIII site was subjected to sequence analysis to confirm both the insert orientation and the integrity of cloning junctions. The resulting plasmid was designated pCGN8622.

The plasmid pCGN8619 was constructed by ligating oligonucleotides 5'-TCGACCTGCAGGAAGCTTGCGGCCGCGGATCC-3' (SEQ ID NO:65) and 5'-TCGAGGATCCGCGGCCGCAAGCTTCCTGCAGG-3' (SEQ ID NO:66) into SalI/XhoI-digested pCGN7770. A fragment containing the napin promoter, polylinker and napin 3' region was removed from pCGN8619 by digestion with Asp718I; the fragment was blunt-ended by filling in the 5' overhangs with Klenow fragment then ligated into pCGN5139 that had been digested with Asp718I and HindIII and blunt-ended by filling in the 5' overhangs with Klenow fragment. A plasmid containing the insert oriented so that the napin promoter was closest to the blunted Asp718I site of pCGN5139 and the napin 3' was closest to the blunted HindIII site was subjected to sequence analysis to confirm both the insert orientation and the integrity of cloning junctions. The resulting plasmid was designated pCGN8623.

The plasmid pCGN8620 was constructed by ligating oligonucleotides 5'-TCGAGGATCCGCGGCCGCAAGCTTCCTGCAGGAGCT -3' (SEQ ID NO:67) and 5'-CCTGCAGGAAGCTTGCGGCCGCGGATCC-3' (SEQ ID NO:68) into SalI/SacI-digested pCGN7787. A fragment containing the d35S promoter, polylinker and tml 3' region was removed from pCGN8620 by complete digestion with Asp718I and partial digestion with NotI. The fragment was blunt-ended by filling in the 5' overhangs with Klenow fragment then ligated into pCGN5139 that had been digested with Asp718I and HindIII and blunt-ended by filling in the 5' overhangs with Klenow fragment. A plasmid containing the insert oriented so that the d35S promoter was closest to the blunted Asp718I site of pCGN5139 and the tml 3' was closest to the blunted HindIII site was subjected to sequence analysis to confirm both the insert orientation and the integrity of cloning junctions. The resulting plasmid was designated pCGN8624.

The plasmid pCGN8621 was constructed by ligating oligonucleotides 5'-TCGACCTGCAGGAAGCTTGCGGCCGCGGATCCAGCT -3' (SEQ ID NO:69) and 5'-GGATCCGCGGCCGCAAGCTTCCTGCAGG-3' (SEQ ID NO:70) into SalI/SacI-digested pCGN7787. A fragment containing the d35S promoter, polylinker and tml 3' region was removed from pCGN8621 by complete digestion with Asp718I and partial digestion with NotI. The fragment was blunt-ended by filling in the 5' overhangs with Klenow fragment then ligated into pCGN5139 that had been digested with Asp718I and HindIII and blunt-ended by filling in the 5' overhangs with Klenow fragment. A plasmid containing the insert oriented so that the d35S promoter was closest to the blunted Asp718I site of pCGN5139 and the tml 3' was closest to the blunted HindIII site was subjected to sequence analysis to confirm both the insert orientation and the integrity of cloning junctions. The resulting plasmid was designated pCGN8625.

The plasmid construct pCGN8640 is a modification of pCGN8624 described above. A 938bp PstI fragment isolated from transposon Tn7 which encodes bacterial spectinomycin and streptomycin resistance (Fling et al. (1985), Nucleic Acids Research 13(19):7095-7106), a determinant for E. coli and Agrobacterium selection, was blunt ended with Pfu polymerase. The blunt ended fragment was ligated into pCGN8624 that had been digested with SpeI and blunt ended with Pfu polymerase. The region containing the PstI fragment was sequenced to confirm both the insert orientation and the integrity of cloning junctions.

The spectinomycin resistance marker was introduced into pCGN8622 and pCGN8623 as follows. A 7.7 Kbp AvrII-SnaBI fragment from pCGN8640 was ligated to a 10.9 Kbp AvrII-SnaBI fragment from pCGN8623 or pCGN8622, described above. The resulting plasmids were pCGN8641 and pCGN8643, respectively.

The plasmid pCGN8644 was constructed by ligating oligonucleotides 5'-GATCACCTGCAGGAAGCTTGCGGCCGCGGATCCAATGCA-3' (SEQ ID NO:71) and 5'-TTGGATCCGCGGCCGCAAGCTTCCTGCAGGT-3' (SEQ ID NO:72) into BamHI-PstI digested pCGN8640.

4C. Plant LCAT Expression Construct Preparation

The coding sequence of LCAT1 was cloned from pCGN9964 as a *Not*I/ *Sse*8387I fragment into pCGN8640, pCGN8641, pCGN8643, and pCGN8644 to create the expression constructs pCGN9960, pCGN9961, pCGN9962, and pCGN9963, respectively. The construct pCGN9960 was designed to express the LCAT1 coding sequence in the sense orientation from the constitutive promoter CaMV 35S. The construct pCGN9961 was designed to express the LCAT1 coding sequence in the antisense orientation from the napin promoter. The construct pCGN9962 was designed to express the LCAT1 coding sequence in the sense orientation from the napin promoter. The construct pCGN9963 was designed to express the LCAT1 coding sequence in the antisense orientation from the constitutive promoter CaMV 35S.

The coding sequence of LCAT2 was cloned from pCGN9985 as a *Not*I/ *Sse*8387I fragment into pCGN8640, pCGN8641, pCGN8643, and pCGN8644 to create the expression constructs pCGN9981, pCGN9982, pCGN9983, and pCGN9984, respectively. The construct pCGN9981 was designed to express the LCAT2 coding sequence in the sense orientation from the constitutive promoter CaMV 35S. The construct pCGN9982 was designed to express the LCAT2 coding sequence in the antisense orientation from the napin promoter. The construct pCGN9983 was designed to express the LCAT2 coding sequence in the sense orientation from the napin promoter. The construct pCGN9984 was designed to express the LCAT2 coding sequence in the antisense orientation from the constitutive promoter CaMV 35S.

The coding sequence of LCAT3 was cloned from pCGN9965 as a *Not*I/ *Sse*8387I fragment into pCGN8640, pCGN8641, pCGN8643, and pCGN8644 to create the expression constructs pCGN9966, pCGN9967, pCGN9968, and pCGN9969, respectively. The construct pCGN9966 was designed to express the LCAT3 coding sequence in the sense orientation from the constitutive promoter CaMV 35S. The construct pCGN9967 was designed to express the LCAT3 coding sequence in the antisense orientation from the napin promoter. The construct pCGN9968 was designed to express the LCAT3 coding sequence in the sense orientation from the napin promoter. The construct pCGN9969 was designed to express the LCAT3 coding sequence in the antisense orientation from the constitutive promoter CaMV 35S.

The coding sequence of LCAT4 was cloned from pCGN9995 as a *Not*I/ *Sse*8387I fragment into pCGN8640, pCGN8641, pCGN8643, and pCGN8644 to create the expression constructs pCGN9996, pCGN9997, pCGN9998, and pCGN9999, respectively. The construct pCGN9996 was designed to express the LCAT4 coding sequence in the sense orientation from the constitutive promoter CaMV 35S. The construct pCGN9997 was designed to express the LCAT4 coding sequence in the antisense orientation from the napin promoter. The construct pCGN9998 was designed to express the LCAT4 coding sequence in the sense orientation from the napin promoter. The construct pCGN9999 was designed to express the LCAT4 coding sequence in the antisense orientation from the constitutive promoter CaMV 35S.

The coding sequence of LCAT5 was cloned from pCGN10964 as a *Not*I/ *Sse*8387I fragment into pCGN9977 and pCGN9979, to create the expression constructs pCGN 10965, and pCGN10966, respectively. The construct pCGN 10965 was designed to express the LCAT5 coding sequence in the sense orientation from the constitutive promoter CaMV 35S. The construct pCGN10966 was designed to express the LCAT5 coding sequence in the sense orientation from the napin promoter.

The coding sequence of *LRO1* was cloned from pCGN10963 as a *Not*I/ *Sse*8387I fragment into pCGN9977 and pCGN9979, to create the expression constructs pCGN10960, and pCGN10961, respectively. The construct pCGN10960 was designed to express the *LRO1* coding sequence in the sense orientation from the constitutive promoter CaMV 35S. The construct pCGN10961 was designed to express the *LRO1* coding sequence in the sense orientation from the napin promoter.

4D (Reference Example). Plant ACAT Expression Construct Preparation

A fragment containing the *Arabidopsis* ACAT-like coding region was removed from pCGN8626 by digestion with Sse8387I and Not I. The fragment containing the ACAT-like sequence was ligated into PstI-Not I digested pCGN8622. The resulting plasmid was designated pCGN8627. DNA sequence analysis confirmed the integrity of the cloning junctions.

A fragment containing the *Arabidopsis* ACAT-like coding region (SEQ ID NO: 42) was removed from pCGN8626 by digestion with Sse8387I and Not I. The fragment was ligated into PstI-Not I digested pCGN8623. The resulting plasmid was designated pCGN8628. DNA sequence analysis confirmed the integrity of the cloning junctions.

A fragment containing the *Arabidopsis* ACAT-like coding region was removed from pCGN8626 by digestion with Sse8387 and Not I. The fragment was ligated into PstI-Not I digested pCGN8624. The resulting plasmid was designated pCGN8629. DNA sequence analysis confirmed the integrity of the cloning junctions.

A fragment containing the *Arabidopsis* ACAT-like coding region was removed from pCGN8626 by digestion with Sse8387 and Not I. The fragment was ligated into PstI-Not I digested pCGN8625. The resulting plasmid was designated pCGN8630. DNA sequence analysis confirmed the integrity of the cloning junctions.

An additional expression construct for the suppression of endogenous ACAT-like activity was also prepared. The construct pCGN8660 was constructed by cloning approximately 1 Kb of the *Arabidopsis* ACAT-like coding region from pCGN8626 in the sense orientation, and the full-length *Arabidopsis* ACAT-like coding region in the antisense orientation under the regulatory control of the napin transcription initiation sequence.

For expression of the rat ACAT-like sequence in plants, the NotI-Sse8387I fragment of pCGN8592 was cloned into NotI-PstI digested binary vectors pCGN8621, pCGN8622, and pCGN8624 to yield plasmids, pCGN 9700, pCGN9701, and pCGN9702, respectively. Plasmid pCGN9700 expresses a sense transcript of the rat ACAT-like cDNA under control of a napin promoter, plasmid pCGN9701 expresses an antisense transcript of the rat ACAT-like cDNA under control of a napin promoter, and plasmid pCGN9702 expresses a sense transcript of the rat ACAT-like cDNA under control of a double 35S promoter. Plasmids pCGN 9700, pCGN9701, and pCGN9702 were introduced in *Agrobacterium tumefaciens* EHA101.

Constructs were prepared to direct the expression of the rat ACAT-like sequence in the seed embryo of soybean and the endosperm of corn. For expression of the rat ACAT-like DNA sequence in soybean, a 1.5 kb *Not*I/*Sse*8387I fragment from pCGN8592 containing the coding sequence of the rat ACAT-like sequence was blunt ended using Mung bean nuclease, and ligated into the *Sma*I site of the turbo 7S binary/cloning vector pCGN8809 to create the vector pCGN8817 for transformation into soybean by particle bombardment. The vector pCGN8817 contained the operably linked components of the promoter region of the soybean α' subunit of β-conglycinin (7S promoter, (Chen et al., (1986), Proc. Natl. Acad. Sci., 83:8560-8564), the DNA sequence coding for the entire rat ACAT-like protein, and the transcriptional termination region of pea RuBisCo small subunit, referred to as E9 3' (Coruzzi; et al. (1984) EMBO J. 3:1671-1679 and Morelli, et al. (1985) Nature 315:200-204). This construct further contained sequences for the selection of positive transformed plants by screening for resistance to glyphosate using the CP4 EPSPS (U.S. Patent 5,633,435) expressed under the control of the figwort mosaic virus (FMV) promoter (U.S. Patent Number 5,378,619) and the transcriptional termination region of E9.

For expression of the rat ACAT-like sequence in the corn endosperm, a 1.5 kb *Not*I/*Sse*8387I fragment from pCGN8592 containing the coding sequence of the rat ACAT-like sequence was blunt ended using Mung bean nuclease, and ligated into the *Bam*HI site of the rice pGt1 expression cassette pCGN8592 for expression from the pGt1 promoter (Leisy, D.J. et al., Plant Mol. Biol. 14 (1989) 41-50) and the HSP70 intron sequence (U.S. Patent Number 5,593,874). This cassette also included the transcriptional termination region downstream of the cloning site of nopaline synthase, *nos* 3' (Depicker et al., J. Molec. Appl. Genet. (1982) 1: 562-573). A 7.5 kb fragment containing the pGt1 promoter, the DNA sequence encoding the rat ACAT-like protein, and the *nos* transcriptional termination sequence was cloned into the binary vector pCGN8816 to create the vector pCGN8818 for transformation into corn. This construct also contained sequences for the selection of positive transformants with kanamycin using the kanamycin resistance gene from Tn5 bacteria under the control of the CAMV 35S promoter and tml transcriptional termination regions.

**Example 5:** Expression in Insect Cell Culture

A baculovirus expression system was used to express the LCAT cDNAs in cultured insect cells. The baculovirus expression constructs pCGN9992, pCGN9993, pCGN9994, pCGN10900, pCGN10962, and pCGN10967 were transformed and expressed using the BAC-to-BAC Baculovirus Expression System (Gibco-BRL, Gaithersburg, MD) according to the manufacturer's directions.

The transformed insect cells were used to assay for acyltransferase activities using methods known in the art (see Example 8).

**Example 6:** Plant Transformation

A variety of methods have been developed to insert a DNA sequence of interest into the genome of a plant host to obtain the transcription or transcription and translation of the sequence to effect phenotypic changes. Transgenic plants were obtained by *Agrobacterium-mediated* transformation as described by Radke *et al.* (Theor. Appl. Genet. (1988) 75:685-694; Plant Cell Reports (1992) 11:499-505). Alternatively, microprojectile bombardment methods, such as described by Klein et al. (Bio/Technology 10:286-291) may also be used to obtain nuclear transformed plants. Other plant species may be similarly transformed using related techniques.

The plant binary constructs described above were used in plant transformation to direct the expression of the sterol acyltransferases in plant tissues. Binary vector constructs were transformed into strain EHA101 *Agrobacterium* cells (Hood et al., J. Bacteriol (1986) 168:1291-1301), by the method of Holsters et al. (Mol. Gen. Genet. (1978) 163: 181-187). Transgenic *Arabidopsis thaliana* plants were obtained by *Agrobacterium*-mediated transformation as described by Valverkens et al., (Proc. Nat. Acad. Sci. (1988) 85:5536-5540), Bent et al. ((1994), Science 265:1856-1860), and Bechtold et al. ((1993), C. R.Acad. Sci., Life Sciences 316:1194-1199).

**Example 7:** Plant Assays for Modified Sterol Content/Profile

7A: NMR of T2 seed

Seed from plants expressing LCAT 1 through 4 under the control of the napin promoter were analyzed by NMR. Arabidopsis seeds from transgenic plants were placed directly into wide-mouth MAS NMR sample tubes.

High-resolution spectra were measured at 11.7 T (1H=500 MHz, 13C=125 mHz) using Varian NMR Instruments (Palo Alto, CA) Inova™ NMR spectrometers equipped with carbon-observe MAS Nanoprobes™. The 13C spectra were acquired without a field-frequency lock at ambient temperature (approx. 21-22°C) for 14 hours using the following conditions: spectral width = 29.996 kHz, acquisition time = 2.185 seconds, p/2 pulse (3.8 ms) with no relaxation delay, 1H g B2 = 2.5 kHz with Waltz decoupling. Data processing conditions were typically: digital resolution = 0.11 Hz, 0.3 to 1.5 Hz line broadening and time-reversed linear prediction of the first three data points. Chemical shifts were referenced by adding neat tetramethylsilane (TMS) to Arabidopsis seeds and using the resulting referencing parameters for subsequent spectra. The 13C resolution was 2-3 Hz for the most narrow seed resonances. Spectral resolution was independent of MAS spinning speeds (1.5-3.5 kHz) and data were typically obtained with 1.5 kHz spinning speeds. Spinning sidebands were approx. 1% of the main resonance. Phytosterol 13C assignments were based on model samples composed of triolein, β-sitosterol and cholesterol oleate. Triacylglycerol 13C assignments were made from comparison with literature assignments or with shifts computed from a 13C NMR database (Advanced Chemical Development, Inc., version 3.50, Toronto Canada).

The results of these analyses are displayed in Figure 2 and show that there was a trend of an approximately 2 fold increase of phytosterols in the seeds derived from plant line 5 expressing the LCAT 4 gene (pCGN9998) under the control of the napin promoter. During the course of this analysis it was also noted that the average oil content of seed from plants expressing the LCAT2 construct (pCGN9983) under the control of the napin promoter was higher than that of controls. This is the first *in planta* evidence supporting the concept that overexpression of a nucleotide sequence encoding a lecithin:cholesterol acyltransferase-like polypeptide can increase oil content.

7B: HPLC/MS of T2 seed

Seed oil from T2 plants expressing LCAT1 through 4 under the control of the napin promoter was extracted using an accelerated solvent extractor (ASE) method. Seed samples were ground, using a mortar and pestle, to achieve a fine homogeneous meal. Oil was obtained using a Dionex Accelerated Solvent Extractor (ASE). Clean ground seed was added to an equal amount of diatomaceous earth. The ground seed sample and the diatomaceous earth were thoroughly mixed until a homogeneous texture was achieved. The sample was then loaded into the instrument and oil extraction was achieved using hexane under validated laboratory protocols.

Oil from these seed samples was then analyzed for sterol ester analysis using HPLC/MS for free campesterol, stigmasterol, and sitosterol and their fatty acid esters. To the autosampler vial containing approximately 0.1 grams oil was added 0.3 mLs $CDCl_3$. One-hundred microliters of this solution was added to 900 microliters $CHCl_3$. Five microliters of this diluted sample was subsequently injected into an HPLC/MS with positive ion atmospheric pressure ionization. The individual components in the oils were separated using two 4.6 x 50 mm $C_8$ Zorbax columns in series and a gradient using acetonitrile and acetonitrile with 40% $CHCl_3$. The sterol concentrations were calculated assuming each sterol and its fatty acids have the same molar responses. This was observed to be the case with cholesterol and its esters and was assumed to be the case for campesterol, stigmasterol, and sitosterol. In the present study, the sterol identified as stigmasterol was actually an isomer of this compound.

The results of these analyses are displayed in Figures 3 and 4 and show that there were sterol ester enhancements on the order of 50%. in the seeds derived from six out of seven T2 plant lines expressing LCAT3 (pCGN9968) under the control of the napin promoter.

**Example 8:** Baculovirus Insect Cell Culture for Sterol Esterification Activity

Baculovirus expression construct pCGN9992, pCGN9993, pCGN9994 and pCGN10900 (see Example 4) were transformed and expressed using the BAC-TOBAC Baculovirus Expression System (Gibco-BRL, Gaithersburg, MD) according to the manufacturer's instructions except harvesting of recombinant viruses was done 5 days post-transfection. The supernatant from the transfection mixture was used for generating virus stock which in turn was used for infecting Sf9 cells used in the assay.

The transformed cells were assayed for lecithin:sterol acyltransferase activities using the method described herein. Insect cells were centrifuged and the resulting cell pellet was either used immediately or stored at -70 C for later analysis. Cells were resuspended in Medium A (100 mM Tricine/NaOH, pH 7.8, 10% (w/v) glycerol, 280 mM NaCl with : 0.1 μM Aprotinin, 1 μM Leupeptin, and 100 μM Pefabloc (all from Boehringer Mannheim, Germany) and lysed by sonication (2 x 10 sec). Cell walls and other debris were pelleted by centrifugation (14,000 x g , 10 min, 4°C). The supernatant was transferred to a new vial and membranes pelleted by centrifugation (100,000 x g, Ti 70.1 rotor, 46,000 rpm for 1 hour at 4°C). Total membranes were resuspended in Medium A. Lecithin:sterol acyltransferase activity was assayed in a 0.1 ml reaction mixture containing 100 mM Tris/HCl, pH 7, 28 mM NaCl, 0.03% Triton X-100, 0.1 mM sitosterol, 20 μM 1,2-[$^{14}$C]-palmitoyl-phosphatidyl choline (246420 dpm/nmole), and 0.05-20 mg of membrane protein. After 15 minutes at 30 °C, the reaction was terminated by addition of a 0.5 ml solution of methylene chloride:methanol (4:1, v/v ) containing 100 μg cholesterol and cholesterol ester as cold carriers. A portion (0.1 ml) of the bottom organic layer was removed and evaporated under nitrogen gas. The concentrated extract was resuspended in 30 μl of hexane and spotted onto a silica gel-G thin layer chromatographic plate. The plate was migrated in hexane:diethyl ether:acetic acid (80:20:1) to the top, then air dried. Radioactivity was determined by exposure to a Low Energy Phosphor-imaging Screen. Following exposure, the screen was read on a phosphorimager.

The LCAT 4 protein from pCGN 10900 in baculovirus membranes showed a radioactive spot in the region of the TLC plate where cholesterol ester migrates indicating that LCAT 4 has the ability to catalyze the transfer of an acyl group from lecithin (PC) to sitosterol to make a sitosterol ester.

**Example 9:** Plant Assay for Modified Lipid Content

Nir (near infrared spectroscopy spectral scanning) can be used to determine the total oil content of Arabidopsis seeds in a non-destructive way provided that a spectral calibration curve has been developed and validated for seed oil content. A seed oil spectral calibartion curve was developed using seed samples from 85 Arabidopsis plants. Seed was cleaned and scanned using a Foss NIR model 6500 (Foss-Nirs Systems, Inc.). Approximately 50 to 100 milligrams of whole seeds, per sample, were packed in a mini sample ring cup with quartz lens [ IH-0307 ] consisting a mini-insert [ IH-0337 ] and scanned in reflectance mode to obtain the spectral data. The seed samples were then ground, using a mortar and pestle, to achieve a fine homogeneous meal. The ground samples were measured for oil using an accelerated solvent extractor (ASE).

Measurement for the total oil content was performed on the Dionex Accelerated Solvent Extractor (ASE). Approximately 500 mg of clean ground seed was weighed to the nearest 0.1 mg onto a 9 x 9 cm weigh boat. An equal amount of diatomaceous earth was added using a top-loading balance accurate to the nearest 0.01 g. The ground seed sample and the diatomaceous earth were thoroughly mixed until a homogeneous texture was achieved. The sample was loaded on to the instrument and oil extraction was achieved using hexane under validated laboratory protocols. Standard Rapeseed samples were obtained from the Community Bureau of Reference (BCR). The ASE extraction method was validated using the BCR reference standards. A total percent oil recovery of 99% to 100% was achieved. "As-is" oil content was calculated to the nearest 0.01 mass percentage using the formula:

$$\text{Oil Content} = 100\% \times (\text{vial plus extracted oil wt} - \text{initial vial wt}) / (\text{sample wt})$$

The analytical data generated by ASE were used to perform spectral calibrations. Nir calibration equations were generated using the built-in statistical package within the NirSytems winisi software. The spectral calibration portion of the software is capable of calibration and self-validation. From a total of 85 samples, 57 samples were used to generate the total percent oil calibration. The remaining samples were used to validate the oil calibrations. Optimized smoothing, derivative size, and mathematical treatment (modified partial least square) was utilized to generate the calibration. The samples that were not used in building respective calibrations were used as a validation set. Statistical tools such as correlation coefficient ( R ), coefficient of determination ($R^2$), standard error of prediction ( SEP ), and the standard error of prediction corrected for bias (SEPC) were used to evaluate the calibration equations.

T2 seeds from plants that had been transformed with the LCAT genes were cleaned and scanned using a Foss NIR model 6500 (Foss-Nirs Systems, Inc.). Approximately 50 to 100 milligrams of whole seeds, per sample, were packed in a mini sample ring cup with quartz lens [ IH-0307 ] consisting a mini-insert [ IH-0337 ] and scanned in reflectance mode to obtain the spectral data. Oil percentage in each seed sample was determined using the seed oil spectral calibration curve detailed above.

The results of these analyses are found in Figure 5 and Table 2 and show that there was a significant increase in the oil level in seed from T2 plants expressing the LCAT2 gene. This increase in oil was seen in plants when LCAT2 was driven by either the 35S constitutive promoter or the seed-specific napin promoter. These results show that overexpression of a nucleic acid sequence encoding a lecithin:cholesterol acyltransferase-like polypeptide can increase seed oil production in plants.

**Table 2**

| | Construct number | Seed Oil Percentage (%) |
|---|---|---|
| CONTROL | | 24.7 |
| CONTROL | | 28.0 |
| CONTROL | | 31.8 |
| CONTROL | | 32.4 |
| NAPIN LCAT1 | PCGN9962 | 28.5 |
| NAPIN LCAT1 | PCGN9962 | 28.9 |
| NAPIN LCAT1 | PCGN9962 | 29.6 |
| NAPIN LCAT1 | PCGN9962 | 30.1 |
| NAPIN LCAT1 | PCGN9962 | 30.1 |
| NAPIN LCAT1 | PCGN9962 | 30.1 |
| NAPIN LCAT1 | PCGN9962 | 30.8 |
| NAPIN LCAT1 | PCGN9962 | 31.0 |
| NAPIN LCAT1 | pCGN9962 | 32.1 |
| NAPIN LCAT1 | pCGN9962 | 34.2 |
| NAPIN LCAT3 | pCGN9968 | 26.8 |
| NAPIN LCAT3 | pCGN9968 | 27.4 |
| NAPIN LCAT3 | pCGN9968 | 29.0 |
| NAPIN LCAT3 | pCGN9968 | 29.0 |
| NAPIN LCAT3 | pCGN9968 | 32.6 |
| NAPIN LCAT2 | pCGN9983 | 26.5 |
| NAPIN LCAT2 | pCGN9983 | 34.7 |
| NAPIN LCAT2 | pCGN9983 | 34.8 |
| NAPIN LCAT2 | pCGN9983 | 35.7 |
| NAPIN LCAT2 | pCGN9983 | 35.8 |
| NAPIN LCAT2 | pCGN9983 | 36.3 |
| NAPIN LCAT2 | pCGN9983 | 36.7 |
| NAPIN LCAT2 | pCGN9983 | 37.0 |
| NAPIN LCAT2 | pCGN9983 | 37.2 |
| NAPIN LCAT2 | pCGN9983 | 37.3 |
| NAPIN LCAT2 | pCGN9983 | 37.3 |
| NAPIN LCAT2 | pCGN9983 | 37.4 |
| NAPIN LCAT2 | pCGN9983 | 37.8 |
| NAPIN LCAT2 | pCGN9983 | 38.0 |
| NAPIN LCAT2 | pCGN9983 | 38.0 |
| 35S LCAT2 | pCGN9981 | 27.3 |
| 35S LCAT2 | pCGN9981 | 28.1 |
| 35S LCAT2 | pCGN9981 | 28.2 |
| 35S LCAT2 | pCGN9981 | 28.6 |
| 35S LCAT2 | pCGN9981 | 29.8 |
| 35S LCAT2 | pCGN9981 | 30.3 |
| 35S LCAT2 | pCGN9981 | 32.4 |
| 35S LCAT2 | pCGN9981 | 32.5 |
| 35S LCAT2 | pCGN9981 | 33.6 |
| 35S LCAT2 | pCGN9981 | 34.1 |
| 35S LCAT2 | pCGN9981 | 35.5 |
| 35S LCAT2 | pCGN9981 | 36.4 |
| 35S LCAT2 | pCGN9981 | 37.1 |
| 35S LCAT2 | pCGN9981 | 38.3 |
| 35S LCAT2 | pCGN9981 | 38.5 |

(continued)

| | Construct number | Seed Oil Percentage (%) |
|---|---|---|
| 35S LCAT2 | pCGN9981 | 39.1 |

In light of the detailed description of the invention and the examples presented above, it can be appreciated that the several aspects of the invention are achieved.

## Claims

1. An isolated nucleic acid sequence comprising a coding sequence that encodes a plant lecithin:cholesterol acyltransferase-like polypeptide that is capable of utilizing lecithin as an acyl donor for acylation of sterols or glycerides to form esters, wherein the coding sequence comprises a polynucleotide selected from:

   a) an isolated polynucleotide encoding the polypeptide of SEQ ID NO: 5;
   b) an isolated polynucleotide sequence comprising SEQ ID NO: 4;
   c) an isolated polynucleotide having at least 95% sequence identity with SEQ ID NO: 4;
   d) an isolated polynucleotide complementary to a polynucleotide of (a), (b) or (c); and
   e) an isolated polynucleotide that hybridizes under stringent conditions to SEQ ID NO: 4.

2. A recombinant nucleic acid construct comprising a heterologous regulatory sequence operably linked to a polynucleotide encoding a lecithin:cholesterol acyltransferase-like polypeptide of claim 1.

3. The recombinant construct of claim 2, wherein said heterologous regulatory sequence is functional in a plant cell.

4. The recombinant construct of claim 2, wherein said heterologous regulatory sequence

   (i) comprises a constitutive promoter; or
   (ii) comprises an inducible promoter; or
   (iii) is selected from a tissue specific promoter, a developmentally regulated promoter, an organelle specific promoter, and a seed specific promoter.

5. The recombinant construct of claim 2, further comprising a termination sequence.

6. A plant cell containing the recombinant construct of claim 2.

7. The plant cell of claim 6, which expresses the lecithin:cholesterol acyltransferase-like polypeptide encoded by the nucleic acid sequence of claim 1.

8. A plant comprising the recombinant construct of claim 2 and/or comprising at least one plant cell of claim 6, or the progeny of said plant or a seed from said plant.

9. The plant of claim 8, wherein expression of said recombinant construct results in modified sterol composition of said plant as compared to the same plant without said recombinant construct.

**10.** The plant of claim 9, wherein said recombinant construct comprises

(i) a polynucleotide sequence encoding a lecithin:cholesterol acyltransferase-like polypeptide that has SEQ ID NO:4; or
(ii) a regulatory sequence comprising a tissue specific promoter; or
(iii) a regulatory sequence comprising a seed specific promoter.

**11.** The plant of claim 9, wherein said modified sterol composition is an increase in sterol esters.

**12.** The plant of claim 8, wherein expression of said recombinant construct results in an altered production of oil by said plant as compared to the same plant without said recombinant construct.

**13.** The plant of claim 12, wherein said oil production is increased.

**14.** The plant of claim 12, wherein said recombinant construct comprises

(i) a polynucleotide sequence encoding a lecithin:cholesterol acyltransferase-like polypeptide that has SEQ ID NO: 4; or
(ii) a regulatory sequence, which is a tissue specific promoter; or
(iii) regulatory sequence, which is a seed specific promoter.

**15.** A purified polypeptide comprising an amino acid sequence of SEQ ID NO: 5.

**16.** A method for producing a lecithin:cholesterol acyltransferase-like polypeptide encoded by the nucleic acid sequence of claim 1 comprising culturing a plant cell of claim 6 under conditions permitting expression of said lecithin:cholesterol acyltransferase-like polypeptide.

**17.** The method of claim 16, further comprising isolating the cholesterol acyltransferase-like polypeptide from the host cell or from the medium in which the host cell is cultured.

**18.** A method for modifying the sterol content of a plant cell, comprising transforming a plant cell with a recombinant construct of claim 2 containing a regulatory sequence operably linked to a polynucleotide encoding a lecithin: cholesterol acyltransferase-like polypeptide encoded by the nucleic acid sequence of claim 1 and culturing said plant cell under conditions wherein said plant cell expresses said lecithin: cholesterol acyltransferase-like polypeptide such that said plant cell has a modified sterol composition as compared to plant cells without the recombinant construct.

**19.** The method of claim 18, wherein said regulatory sequence

(i) comprises a constitutive promoter; or
(ii) is an inducible promoter; or
(iii) is a tissue specific promoter; or
(iv) is a seed specific promoter.

**20.** A method for altering oil production by a plant cell comprising, transforming a plant cell with a recombinant construct of claim 2 containing a regulatory sequence operably linked to a polynucleotide encoding a lecithin:cholesterol acyltransferase-like polypeptide encoded by the nucleic acid sequence of claim 1 and culturing said plant cell under conditions wherein said plant cell expresses said lecithin:cholesterol acyltransferase-like polypeptide such that said plant cell has an altered oil production as compared to plant cells without the recombinant construct.

**21.** The method of claim 20, wherein said oil production is increased.

**22.** The method of claim 20, wherein said construct comprises

(i) a polynucleotide sequence encoding a lecithin:cholesterol acyltransferase-like polypeptide that has SEQ ID NO:4; or

(ii) a regulatory sequence that is a tissue specific promoter; or
(iii) a regulatory sequence that is a seed specific promoter.

**Patentansprüche**

1. Isolierte Nucleinsäuresequenz, die eine codierende Sequenz umfasst, welche ein Pflanzen-Lecithin:Cholesterin-Acyltransferase-artiges Polypeptid codiert, das Lecithin als Acyldonor für die Acylierung von Sterolen oder Glyceriden unter Bildung von Estern nutzen kann, wobei die codierende Sequenz ein Polynucleotid umfasst, das ausgewählt ist aus:

    a) einem isolierten Polynucleotid, das das Polypeptid von SEQ ID Nr. 5 codiert;
    b) einer isolierten Polynucleotidsequenz, die SEQ ID Nr. 4 umfasst;
    c) einem isolierten Polynucleotid, das wenigstens 95% Sequenzidentität mit SEQ ID Nr. 4 aufweist;
    d) einem isolierten Polynucleotid, das komplementär zu einem Polynucleotid gemäß (a), (b) oder (c) ist; und
    e) einem isolierten Polynucleotid, das unter stringenten Bedingungen mit SEQ ID Nr. 4 hybridisiert.

2. Rekombinantes Nucleinsäurekonstrukt, das eine heterologe regulatorische Sequenz umfasst, die funktionell mit einem Polynucleotid, das ein Lecithin:Cholesterin-Acyltransferase-artiges Polypeptid codiert, gemäß Anspruch 1 verknüpft ist.

3. Rekombinantes Konstrukt gemäß Anspruch 2, wobei die heterologe regulatorische Sequenz in einer Pflanzenzelle funktionsfähig ist.

4. Rekombinantes Konstrukt gemäß Anspruch 2, wobei die heterologe regulatorische Sequenz

    (i) einen konstitutiven Promotor umfasst; oder
    (ii) einen induzierbaren Promotor umfasst; oder
    (iii) aus einem gewebespezifischen Promotor, einem entwicklungsabhängig regulierten Promotor, einem organellspezifischen Promotor und einem samenspezifischen Promotor ausgewählt ist.

5. Rekombinantes Konstrukt gemäß Anspruch 2, das weiterhin eine Terminatorsequenz umfasst.

6. Pflanzenzelle, die das rekombinante Konstrukt gemäß Anspruch 2 enthält.

7. Pflanzenzelle gemäß Anspruch 6, die das von der Nucleinsäuresequenz gemäß Anspruch 1 codierte Lecithin: Cholesterin-Acyltransferase-artige Polypeptid exprimiert.

8. Pflanze, die das rekombinante Konstrukt gemäß Anspruch 2 umfasst und/oder wenigstens eine Pflanzenzelle gemäß Anspruch 6 umfasst, oder die Nachkommen einer solchen Pflanze oder ein Samen von einer solchen Pflanze.

9. Pflanze gemäß Anspruch 8, wobei die Expression des rekombinanten Konstrukts zu einer modifizierten Sterolzusammensetzung der Pflanze im Vergleich zu derselben Pflanze ohne das rekombinante Konstrukt führt.

10. Pflanze gemäß Anspruch 9, wobei das rekombinante Konstrukt Folgendes umfasst:

    (i) eine Polynucleotidsequenz, die ein Lecithin:Cholesterin-Acyltransferase-artiges Polypeptid codiert, das SEQ ID Nr. 4 aufweist; oder
    (ii) eine regulatorische Sequenz, die einen gewebespezifischen Promotor umfasst; oder
    (iii) eine regulatorische Sequenz, die einen samenspezifischen Promotor umfasst.

11. Pflanze gemäß Anspruch 9, wobei es sich bei der modifizierten Sterolzusammensetzung um eine Erhöhung des Anteils von Sterolestern handelt.

12. Pflanze gemäß Anspruch 8, wobei die Expression des rekombinanten Konstrukts zu einer veränderten Produktion von Öl durch die Pflanze im Vergleich zu derselben Pflanze ohne das rekombinante Konstrukt führt.

13. Pflanze gemäß Anspruch 12, wobei die Ölproduktion erhöht ist.

**14.** Pflanze gemäß Anspruch 12, wobei das rekombinante Konstrukt Folgendes umfasst:

(i) eine Polynucleotidsequenz, die ein Lecithin:Cholesterin-Acyltransferase-artiges Polypeptid codiert, das SEQ ID Nr. 4 aufweist; oder
(ii) eine regulatorische Sequenz, die einen gewebespezifischen Promotor umfasst; oder
(iii) eine regulatorische Sequenz, die einen samenspezifischen Promotor umfasst.

**15.** Gereinigtes Polypeptid, das eine Aminosäuresequenz von SEQ ID Nr. 5 umfasst.

**16.** Verfahren zur Herstellung eines Lecithin:Cholesterin-Acyltransferaseartigen Polypeptids, das durch die Nucleinsäuresequenz gemäß Anspruch 1 codiert wird, umfassend das Kultivieren einer Pflanzenzelle gemäß Anspruch 6 unter Bedingungen, die die Expression des Lecithin:Cholesterin-Acyltransferase-artigen Polypeptids ermöglichen.

**17.** Verfahren gemäß Anspruch 16, das weiterhin das Isolieren des Cholesterin-Acyltransferase-artigen Polypeptids aus der Wirtszelle oder aus dem Medium, in dem die Wirtszelle kultiviert wird, umfasst.

**18.** Verfahren zum Modifizieren des Sterolgehalts einer Pflanzenzelle, umfassend das Transformieren einer Pflanzenzelle mit einem rekombinanten Konstrukt gemäß Anspruch 2, das eine regulatorische Sequenz umfasst, die funktionell mit einem Polynucleotid verknüpft ist, das ein von der Nucleinsäuresequenz gemäß Anspruch 1 codiertes Lecithin:Cholesterin-Acyltransferase-artiges Polypeptid codiert, und das Kultivieren der Pflanzenzelle unter Bedingungen, bei denen die Pflanzenzelle das Lecithin:Cholesterin-Acyltransferase-artige Polypeptid exprimiert, so dass die Pflanzenzelle eine modifizierte Sterolzusammensetzung im Vergleich zu Pflanzenzellen ohne das rekombinante Konstrukt aufweist.

**19.** Verfahren gemäß Anspruch 18, wobei die regulatorische Sequenz

(i) einen konstitutiven Promotor umfasst; oder
(ii) ein induzierbarer Promotor ist; oder
(iii) ein gewebespezifischer Promotor ist; oder
(iv) ein samenspezifischer Promotor ist.

**20.** Verfahren zur Veränderung der Ölproduktion durch eine Pflanzenzelle, umfassend das Transformieren einer Pflanzenzelle mit einem rekombinanten Konstrukt gemäß Anspruch 2, das eine regulatorische Sequenz umfasst, die funktionell mit einem Polynucleotid verknüpft ist, das ein von der Nucleinsäuresequenz gemäß Anspruch 1 codiertes Lecithin:Cholesterin-Acyltransferase-artiges Polypeptid codiert, und das Kultivieren der Pflanzenzelle unter Bedingungen, bei denen die Pflanzenzelle das Lecithin:Cholesterin-Acyltransferase-artige Polypeptid exprimiert, so dass die Pflanzenzelle eine veränderte Ölproduktion im Vergleich zu Pflanzenzellen ohne das rekombinante Konstrukt aufweist.

**21.** Verfahren gemäß Anspruch 20, wobei die Ölproduktion erhöht ist.

**22.** Verfahren gemäß Anspruch 20, wobei das Konstrukt Folgendes umfasst:

(i) eine Polynucleotidsequenz, die ein Lecithin:Cholesterin-Acyltransferase-artiges Polypeptid codiert, das SEQ ID Nr. 4 aufweist; oder
(ii) eine regulatorische Sequenz, die ein gewebespezifischer Promotor ist; oder
(iii) eine regulatorische Sequenz, die ein samenspezifischer Promotor ist.

## Revendications

**1.** Séquence d'acide nucléique isolé comprenant une séquence codante qui code pour un polypeptide végétal pseudo-lécithine:cholestérol acyltransférase qui est capable d'utiliser la lécithine comme donneur d'acyle pour une acylation des stérols ou des glycérides pour former des esters, dans laquelle la séquence codante comprend un polynucléotide choisi parmi :

a) un polynucléotide isolé codant pour le polypeptide de SEQ ID NO: 5 ;
b) une séquence polynucléotidique isolée comprenant SEQ ID NO: 4 ;

c) un polynucléotide isolé ayant au moins 95 % d'identité de séquence avec SEQ ID NO: 4 ;
d) un polynucléotide isolé complémentaire d'un polynucléotide selon (a), (b) ou (c) ; et
e) un polynucléotide isolé qui s'hybride dans des conditions stringentes à SEQ ID NO: 4.

**2.** Construction d'acide nucléique recombinante comprenant une séquence régulatrice hétérologue liée de manière fonctionnelle à un polynucléotide codant pour un polypeptide pseudo-lécithine:cholestérol acyltransférase selon la revendication 1.

**3.** Construction recombinante selon la revendication 2, dans laquelle ladite séquence régulatrice hétérologue est fonctionnelle dans une cellule végétale.

**4.** Construction recombinante selon la revendication 2, dans laquelle ladite séquence régulatrice hétérologue

(i) comprend un promoteur constitutif ; ou
(ii) comprend un promoteur inductible ; ou
(iii) est choisi parmi un promoteur spécifique de tissu, un promoteur régulé par le développement, un promoteur spécifique d'un organite, et un promoteur spécifique de graine.

**5.** Construction recombinante selon la revendication 2, comprenant en outre une séquence de terminaison.

**6.** Cellule végétale contenant la construction recombinante selon la revendication 2.

**7.** Cellule végétale selon la revendication 6, qui exprime le polypeptide pseudo-lécithine:cholestérol acyltransférase codé par la séquence d'acide nucléique selon la revendication 1.

**8.** Plante comprenant la construction recombinante selon la revendication 2 et/ou comprenant au moins une cellule végétale selon la revendication 6, ou la descendance de ladite plante ou une graine provenant de ladite plante.

**9.** Plante selon la revendication 8, dans laquelle l'expression de ladite construction recombinante résulte en une composition en stérols modifiée de ladite plante par rapport à la même plante sans ladite construction recombinante.

**10.** Plante selon la revendication 9, dans laquelle ladite construction recombinante comprend

(i) une séquence polynucléotidique codant pour un polypeptide pseudo-lécithine:cholestérol acyltransférase qui possède SEQ ID NO: 4 ; ou
(ii) une séquence régulatrice comprenant un promoteur spécifique de tissu ; ou
(iii) une séquence régulatrice comprenant un promoteur spécifique de graine.

**11.** Plante selon la revendication 9, dans laquelle ladite composition en stérols modifiée est une augmentation des esters de stérol.

**12.** Plante selon la revendication 8, dans laquelle l'expression de ladite construction recombinante résulte en une production modifiée d'huile par ladite plante par rapport à la même plante sans ladite construction recombinante.

**13.** Plante selon la revendication 12, dans laquelle ladite production d'huile est augmentée.

**14.** Plante selon la revendication 12, dans laquelle ladite construction recombinante comprend

(i) une séquence polynucléotidique codant pour un polypeptide pseudo-lécithine:cholestérol acyltransférase qui possède SEQ ID NO: 4 ; ou
(ii) une séquence régulatrice, qui est un promoteur spécifique de tissu ; ou
(iii) une séquence régulatrice, qui est un promoteur spécifique de graine.

**15.** Polypeptide purifié comprenant une séquence d'acides aminés de SEQ ID NO: 5.

**16.** Procédé pour produire un polypeptide pseudo-lécithine:cholestérol acyltransférase codé par la séquence d'acide nucléique selon la revendication 1 comprenant la culture d'une cellule végétale selon la revendication 6 dans des conditions permettant l'expression dudit polypeptide pseudo-lécithine:cholestérol acyltransférase.

**17.** Procédé selon la revendication 16, comprenant en outre l'isolement du polypeptide pseudo-cholestérol acyltransférase de la cellule hôte ou du milieu dans lequel la cellule hôte est cultivée.

**18.** Procédé pour modifier la teneur en stérols d'une cellule végétale, comprenant la transformation d'une cellule végétale avec une construction recombinante selon la revendication 2 contenant une séquence régulatrice liée de manière fonctionnelle à un polynucléotide codant pour un polypeptide pseudo-lécithine:cholestérol acyltransférase codé par la séquence d'acide nucléique selon la revendication 1 et la culture de ladite cellule végétale dans des conditions dans lesquelles ladite cellule végétale exprime ledit polypeptide pseudo-lécithine:cholestérol acyltransférase de sorte que ladite cellule végétale a une composition en stérols modifiée par rapport aux cellules végétales sans la construction recombinante.

**19.** Procédé selon la revendication 18, dans lequel ladite séquence régulatrice

(i) comprend un promoteur constitutif ; ou
(ii) est un promoteur inductible ; ou
(iii) est un promoteur spécifique de tissu ; ou
(iv) est un promoteur spécifique de graine.

**20.** Procédé pour changer la production d'huile par une cellule végétale comprenant, la transformation d'une cellule végétale avec une construction recombinante selon la revendication 2 contenant une séquence régulatrice liée de manière fonctionnelle à un polynucléotide codant pour un polypeptide pseudo-lécithine:cholestérol acyltransférase codé par la séquence d'acide nucléique selon la revendication 1 et la culture de ladite cellule végétale dans des conditions dans lesquelles ladite cellule végétale exprime ledit polypeptide pseudo-lécithine:cholestérol acyltransférase de sorte que ladite cellule végétale a une production en huile modifiée par rapport aux cellules sans la construction recombinante.

**21.** Procédé selon la revendication 20, dans lequel ladite production d'huile est augmentée.

**22.** Procédé selon la revendication 20, dans lequel ladite construction comprend

(i) une séquence polynucléotidique codant pour un polypeptide pseudo-lécithine:cholestérol acyltransférase qui possède SEQ ID NO: 4 ; ou
(ii) une séquence régulatrice qui est un promoteur spécifique de tissu ; ou
(iii) une séquence régulatrice qui est un promoteur spécifique de graine.

# FIG. 1A

## ClustalW Formatted Alignments

```
                                   10                  20                  30                  40                  50
Yeast (YNR008W)  M G T L F R R N V Q N Q K S D S D E N N K G G S V H N K R E S R N H I H H Q Q G L G H K R R R G I S
Human LCAT
Rat LCAT
At LCAT1
At LCAT2
At LCAT3
At LCAT4

                                   60                  70                  80                  90                 100
Yeast (YNR008W)  G S A K R N E R G K D F D R K R D G N G R K R W R D S R R L I F I L G A F L G V L L P F S F G A Y H
Human LCAT                                                                 L L L G L L L P P A A P F W L L N V L
Rat LCAT                                 M G L P G S P W Q W V L - - - L L L G L L L P P A T S F W L L N V L
At LCAT1                                                     M K K - - - I S S H Y S V V I A I L V V V T M T S
At LCAT2                                               M G A N S K - - - S V T A S F T V I A V F F L I C G G R
At LCAT3                                                     M S L - - - L L E E I I R S V E A L L K L R N R N
At LCAT4                                                                           M G W I P C P C W G T N D D
                                                                   . .         . . . A .   F   .   N

                                  110                 120                 130                 140                 150
Yeast (YNR008W)  V H N S D S D L F D N F V N F D S L K V Y L D D W K D V L P Q G I S S F I D D I Q A G N Y S T S S L
Human LCAT       F P P H T T P K A E L S N - - - - - - - - - H T R P V I L V P G C L - - - - - - - - - - - - - - - -
Rat LCAT         F P P H T T P K A E L S N - - - - - - - - - H T R P V I L V P G C M - - - - - - - - - - - - - - - -
At LCAT1         M C Q A V G S N V Y - - - - - - - - - - - - - - - P L I L V P G N G - - - - - - - - - - - - - - - -
At LCAT2         T A V E D E T E F H G D Y - - - - - - - - - S K L S G I I I P G F A - - - - - - - - - - - - - - - -
At LCAT3         Q E P Y V D P N L N - - - - - - - - - - - - - - - P V L L V P G I A - - - - - - - - - - - - - - - -
At LCAT4         E N A G E V A D R D - - - - - - - - - - - - - - - P V L L V S G I G - - - - - - - - - - - - - - - -
                                   .                                 P V I L V P G
```

FIG. 1B

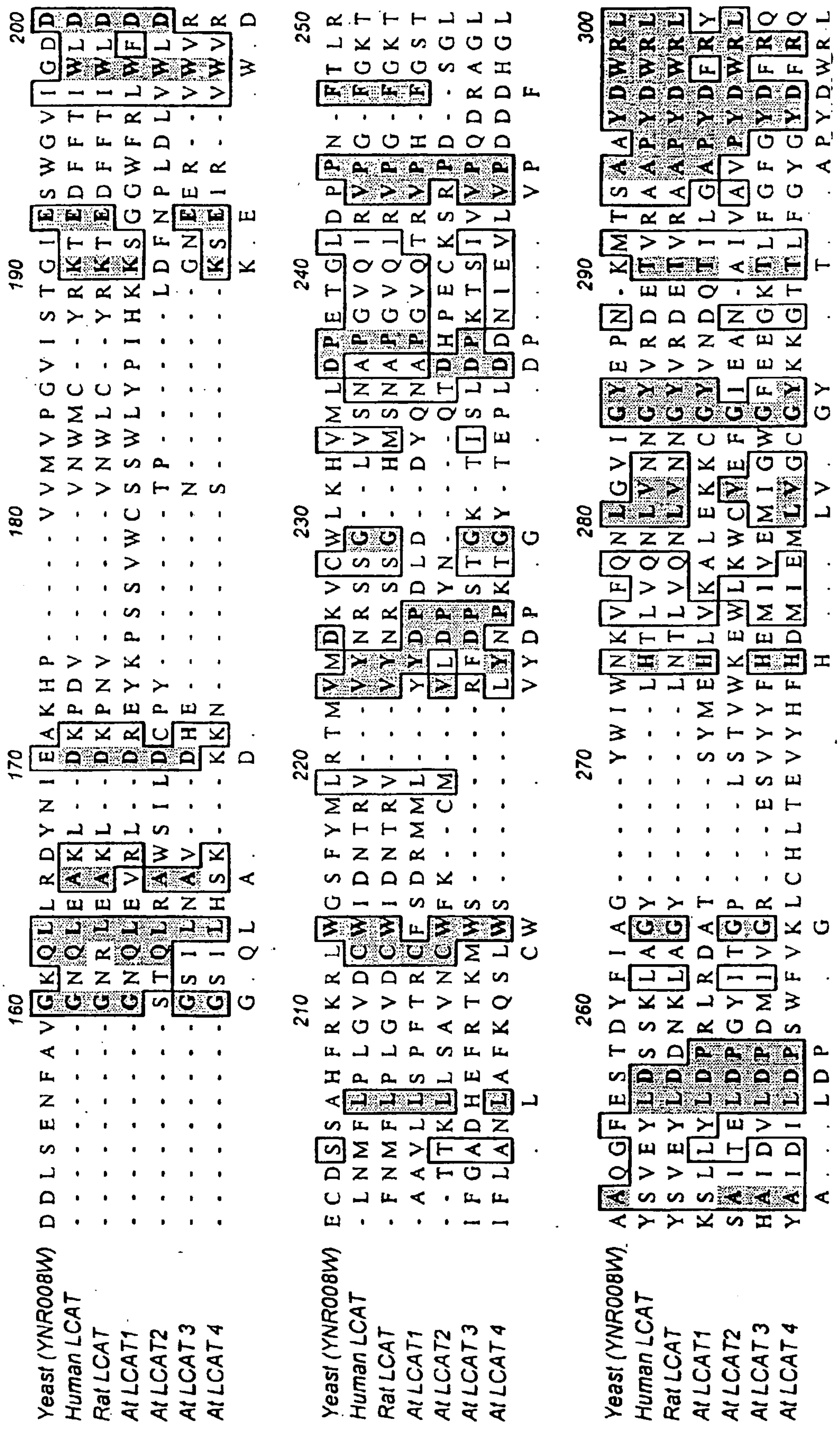
Yeast (YNR008W)
Human LCAT
Rat LCAT
At LCAT1
At LCAT2
At LCAT3
At LCAT4
160
170
180
190
200
210
220
230
240
250
260
270
280
290
300

FIG. 1C

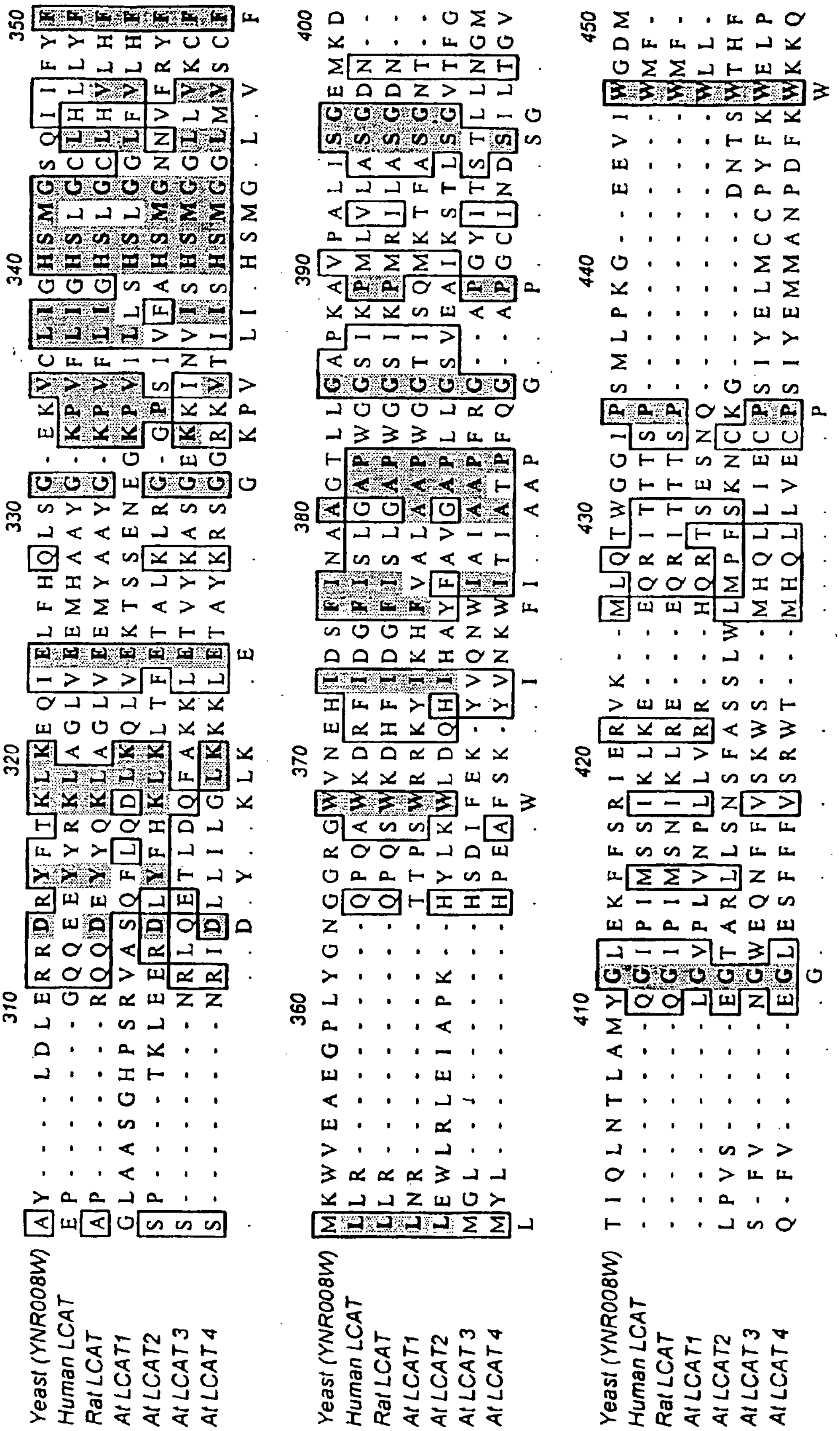
Yeast (YNR008W)
Human LCAT
Rat LCAT
At LCAT1
At LCAT2
At LCAT3
At LCAT4
310
320
330
340
350
360
370
380
390
400
410
420
430
440
450

FIG. 2

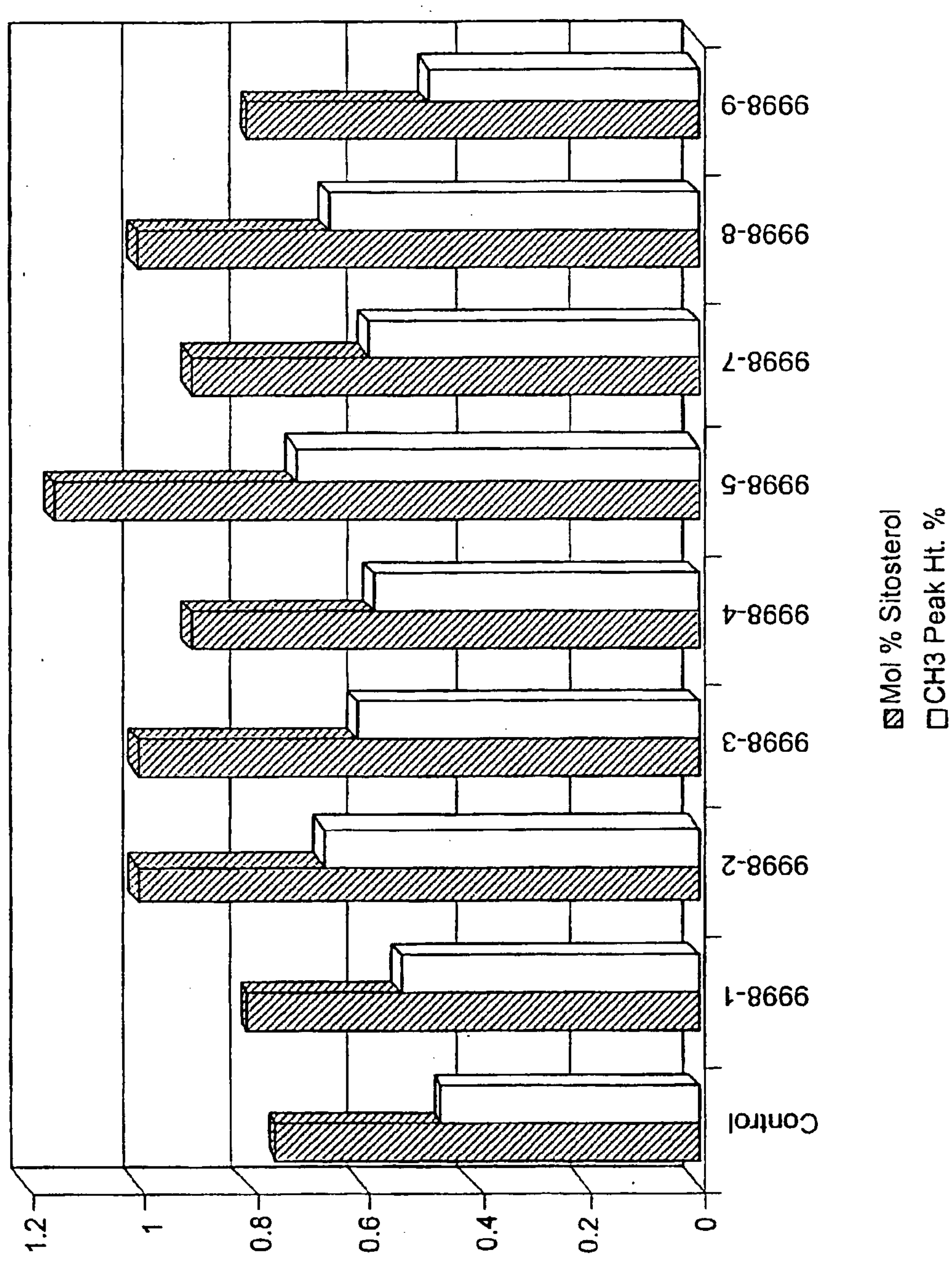
1.2
1
0.8
0.6
0.4
0.2
0
Control
9998-1
9998-2
9998-3
9998-4
9998-5
9998-7
9998-8
9998-9
Mol % Sitosterol
CH3 Peak Ht. %

FIG. 3

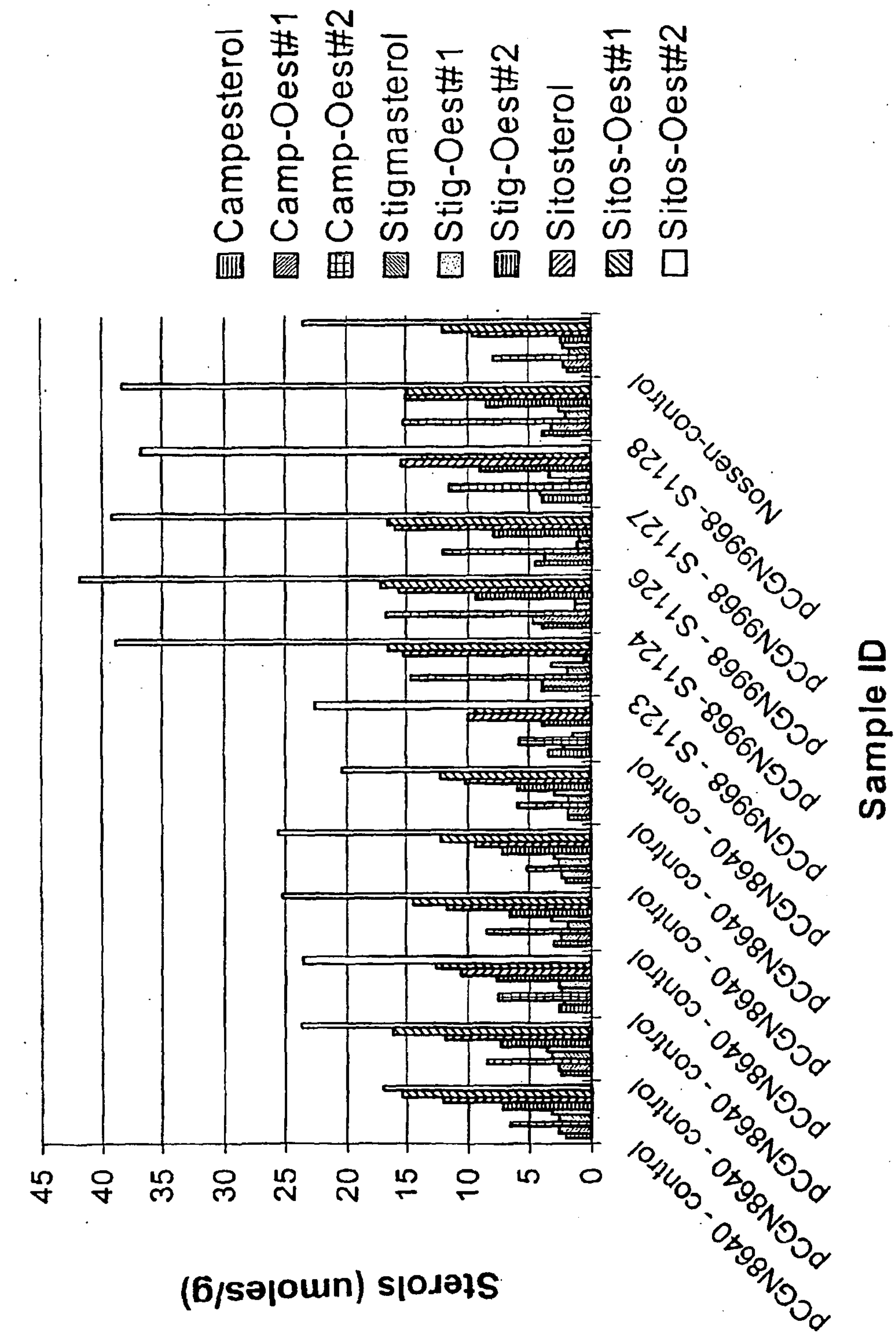
Sterol Distribution
Sterols (umoles/g)
45
40
35
30
25
20
15
10
5
0
Campesterol
Camp-Oest#1
Camp-Oest#2
Stigmasterol
Stig-Oest#1
Stig-Oest#2
Sitosterol
Sitos-Oest#1
Sitos-Oest#2
pCGN8640 - control
pCGN8640 - control
pCGN8640 - control
pCGN8640 - control
pCGN8640 - control
pCGN8640 - control
pCGN9968 - S1123
pCGN9968 - S1124
pCGN9968 - S1126
pCGN9968 - S1127
pCGN9968 - S1128
Nossen-control
Sample ID

FIG. 4

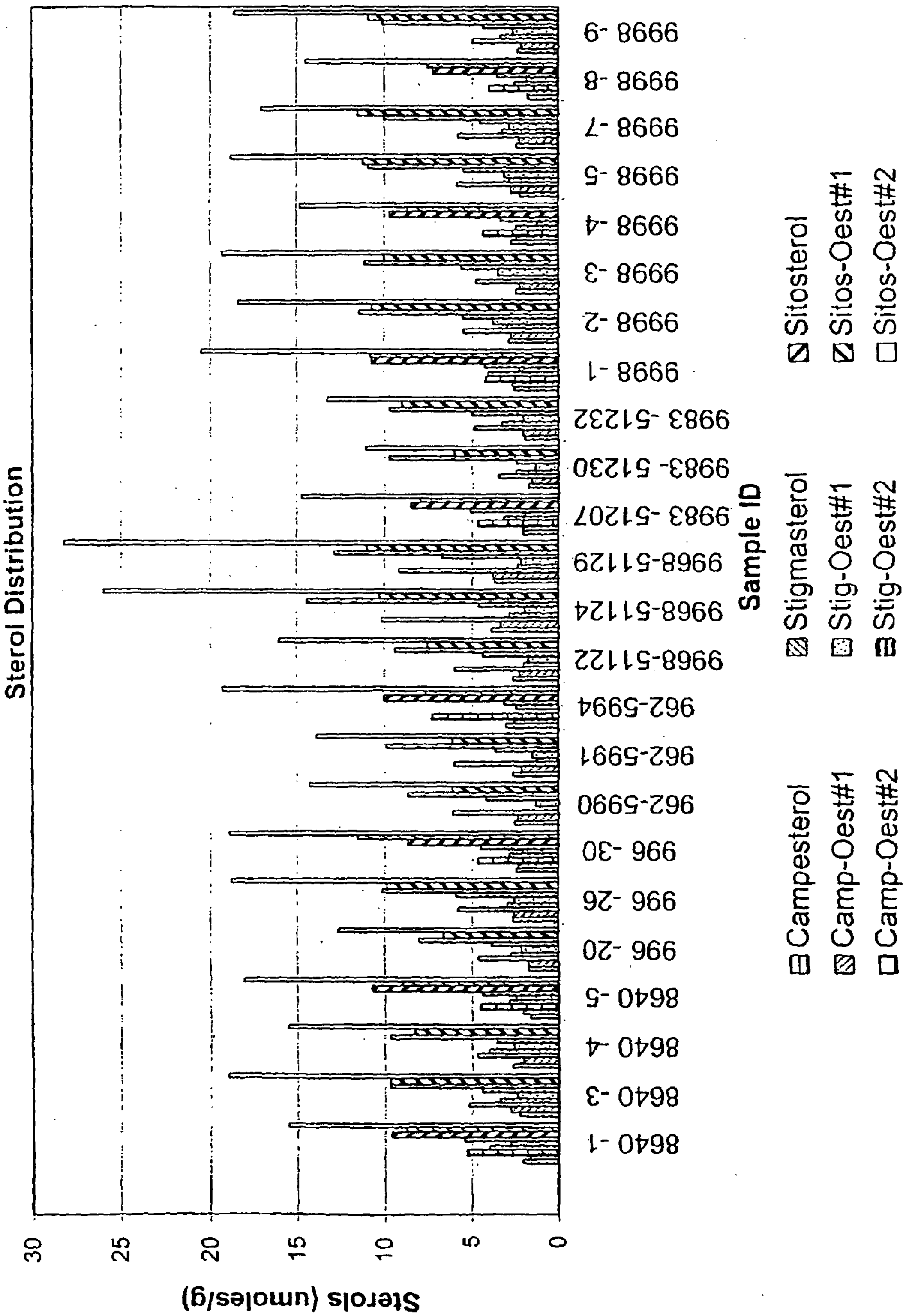
Sterol Distribution
Sterols (umoles/g)
0
5
10
15
20
25
30
Sample ID
8640 -1
8640 -3
8640 -4
8640 -5
996 -20
996 -26
996 -30
962-5990
962-5991
962-5994
9968-51122
9968-51124
9968-51129
9983 -51207
9983 -51230
9983 -51232
9998 -1
9998 -2
9998 -3
9998 -4
9998 -5
9998 -7
9998 -8
9998 -9
Campesterol
Camp-Oest#1
Camp-Oest#2
Stigmasterol
Stig-Oest#1
Stig-Oest#2
Sitosterol
Sitos-Oest#1
Sitos-Oest#2

FIG. 5

NIR Analysis of LCAT

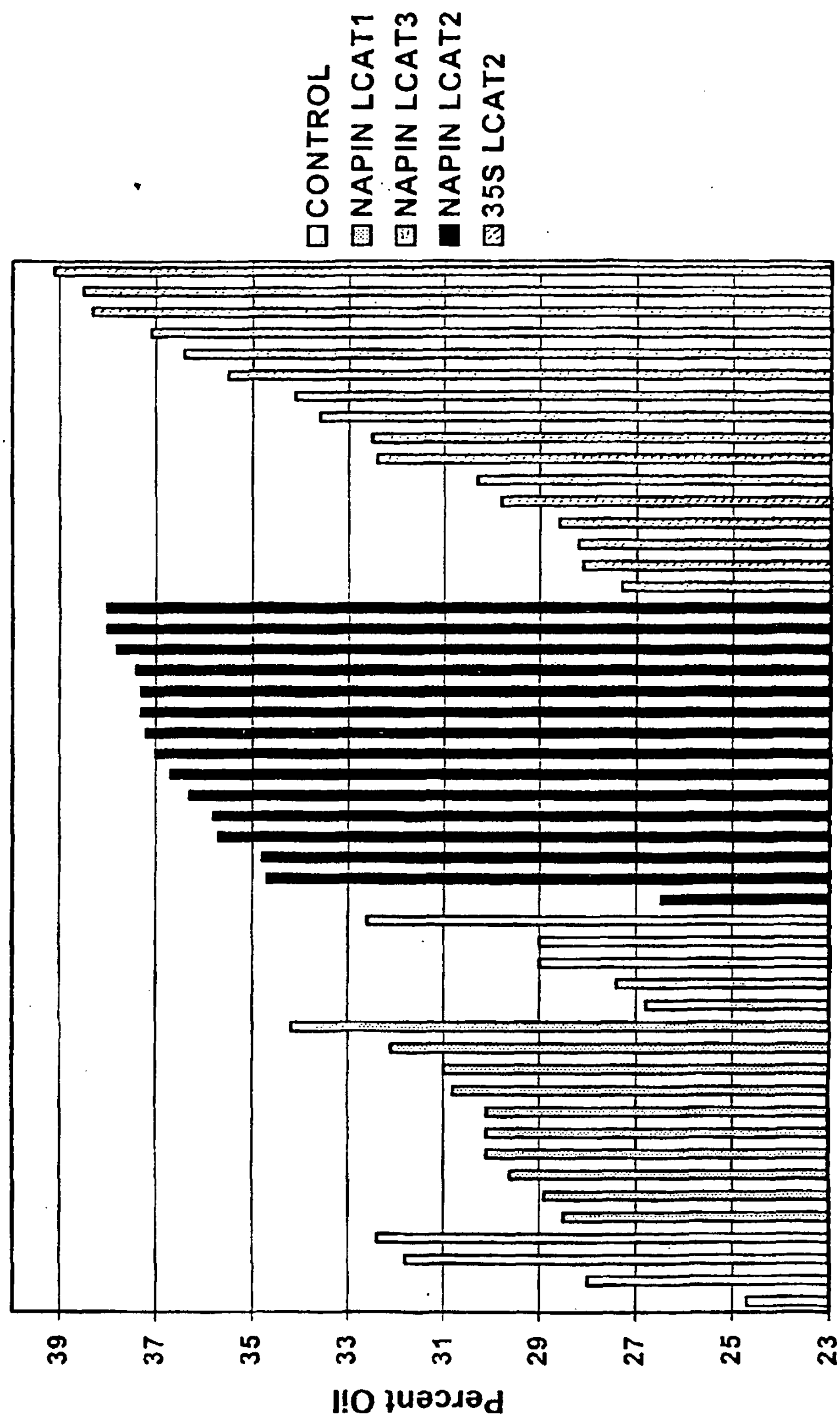

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5378619 A, Rogers
- US 4943674 A, Houck
- US 5175095 A
- US 5530185 A
- US 5753475 A
- US 5420034 A
- US 5530194 A
- EP 0255378 B1
- US 5608152 A
- US 5106739 A, Hoffman
- US 5693507 A
- US 5348886 A, Lee
- EP 0120515 A
- US 4588717 A
- US 5244887 A
- WO 9638047 A
- WO 9742830 A
- WO 9806405 A
- WO 9806714 A
- US 5270041 A
- US 5639790 A
- US 5633435 A
- US 5593874 A
- EP 00959644 A
- US 60152493 B


### Non-patent literature cited in the description


- **Goodwin.** Biosynthesis of Isoprenoid Compounds. John Wiley and Sons, 1981, vol. 1, 443-480
- **Schwender et al.** Physiology, Biochemistry, and Molecular Biology of Plant Lipids. Kluwer Academic Publishers, 1997, 180-182
- **Chang et al.** *Annu. Rev. Biochem.,* 1997, vol. 66, 613-638
- **Oelkers et al.** *J. Biol. Chem.,* 2000, vol. 26, 15609-15612
- **Dahlqvist et al.** *Proc. Natl. Acad. Sci. USA,* 2000, vol. 97, 6487-6492
- **Parker et al.** Regulation of Isopentenoid Metabolism, ACS Symposium Series. American Chemical Society, 1992, 110
- **Sambrook et al.** Molecular Cloning: A Laboratory Manual. Cold Spring Harbor, 1989
- **Ausubel et al.** Protocols in Molecular Biology. Wiley and Sons, 1995
- **Gould et al.** *PNAS USA,* 1989, vol. 86, 1934-1938
- **Odell et al.** *Nature,* 1985, vol. 313, 810-812
- **Ou-Lee et al.** *Proc. Natl. Acad. Sci. USA,* 1986, vol. 83, 6815
- **Ainley et al.** *Plant Mol. Biol.,* 1990, vol. 14, 949
- **Back et al.** *Plant Mol. Biol.,* 1991, vol. 17, 9
- **Yamaguchi-Shinozaki et al.** *Plant Mol. Biol.,* 1990, vol. 15, 905
- **Kares et al.** *Plant Mol. Biol.,* 1990, vol. 15, 905
- **Kuhlemeier et al.** *Plant Cell,* 1989, vol. 1, 471
- **Feinbaum et al.** *Mol. Gen. Genet.,* 1991, vol. 226, 449
- **Weisshaar et al.** *EMBO J.,* 1991, vol. 10, 1777
- **Lam ; Chua.** *Science,* 1990, vol. 248, 471
- **Castresana et al.** *EMBO J.,* 1988, vol. 7, 1929
- **Schulze-Lefert et al.** *EMBO J.,* 1989, vol. 8, 651
- **Cordes et al.** *Plant Cell,* 1989, vol. 1, 1025
- **Deikman et al.** *EMBO J.,* 1988, vol. 7, 3315
- **Lin et al.** *PNAS,* 1993, vol. 90, 5939
- **Doyle et al.** *J. Biol. Chem.,* 1986, vol. 261, 9228
- **Slighton ; Beachy.** *Planta,* 1987, vol. 172, 356
- **Knutzon et al.** *Proc. Natl. Acad Sci. USA,* 1992, vol. 89, 2624
- **Bustos et al.** *EMBO J.,* 1991, vol. 10, 1469
- **Lam ; Chua.** *J. Biol. Chem.,* 1991, vol. 266, 17131
- **Stayton et al.** *Aust. J. Plant. Physiol.,* 1991, vol. 18, 507
- **Bartels.** *Plant J.,* 1995, vol. 7, 809-822
- **Rogers et al.** *J. Biol. Chem.,* 1984, vol. 259, 12234
- **Khurseed et al.** *J. Biol. Chem.,* 1988, vol. 263, 18953
- **Whittier et al.** *Nucleic Acids Res.,* 1987, vol. 15, 2515
- **Kridl et al.** *Seed Sci. Res.,* 1991, vol. 1, 209-219
- **Chen et al.** *Proc. Natl. Acad. Sci,* 1986, vol. 83, 8560-8564

- **Von Heijne et al.** *Plant Mol. Biol. Rep.,* 1991, vol. 9, 104-126
- **Clark et al.** *J. Biol. Chem.,* 1989, vol. 264, 17544-17550
- **della-Cioppa et al.** *Plant Physiol.,* 1987, vol. 84, 965-968
- **Romer et al.** *Biochem. Biophys. Res Commun.,* 1993, vol. 196, 1414-1421
- **Shah et al.** *Science,* 1986, vol. 233, 478-481
- **Svab et al.** *Proc. Natl. Acad. Sci. USA,* 1990, vol. 87, 8526-8530
- **Svab ; Maliga.** *Proc. Natl. Acad. Sci. USA,* 1993, vol. 90, 913-917
- **Doolittle, R.F.** OF URFS and ORFS. University Science Books, 1986
- **Sambrook et al.** Molecular Cloning: A Laboratory Manual. Cold Spring Harbor Laboratory, 1989
- **Maliga et al.** Methods in Plant Molecular Biology. Cold Spring Harbor Laboratory Press, 1995, 39
- **Ditta et al.** *Proc. Nat. Acad. Sci., U.S.A.,* 1980, vol. 77, 7347-7351
- **McBride ; Summerfelt.** *Plant Mol. Biol.,* 1990, vol. 14, 269-276
- **Jouanin et al.** *Mol. Gen. Genet.,* 1985, vol. 201, 370-374
- **Ling et al.** *Life Sciences,* 1995, vol. 57, 195-206
- **D.M. Webb ; S.J. Knapp.** *Plant Molec. Reporter,* 1990, vol. 8, 180-185
- **Smith ; Waterman.** *J. Molec. Biol.,* 1981, vol. 147, 195-197
- **McLean J et al.** *Nucleic Acids Res,* 1986, vol. 14 (23), 9397-406
- **Chang et al.** *J. Biol. Chem.,* 1993, vol. 268, 20747-20755
- **Uelmen et al.** *J. Biol. Chem.,* 1995, vol. 270, 26192-26201
- **Yu et al.** *J. Biol. Chem.,* 1996, vol. 271, 24157-24163
- **Yang et al.** *Science,* 1996, vol. 272, 1353-1356
- **Chang et al.** *Ann. Rev. Biochem.,* 1997, vol. 66, 613-638
- **Frohman et al.** *Proc. Natl. Acad. Sci. USA,* 1988, vol. 85, 8998-9002
- **McBride ; Summerfelt.** *Plant Molecular Biology,* 1990, vol. 14, 269-276
- **Fling et al.** *Nucleic Acids Research,* 1985, vol. 13 (19), 7095-7106
- **Coruzzi et al.** *EMBO J.,* 1984, vol. 3, 1671-1679
- **Morelli et al.** *Nature,* 1985, vol. 315, 200-204
- **Leisy, D.J. et al.** *Plant Mol. Biol.,* 1989, vol. 14, 41-50
- **Depicker et al.** *J. Molec. Appl. Genet.,* 1982, vol. 1, 562-573
- *Theor. Appl. Genet.,* 1988, vol. 75, 685-694
- *Plant Cell Reports,* 1992, vol. 11, 499-505
- **Klein et al.** *Bio/Technology,* vol. 10, 286-291
- **Hood et al.** *J. Bacteriol,* 1986, vol. 168, 1291-1301
- **Holsters et al.** *Mol. Gen. Genet.,* 1978, vol. 163, 181-187
- **Valverkens et al.** *Proc. Nat. Acad. Sci.,* 1988, vol. 85, 5536-5540
- **Bent et al.** *Science,* 1994, vol. 265, 1856-1860
- **Bechtold et al.** *C. R.Acad. Sci., Life Sciences,* 1993, vol. 316, 1194-1199